(19) Europäisches Patentamt
European Patent Office
Office européen des brevets

(11) EP 1 238 678 A1

(12) EUROPEAN PATENT APPLICATION

(43) Date of publication:
**11.09.2002 Bulletin 2002/37**

(51) Int Cl.[7]: **A61K 47/48**

(21) Application number: **01105350.1**

(22) Date of filing: **08.03.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Bayer Aktiengesellschaft**
**51368 Leverkusen (DE)**

(72) Inventors:
- **Lerchen, Hans-Georg Dr.**
  **51375 Leverkusen (DE)**
- **Baumgarten, Jörg Dr.**
  **42115 Wuppertal (DE)**
- **Schoop, Andreas Dr.**
  **88441 Mittelbiberach (DE)**
- **Albers, Markus Dr.**
  **51375 Leverkusen (DE)**

(54) **Enzyme-activated cytostatic conjugates with integrin ligands**

(57) The present invention relates to cytostatics which have a tumor-specific action as a result of linkage to $\alpha_v\beta_3$ integrin antagonists via preferred linking units which can be selevtively cleaved by elastase, i.e. by an enzyme which can especially be found in tumor tissue. The preferred linking units provide sufficient stability of the conjugateof cytostatic and $\alpha_v\beta_3$ integrin antagonist in biological fluids and, at the same time, the desired intracellular action within tumour cells as a result of its specific enzymatic or hydrolytic cleavability with release of the cytostatic.



Printed by Jouve, 75001 PARIS (FR)

## Description

The present invention relates to cytostatics which have a tumor-specific action as a result of linkage to $\alpha_v\beta_3$ integrin antagonists via preferred linking units which can be selevtively cleaved by elastase, i.e. by an enzyme which can especially be found in tumor tissue. The preferred linking units provide sufficient stability of the conjugate of cytostatic and $\alpha_v\beta_3$ integrin antagonist in biological media , e.g. culture medium or serum and, at the same time, the desired intracellular action within tumour tissue as a result of its specific enzymatic or hydrolytic cleavability with release of the cytostatic.

Chemotherapy in cancer is accompanied by usually serious side effects which are to be attributed to the toxic action of chemotherapeutics on proliferating cells of other tissue types than tumour tissue. For many years, scientists have occupied themselves with the problem of improving the selectivity of active compounds employed. A frequently followed approach is the synthesis of prodrugs which are released more or less selectively in the target tissue, for example, by change of the pH (Tietze et al., e.g. DE-A-4229903), by enzymes (e.g. glucuronidases; Jacquesy et al., EP-A-0 511 917; Bosslet et al., EP-A-0 595 133) or by antibody-enzyme conjugates (Bagshawe et al., WO 88/07378; Senter et al., US-4 975 278; Bosslet et al., EP-A-0 595 133). A problem in these approaches is, inter alia, the lack of stability of the conjugates in other tissues and organs, and in particular the ubiquitous active compound distribution which follows the extracellular release of active compound in the tumour tissue.

The marked lectin pattern on tumour cell surfaces (Gabius; Onkologie 12, (1989), 175) opens up the fundamental possibility of addressing these specifically on tumour cells by linkage of appropriate carbohydrate units to cytostatics. This prospect is restricted by the fact that, even in other tissues, in particular in the liver, lectins having similar carbohydrate specificities (galactose, lactose, mannose, N-acetylglucosamine, fucose etc.) occur (Ashwell et al., Annu. Rev. Biochem. 46 (1982), 531; Stahl et al. Proc. Natl. Acad. Sci. USA 74 (1977), 1521; Hill et al., J. Biol. Chem. 262 (1986), 7433; Jansen et al., J. Biol. Chem. 266 (1991), 3343). Accordingly, a marked concentration of active compound-containing glycoconjugates in the liver and other lectin-rich organs must be expected if, in this approach, carbohydrates are used without particular modification establishing a selectivity to tumour tissue.

The heterocyclic amine batracylin (1) shows a good antitumour action in various stomach cancer models (US-4 757 072).

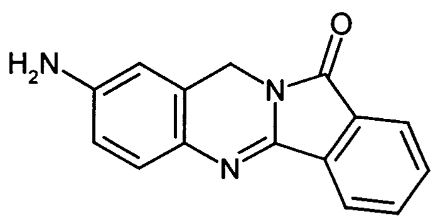

(1)

Peptide conjugates of (1) having good in-vitro action and more favourable solubility properties (US-4 180 343) are more poorly tolerable in animal experiments than free batracylin. The fucose conjugates of batracylin (1) described in EP-A-0 501 250 disadvantageously concentrate very strongly in the liver.

Quinolone-a (2), 7-[(3a-R,S, 4-R,S, 7a-S,R)-4-amino-1,3,3a,4,7,7a-hexahydro-iso-indol-2-yl]-8-chloro-1-cyclopropyl-6-fluoro-1,4-dihydro-4-oxo-3-quinolinecarboxylic acid, also shows, in addition to its outstanding antibacterial activity, a very good activity against various tumour cell lines (EP-A-0 520 240, JP-4 253 973). However, considerable toxicological problems face it (e.g. genotoxicity, bone marrow toxicity, high acute toxicity in vivo etc.).

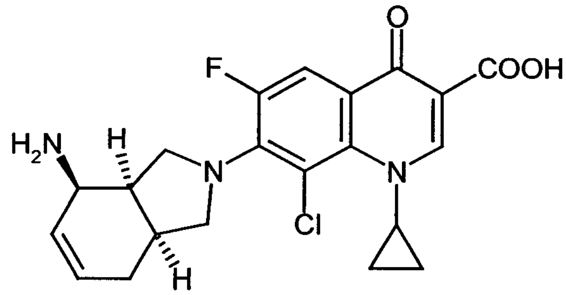

(2) (quinolone-a)

20(S)-Camptothecin is a pentacyclic alkaloid which was isolated in 1966 by Wall et al. (J. Am. Chem. Soc. 88, 3888 (1966)). It has a high active antitumour potential in numerous in-vitro and in-vivo tests. Unfortunately, however, the realization of the promising potential in the clinical investigation phase failed because of toxicity and solubility problems.

By opening of the E ring lactone and formation of the sodium salt, a water-soluble compound was obtained which is in a pH-dependent equilibrium with the ring-closed form. Here too, clinical studies have not led to success as yet.

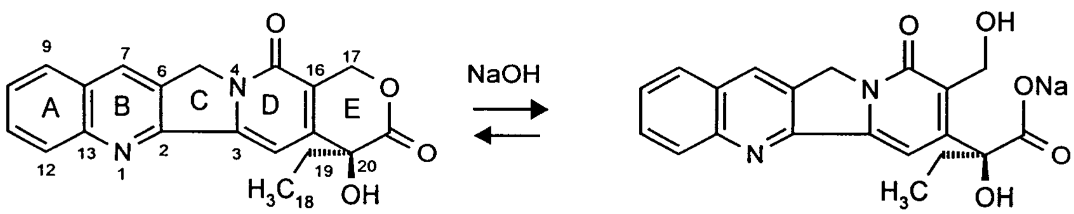


About 20 years later, it was found that the biological activity is to be attributed to enzyme inhibition of topoisomerase I. Since then, the research activities have again been increased in order to find a camptothecin derivative which is more soluble and more tolerable and which is active in vivo.

For improvement of the water solubility, salts of A ring- and B ring-modified camptothecin derivatives and of 20-O-acyl derivatives with ionizable groups have been described (Vishnuvajjala et al. US 4 943 579). The latter prodrug concept was later also transferred to modified camptothecin derivatives (Wani et al. WO 9602546). The described 20-O-acyl prodrugs, however, have a very short half-life in vivo and are very rapidly cleaved to give the parent structure.

WO 96/31532 describes carbohydrate-modified cytostatics in which both serum stability and release of the cytostatic within the tumour cells and a specific concentration of the cytostatic in tumour tissue is achieved by a novel linkage of selectively modified carbohydrates to cytostatics (for example batracylin, quinolone-a, camptothecin) via preferred spacer and linker groups.

Integrins are heterodimeric transmembrane proteins found on the surface of cells, which play an important part in the adhesion of the cells to an extracellular matrix. They recognize extracellular glycoproteins such as fibronectin or vitronectin on the extracellular matrix via the RGD sequence occurring in these proteins (RGD is the single-letter code for the amino acid sequence arginine-glycine-aspartate).

In general, integrins such as, for example, the vitronectin receptor, which is also called the $\alpha_v\beta_3$ receptor, or alternatively the $\alpha_v\beta_5$ receptor or the GpIIb/IIIa receptor play an important part in biological processes such as cell migration, angiogenesis and cell-matrix adhesion and thus for diseases in which these processes are crucial steps. Cancer, osteoporosis, arteriosclerosis, restenosis and ophthalmia may be mentioned by way of example.

The $\alpha_v\beta_3$ receptor occurs, for example, in large amounts on growing endothelial cells and makes possible their adhesion to an extracellular matrix. The $\alpha_v\beta_3$ receptor thus plays an important part in angiogenesis, i.e. the formation of new blood vessels, which is a crucial prerequisite for tumour growth and metastasis formation in carcinomatous disorders.

It was possible to show that the blockade of the abovementioned receptors is an important starting point for the treatment of disorders of this type. If the adhesion of growing endothelial cells to an extracellular matrix is suppressed by blocking their corresponding integrin receptors, for example, by a cyclic peptide or a monoclonal antibody,

angiogenesis does not occur, which leads to a stoppage or regression of tumour growth (cf., for example, Brooks et al., Cell, Volume 79, 1157-1164, 1994).

Moreover, the invasive properties of tumour cells and thus their capability to form metastases markedly decrease when their $\alpha_v\beta_3$ receptor is blocked by an antibody (Brooks et al., J. Clin. Invest., Volume 96, 1815, 1995).

WO 98/10795 describes conjugates in which a molecule adding to tumours is linked to a functional unit such as, for example, a cytostatic or a detectable label such as, for example, a radioactive nuclide. Inter alia, integrin antagonists such as, for example, peptides having the RGD sequence described above are described as molecules adding to tumours. Doxorubicin is described as an example of a cytostatic which is linked to a molecule of this type addressing tumours.

In the case of the compounds of WO 98/10795, the linkage is carried out such that the molecule addressing a tumour and the functional unit are directly bonded to one another with retention of their respective properties (cf., for example, p. 56, 1. 17, to p. 58, 1. 10, and Ex. 6). This has the result that these compounds are indeed selectively concentrated in the immediate vicinity of tumour cells by binding of the entity addressing a tumour (in the case of a radical having $\alpha_v\beta_3$ integrin-antagonistic action by binding to the $\alpha_v\beta_3$ integrin receptor which, in particular, is expressed on endothelial cells newly formed by angiogenesis), but on account of the direct combination the functional unit such as, for example, a cytostatic cannot be released into the intracellular space of the tumour tissue.

Fundamentally, the conjugate which on the one hand is selectively concentrated in tumour tissue by the effect of a part addressing $\alpha_v\beta_3$ or $\alpha_v\beta_5$ integrin receptors found in the conjugate, but on the other hand comprises a cytostatic which can be released from the conjugate, should have an increased toxophoric effect on tumour tissue due to the possibility of the more direct action of the cytostatic on the tumour cells compared with the conjugates described in WO 98/10795. In particular, such a toxophoric effect and tumor selectivity should even be higher if the release of the cytostatic takes place in the immediate vicinity of the tumor tissue or even in the tumor cells.

In principle, medicament-containing conjugates are complex, difficult-to-prepare compounds, as is explained, for example, in Anti-Cancer Drug Design 10 (1995), 1-9, in particular p. 1. In this article, conjugates of the cytostatic methotrexate, an oligopeptide spacer and a protein (human serum albumin) are described. However, it is also pointed out (cf. p. 7, first paragraph) that the nature of the linking unit and the type of linkage of this unit to the toxophore and the carrier (for example an antibody) can affect the cleavage of the linking unit. This article therefore teaches that the linkage presented there cannot be transferred to other conjugate systems without difficulty. In particular, nothing is said about whether moieties addressed also to $\alpha_v\beta_3$ integrin receptors in this manner can be linked to toxophores without the moiety addressing $\alpha_v\beta_3$ integrin receptors by this means losing its ability to bind to $\alpha_v\beta_3$ integrin receptors.

The linking units disclosed in WO 96/31532 are used specifically for the linkage of a toxophore to an oligosaccharide radical. Nothing is said about whether moieties addressed also to $\alpha_v\beta_3$ integrin receptors can be linked to toxophores in this manner, without, by this means, the moiety addressing $\alpha_v\beta_3$ integrin receptors losing its ability to bind to $\alpha_v\beta_3$ integrin receptors.

In WO 00/69472 enzyme-activated anti-tumor prodrug compounds are disclosed which can be specifically cleaved by collagenase (IV) and elastase. With respect to linking units cleavable by elastase this application describes that the specific tetrapeptide sequences Ala-Ala-Pro-Val and Ala-Ala-Pro-Nva are suitable therefore. Furthermore, in this reference, no conjugates which comprise a moiety addressing $\alpha_v\beta_3$ integrin receptors and a cytostatic are mentioned.

It was therefore the object of the present invention to develop conjugates which comprise a moiety addressing $\alpha_v\beta_3$ integrin receptors and a cytostatic which can be released from the conjugate preferably at least in the vicinity of tumor tissue, where the moiety in the conjugate addressing $\alpha_v\beta_3$ integrin receptors retains its ability to bind to the $\alpha_v\beta_3$ integrin receptor and therefore provides tissue selectivity to such compounds.

The above object is achieved by conjugates which comprise a non-peptide moiety addressing $\alpha_v\beta_3$ integrin receptors, a cytostatic and a linking unit which is selevtively enzymatically cleavable with release of the cytostatic by elastase, i.e. by an enzyme which can especially be found in tumor tissue.

According to the present invention, it has been surprisingly found that a linking unit consisting of three specific amino acids can be selectively cleaved by the tumour-associated enzyme elastase or an elastase like activity. Since advantageously the bond between this specific linking unit and the cytostatic is cleaved by elastase, the cytostatic is directly relased. This leads to an increase in the tissue specificity of the conjugates according to the invention and thus to an additional decrease of toxicity of the conjugates according to the invention in other tissue types.

According to a further preferred embodiment of the invention, the linking unit can be cleaved by enzymes which are coupled to antibodies with selectivity for tumour tissue and are thus addressed to tumour tissue. This is also called the ADEPT approach. This likewise leads to a further increase in the tissue specificity of the conjugates according to the invention and thus to an additional decrease of the conjugates according to the invention in other tissue types.

The present invention concerns conjugates of the general formula (I)

CT - LI - Sp - IA (I)

in which

CT denotes a cytotoxic radical or a radical of a cytostatic or of a cytostatic derivative, which can additionally carry a hydroxyl, carboxyl or amino group,

LI is a linker group comprising 3 amino acid residues in the D or L configuration, which can each optionally carry protective groups,

Sp is absent or a carbonyl or a thiocarbonyl radical,

IA is a non-peptide radical addressing an $\alpha_v\beta_3$ integrin receptor, which is selected from the group consisting of

A) a radical of the formula (II)

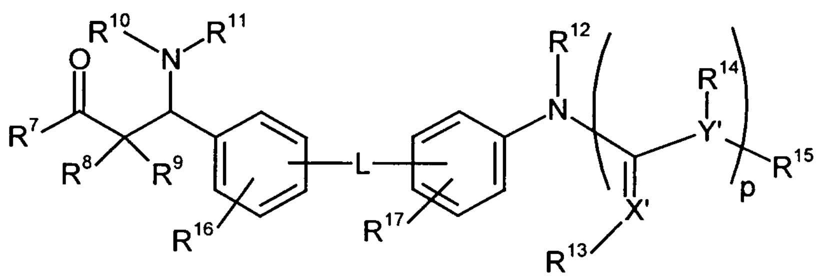

(II)

in which

$R^7$ is OH, a substituted or unsubstituted alkoxy or cycloalkoxy radical, a substituted or unsubstituted aryloxy radical or a saturated or unsaturated, optionally substituted heterocyclyloxy radical, or optionally represents a direct bond or an atom from the group consisting of N, O and S, via which the radical of the formula (II) is bonded to the rest of the conjugate;

$R^8$ is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical or is bonded to $R^9$ with formation of an optionally substituted carbocyclic or heterocyclic ring system which includes the carbon atom to which $R^8$ is bonded and can optionally contain heteroatoms;

$R^9$ is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical or is bonded to $R^8$ with formation of an optionally substituted carbocyclic or heterocyclic ring system which includes the carbon atom to which $R^9$ is bonded and can optionally contain heteroatoms;

$R^{10}$ is $-SO_2R^{10'}$, $-COOR^{10''}$, $-COR^{10'}$, $-CONR^{10'}_2$ or $-CS-NR^{10'}_2$, or represents a direct bond via which the radical of the formula (II) is optionally bonded to the rest of the conjugate;

$R^{10'}$ independently of one another is hydrogen, a substituted or unsubstituted alkyl or cycloalkyl radical, a substituted or unsubstituted aryl radical or a saturated or unsaturated, optionally substituted heterocyclic radical, via which the radical of the formula (II) is optionally bonded to the rest of the conjugate;

$R^{10''}$ is a substituted or unsubstituted alkyl or cycloalkyl radical, a substituted or unsubstituted aryl radical or a

saturated or unsaturated, optionally substituted heterocyclic radical, via which the radical of the formula (II) is optionally bonded to the rest of the conjugate;

R" is hydrogen, a substituted or unsubstituted alkyl or cycloalkyl radical or a substituted or unsubstituted aryl radical,

$R^{16}$ is hydrogen, CN, a substituted or unsubstituted alkyl or cycloalkyl radical, a substituted or unsubstituted alkoxy radical or a halogen atom;

$R^{17}$ is hydrogen, CN, a substituted or unsubstituted alkyl or cycloalkyl radical, a substituted or unsubstituted alkoxy radical or a halogen atom;

L is $-(CH_2)_nNHSO_2(CH_2)_o-$, $-(CH_2)_nSO_2NH(CH_2)_o-$, $-(CH_2)_nNH-CO(CH_2)_o-$, $-(CH_2)_nCONH(CH_2)_o-$, $-(CH_2)_nOCH_2(CH_2)_o-$, $-(CH_2)_nCH_2O(CH_2)_o-$, $-(CH_2)_nCOO(CH_2)_o-$, $-(CH_2)_nOOC-(CH_2)_o-$, $-(CH_2)_nCH_2CO(CH_2)_o-$, $-(CH_2)_nCOCH_2(CH_2)_o-$, -NHCONH-, $-(CH_2)_nSCH_2(CH_2)_o-$, $-(CH_2)_nCH_2S(CH_2)_o-$, $-(CH_2)_nCH_2SO(CH_2)_o-$, $-(CH_2)_nSOCH_2(CH_2)_o-$, $-(CH_2)_nCH_2- SO_2(CH_2)_o-$ or $-(CH_2)_nSO_2CH_2(CH_2)_o-$,
where n and o each is an integer of 0 or 1 and $n + o \leq 1$;

$R^{12}$ is hydrogen, a substituted or unsubstituted alkyl or cycloalkyl radical, a substituted or unsubstituted aryl radical, a saturated or unsaturated, optionally substituted heterocyclic radical or is bonded to one of $R^{13}$, $R^{14}$ or $R^{15}$, if present, with formation of an optionally substituted heterocyclic ring system which includes the nitrogen atom, to which $R^{12}$ is bonded and can be saturated or unsaturated and/or can contain further heteroatoms;

X' is N, O or S;

p is 0 or 1;

$R^{13}$ is absent, is -H, a substituted or unsubstituted alkyl or cycloalkyl radical, $-NO_2$, -CN, $-COR^{13'}$, $-COOR^{13'}$, or is bonded to one of $R^{12}$, $R^{14}$ or $R^{15}$ with formation of an optionally substituted heterocyclic ring system which includes X' and can be saturated or unsaturated and/or can contain further heteroatoms;

$R^{13'}$ is hydrogen, a substituted or unsubstituted alkyl or cycloalkyl radical, a substituted or unsubstituted aryl radical or a saturated or unsaturated, optionally substituted heterocyclic radical which can be saturated or unsaturated and/or can contain further heteroatoms;

Y' is N or S;

$R^{14}$ is absent, hydrogen, a substituted or unsubstituted alkyl or cycloalkyl radical, a substituted or unsubstituted aryl radical, a saturated or unsaturated, optionally substituted heterocyclic radical or is bonded to one of $R^{12}$, $R^{13}$ or $R^{15}$, if present, with formation of an optionally substituted heterocyclic ring system which includes the nitrogen atom to which $R^{14}$ is bonded and can be saturated or unsaturated and/or can contain further heteroatoms;

$R^{15}$ is hydrogen, a substituted or unsubstituted alkyl or cycloalkyl radical, a substituted or unsubstituted aryl radical, a saturated or unsaturated, optionally substituted heterocyclic radical or is bonded to one of $R^{12}$, $R^{13}$ or $R^{14}$, if present, with formation of an optionally substituted heterocyclic ring system which includes the nitrogen atom to which $R^{15}$ is bonded and can be saturated or unsaturated and/or can contain further heteroatoms, or optionally represents a direct bond via which the radical of the formula (II) is bonded to the rest of the conjugate;

and their physiologically acceptable salts and stereoisomers.

Of the conjugates of the formula (I), according to a preferred embodiment those conjugates are preferred in which

LI is a linker group having the formula

-AA1-AA2-AA3

wherein AA1 is bonded to the radical CT and

AA1 is a naturally occurring amino acid in the D or L configuration, which is selected from the group consisting of glycine, alanine, valine, leucine, isoleucine, histidine, glutamate, aspartate, serine, lysine, ornithine and phenylalanine;

AA2 is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine, and can optionally carry protective groups,

AA3 is a naturally occurring amino acid in the D or L configuration, which is selected from the group consisting of alanine, valine, phenylalanine, tyrosine, serine, isoleucine, lysine, glutamine, glutamate, histidine, glycine, arginine, asparagine, aspartate, tryptophane, proline, ornithine, methionine, S-methyl-cysteine, norvaline and leucine, and can optionally carry protective groups,

and the other radicals CT, Sp and IA are as defined above.

Particularly preferred are conjugates according to formula (I), in which

LI is a linker group having the formula

-AA1-AA2-AA3-

wherein AA1 is bonded to the radical CT and

AA1 is valine, leucine, isoleucine;

AA2 is proline,

AA3 is alanine, norvaline, histidine, glycine, asparagine, aspartate,

and the other radicals CT, Sp and IA are as defined above.

Of the conjugates of the formula (I), according to yet a further preferred embodiment those conjugates are particularly preferred in which

CT is camptothecin, which can be linked to the rest of the conjugate via the C20-OH group;

LI is as defined above;

Sp is absent, or a carbonyl or a thiocarbonyl radical,

IA is a non-peptide radical of the formula (II) addressing an $\alpha_v\beta_3$ integrin receptor, in which

$R^7$ is OH, methoxy, ethoxy, propoxy, isopropoxy, butoxy, isobutoxy, t-butoxy, pentoxy, isopentoxy, neopentoxy, hexoxy, cyclopropoxy, cyclopropylmethoxy, cyclobutoxy, cyclopentoxy, cyclohexoxy, phenoxy, benzyloxy, tolyloxy or a substituted derivative thereof, or optionally represents a direct bond or an atom from the group consisting of N, O and S, via which the radical of the formula (II) is bonded to the rest of the conjugate;

$R^8$ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -OH, methoxy, ethoxy, propoxy, butoxy, pentoxy, hexoxy, benzyloxy or is bonded to $R^9$ with formation of an optionally substituted 3- to 6-membered carbocyclic or heterocyclic ring system, which includes the carbon atom to which $R^8$ is bonded and can optionally contain heteroatoms;

$R^9$ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -OH, methoxy, ethoxy, propoxy, butoxy, pentoxy, hexoxy or is bonded to $R^8$ with formation of an optionally substituted 3- to 6-membered carbocyclic or heterocyclic ring system which includes the carbon atom to which $R^9$ is bonded and can optionally contain heteroatoms;

$R^{10}$ is $SO_2R^{10'}$, $-COOR^{10''}$, $-COR^{10'}$, $-CONR^{10'}_2$ or $-CSNR^{10'}_2$ or represents a direct bond, via which the radical of the formula (II) is optionally bonded to the rest of the conjugate;

$R^{10'}$ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, $-C_6H_2(CH_3)_3$, $-C_6(CH_3)_5$, $-CH_2C_6H_2(CH_3)_3$, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichlorophenylmethyl, 2,6-dichlorophenylmethyl, 3-aminophenyl, 4-aminophenyl, 4-carboxyphenyl, 2-methoxycarbonylphenylmethyl, 3-trifluoromethylphenyl, 4-trifluoromethylphenyl, 3,5-bis(trifluoromethyl)phenyl, 4-trifluo-romethoxyphenyl, phenylmethyl, 2-acetamido-4-meth-ylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimeth-oxyphenyl, 3methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluoro-phenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridine-5-methyl, 5,7-dimethyl-1,3,4-triazaindolizin-2-yl, (S)-camphan-1-yl, (R)-camphan-1-yl or 8-quinolinyl;

$R^{10''}$ is a $C_{1-6}$-alkyl radical, a $C_{3-7}$-cycloalkyl radical, a substituted or unsubstituted aryl radical or a saturated or unsaturated, optionally substituted heterocyclic radical, via which the radical of the formula (II) is optionally bonded to the rest of the conjugate;

$R^{11}$ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl, dialkylamino-$C_{1-4}$-alkyl, amino-$C_{1-4}$-alkyl, $C_{1-4}$-alkyloxy-$C_{1-4}$-alkyl or

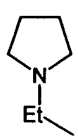 , 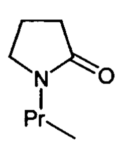 , 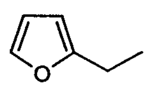 , 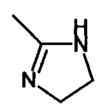 , 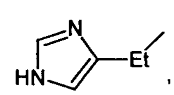 ,

(a1) (a2) (a3) (a4) (a5)

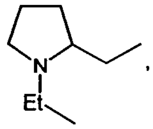 , 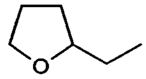 , 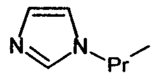 , 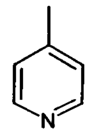 , 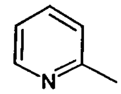 ,

(a6) (a7) (a8) (a9) (a10)

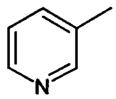 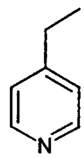 , 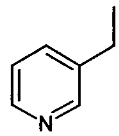 , 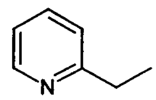 , 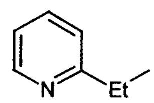 ,

(a11) (a12) (a13) (a14) (a15)

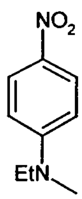 , 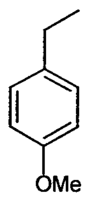 , 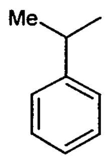 , 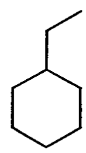 , 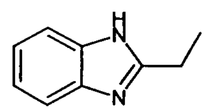

(a16) (a17) (a18) (a19) (a20)

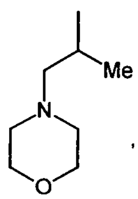 , 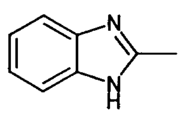 , 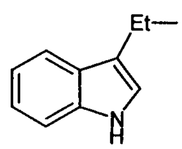 , 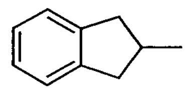 ,

(a21) (a22) (a23) (a24)

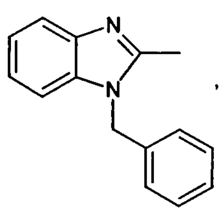 , 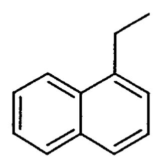 , 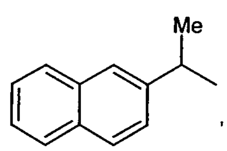 , 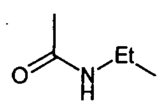 ,

(a25) (a26) (a27) (a28)

$R^{16}$ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;

$R^{17}$ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, ethoxy, trifluoromethoxy,

propoxy, butoxy, pentoxy or hexoxy, fluorine, chlorine, bromine or iodine;

L is $-NHSO_2-$, $-CH_2NHSO_2-$, $-NHSO_2CH_2-$, $-SO_2NH-$, $-CH_2SO_2NH-$, $-SO_2NHCH_2-$, -NHCO-, $-CH_2NHCO-$, $-NHCOCH_2-$, -CONH-, $-CH_2CONH-$, $-CONHCH_2-$, $-OCH_2-$, $-CH_2OCH_2$, $-OCH_2CH_2-$, $-CH_2O-$ or $-CH_2CH_2O-$;

$R^{12}$ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl, one of the radicals (a1) to (a28) or is bonded to one of $R^{13}$, $R^{14}$ or $R^{15}$, if present, with formation of an optionally substituted heterocyclic 4- to 6-membered ring system which includes the nitrogen atom to which $R^{12}$ is bonded and can be saturated or unsaturated and/or can contain further heteroatoms;

X' is N, O or S;

p is 0 or 1;

$R^{13}$ is absent, is -H, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, $-NO_2$, -CN, $-COR^{7'}$, $-COOR^{7'}$, or is connected to one of $R^{12}$, $R^{14}$ or $R^{15}$ with formation of an optionally substituted carbocyclic or heterocyclic 4- to 6-membered ring system which includes X' and can be saturated or unsaturated and/or can contain further heteroatoms;

$R^{13'}$ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof;

Y' is N or S;

$R^{14}$ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl, one of the radicals (a1) to (a28), or is connected to one of $R^{12}$, $R^{13}$ or $R^{15}$, if present, with formation of an optionally substituted heterocyclic 4- to 6-membered ring system which includes the nitrogen atom to which $R^{14}$ is bonded and can be saturated or unsaturated and/or can contain further hetero atoms; and

$R^{15}$ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl, one of the radicals (a1) to (a28), or is bonded to one of $R^{12}$, $R^{13}$ or $R^{14}$, if present, with formation of an optionally substituted heterocyclic 4- to 6-membered ring system which includes the nitrogen atom to which $R^{15}$ is bonded and can be saturated or unsaturated and/or can contain further heteroatoms, and or optionally represents a direct bond via which the radical of the formula (II) is bonded to the rest of the conjugate.

Particularly preferred conjugates of the formula (I) in this further preferred embodiment are those in which $R^7$ represents a direct bond or an atom from the group consisting of N, O and S, via which the radical of the formula (II) is bonded to the rest of the conjugate, and the other radicals of the formula (II) are as defined above.

Likewise, particularly preferred conjugates of the formula (I) in this further preferred embodiment are those in which $R^{15}$ represents a direct bond, via which the radical of the formula (II) is bonded to the rest of the conjugate, and the other radicals of the formula (II) are as defined above.

Likewise particularly preferred conjugates of the formula (I) in this further preferred embodiment are those in which the radical of the formula (II) is linked to the rest of the conjugate via a radical in the α- or β-position relative to the carboxyl group, and the other radicals of the formula (II) are as defined above. Form these conjugates especially preferred are the ones wherein

CT is camptothecin which is linked to the rest of the conjugate via the C20-OH group;

LI is as defined in claim 3;

Sp is a thiocarbonyl radical,

IA is a non-peptide radical of the formula (II) addressing an $\alpha_v\beta_3$ integrin receptor, in which

| | |
|---|---|
| $R^7$ | is OH; |
| $R^8$, $R^9$, $R^{12}$, $R^{14}$, $R^{16}$ and $R^{17}$ | are H; |
| $R^{10}$ | is $CONHR^{10'}$; |
| $R^{10'}$ | is -Ph-NH-, wherein the radical of the formula (II) is linked to the rest of the conjugate via the nitrogen atom in $R^{10'}$; |
| L | is $-NH-SO_2-$ being linked to the adjacent phenylene units such that each of these phenylene units is 1,3-substituted; |
| X' | is O; |
| $R^{13}$ | is absent; |
| P | is 1; |
| R15 | is propyl. |

The compounds of the formula (I) according to the invention can also be present in the form of their salts. In general salts with organic or inorganic bases or acids may be mentioned here.

In particular, the compounds of the formula (I) according to the invention can be employed in the form of their physiologically acceptable salts. Physiologically acceptable salts are understood according to the invention as meaning non-toxic salts which in general are accessible by reaction of the compounds of the formula (I) according to the invention with an inorganic or organic base or acid conventionally used for this purpose. Examples of preferred salts of the compounds of the formula (I) according to the invention are the corresponding alkali metal salt, e.g. lithium, potassium or sodium salt, the corresponding alkaline earth metal salt such as the magnesium or calcium salt, a quaternary ammonium salt such as, for example, the triethylammonium salt, acetate, benzenesulphonate, benzoate, dicarbonate, disulphate, ditartrate, borate, bromide, carbonate, chloride, citrate, dihydrochloride, fumarate, gluconate, glutamate, hexylresorcinate, hydrobromide, hydrochloride, hydroxynaphthoate, iodide, isethionate, lactate, laurate, malate, maleate, mandelate, mesylate, methylbromide, methylnitrate, methylsulphate, nitrate, oleate, oxalate, palmitate, pantothenate, phosphate, diphosphate, polygalacturonate, salicylate, stearate, sulphate, succinate, tartrate, tosylate and valerate and other salts used for medicinal purposes.

The present invention includes both the individual enantiomers or diastereomers and the corresponding racemates, diastereomer mixtures and salts of the compounds according to the invention. In addition, all possible tautomeric forms of the compounds described above are included according to the present invention. Furthermore, the present invention includes both the pure E and Z isomers of the compounds of the formula (I) and their E/Z mixtures in all ratios. The diastereomer mixtures or E/Z mixtures can be separated into the individual isomers by chromatographic processes. The racemates can be resolved into the respective enantiomers either by chromatographic processes on chiral phases or by resolution.

In the context of the present invention, the substituents, if not stated otherwise, in general have the following meaning:

Alkyl in general represents a straight-chain or branched hydrocarbon radical having 1 to 20 carbon atoms. Examples which may be mentioned are methyl, ethyl, propyl, isopropyl, butyl, isobutyl, pentyl, isopentyl, hexyl, isohexyl, heptyl, isoheptyl, octyl and isooctyl, nonyl, decyl, dodeyl, eicosyl.

Alkenyl in general represents a straight-chain or branched hydrocarbon radical having 2 to 20 carbon atoms and

one or more, preferably having one or two, double bonds. Examples which may be mentioned are allyl, propenyl, isopropenyl, butenyl, isobutenyl, pentenyl, isopentenyl, hexenyl, isohexenyl, heptenyl, isoheptenyl, octenyl, isooctenyl.

Alkinyl in general represents a straight-chain or branched hydrocarbon radical having 2 to 20 carbon atoms and one or more, preferably having one or two, triple bonds. Examples which may be mentioned are ethinyl, 2-butinyl, 2-pentinyl and 2-hexinyl.

Acyl in general represents straight-chain or branched lower alkyl having 1 to 9 carbon atoms, which is bonded via a carbonyl group. Examples which may be mentioned are: acetyl, ethylcarbonyl, propylcarbonyl, isopropylcarbonyl, butyl-carbonyl and isobutylcarbonyl.

Alkoxy in general represents a straight-chain or branched hydrocarbon radical having 1 to 14 carbon atoms and bonded via an oxygen atom. Examples which may be mentioned are methoxy, ethoxy, propoxy, isopropoxy, butoxy, isobutoxy, pentoxy, isopentoxy, hexoxy, isohexoxy, heptoxy, isoheptoxy, octoxy or isooctoxy, The terms "alkoxy" and "alkyloxy" are used synonymously.

Alkoxyalkyl in general represents an alkyl radical having up to 8 carbon atoms, which is substituted by an alkoxy radical having up to 8 carbon atoms.

Alkoxycarbonyl can be represented, for example, by the formula

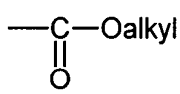

Alkyl here in general represents a straight-chain or branched hydrocarbon radical having 1 to 13 carbon atoms. Examples which may be mentioned are the following alkoxycarbonyl radicals: methoxycarbonyl, ethoxycarbonyl, propoxycarbonyl, isopropoxycarbonyl, butoxycarbonyl or isobutoxycarbonyl.

Cycloalkyl in general represents a cyclic hydrocarbon radical having 3 to 8 carbon atoms. Cyclopropyl, cyclopentyl and cyclohexyl are preferred. Examples which may be mentioned are cyclopentyl, cyclohexyl, cycloheptyl and cyclooctyl.

Cycloalkoxy in the context of the invention represents an alkoxy radical whose hydrocarbon radical is a cycloalkyl radical. The cycloalkyl radical in general has up to 8 carbon atoms. Examples which may be mentioned are: cyclopropyloxy and cyclohexyloxy. The terms "cycloalkoxy" and "cycloalkyloxy" are used synonymously.

Aryl in general represents an aromatic radical having 6 to 10 carbon atoms. Preferred aryl radicals are phenyl, benzyl and naphthyl.

Halogen in the context of the invention represents fluorine, chlorine, bromine and iodine.

Heterocycle in the context of the invention in general represents a saturated, unsaturated or aromatic 3- to 10-membered, for example 5- or 6-membered, heterocycle which can contain up to 3 heteroatoms from the group consisting of S, N and/or O and which, in the case of a nitrogen atom, can also be bonded via this. Examples which may be mentioned are: oxadiazolyl, thiadiazolyl, pyrazolyl, pyridyl, pyrimidinyl, pyridazinyl, pyrazinyl, thienyl, furyl, pyrrolyl, pyrrolidinyl, piperazinyl, tetrahydropyranyl, tetrahydrofuranyl, 1,2,3-triazolyl, thiazolyl, oxazolyl, imidazolyl, morpholinyl or piperidyl. Thiazolyl, furyl, oxazolyl, pyrazolyl, triazolyl, pyridyl, pyrimidinyl, pyridazinyl and tetrahydropyranyl are preferred. The term "heteroaryl" (or "hetaryl") represents an aromatic heterocyclic radical.

The conjugates according to the invention are characterized in that a cytotoxic radical or a radical of a cytostatic or of a cytostatic derivative is bonded via a linking unit to a non-peptide moiety addressing $\alpha_v\beta_3$ integrin receptors.

The non-peptide moiety of the conjugate addressing $\alpha_v\beta_3$ integrin receptors serves to bring the toxophoric part of the conjugate into or into the vicinity of tumour cells and thus to achieve tissue selectivity. Growing tumour tissue stimulates the formation of new blood vessels, i.e. angiogenesis, to a considerable extent in order to cover its increasing

nutritional need. The blood vessels newly formed by angiogenesis differ from conventional tissue by specific markers on the surfaces of the endothelial cells formed. Moreover, the $\alpha_v\beta_3$ integrin receptor is expressed by many human tumours (cf. WO 98/10795 and the references indicated there). Thus the conjugate is brought selectively into or into the vicinity of the tumour tissue to be treated by the interaction of its non-peptide part addressing $\alpha_v\beta_3$ integrin receptors with $\alpha_v\beta_3$ integrin receptors found on endothelial cells or on tumour cells formed by angiogenesis.

Unlike peptide radicals addressing $\alpha_v\beta_3$ integrin receptors (such as disclosed, for example, in WO 98/10795), the non-peptide moieties according to the invention addressing $\alpha_v\beta_3$ integrin receptors are distinguished by an increased serum stability, whereby the transport of the toxophore in the conjugate to the tumour tissue is ensured to an increased extent.

The abovementioned compounds having antagonistic action against $\alpha_v\beta_3$ integrin receptors must be able to retain their property of addressing $\alpha_v\beta_3$ integrin receptors in the conjugate. This means that these compounds must be linked to a toxophore in such a way that no or only a slight impairment of the abovementioned action of the compounds results thereby. In the normal case, the linkage with the linking unit will take place via a functional group suitable for this in the molecule, for example via an amino, hydroxyl or carboxyl function. If the abovementioned compounds have no functional group, one of these is easily insertable into the molecule by conventional processes known to the person in the art without the loss of the antagonistic action against $\alpha_v\beta_3$ integrin receptors occurring here.

The conjugate according to the invention can release its toxophoric radical at its target site and this can thus make possible penetration into the tumour tissue. This is carried out by the specific choice of a unit linking the toxophoric radical to the moiety addressing $\alpha_v\beta_3$ integrin receptors. The linking unit of the conjugates of the present invention is designed such that it can be cleaved by the tumor-associated enzyme elastase or an elastase-like activity. It is surprising that the enzyme recognizes and cleaves a substrate seuqence as in the compounds of the present invention which is bonded to rather bulky moieties both at the amino and the carboxy terminus and that it directly releases the toxophore.

A further suitable starting point for promoting the tissue selectivity of the action of the conjugates according to the invention consists in the so-called ADEPT approach. In this, conjugates are cleaved by certain enzymes. These enzymes are introduced into the body coupled to antibodies together with the conjugates according to the invention, the antibodies serving as vehicles specifically addressing tumour tissue. This leads to a selective concentration both of the conjugate and of the enzyme/antibody system in the tumour tissue, whereby the toxophore is released in the tumour tissue with even greater selectivity and can display its action there.

Suitable linking units according to the invention are all linking units which fulfil the abovementioned criteria and can be linked to the moiety addressing $\alpha_v\beta_3$ integrin receptors in such a way that this retains its binding action to $\alpha_v\beta_3$ integrin receptors.

In the conjugates according to the invention, toxophores used can be all cytotoxic radicals or radicals of a cytostatic or of a cytostatic derivative which are conventionally employed in tumour therapy, e.g. camptothecin or quinolone-a.

According to a preferred embodiment, conjugates according to the invention which can be employed are compounds of the formula (I) in which a toxophore is linked via a linking unit consisting of 3 amino acids and, if appropriate, of a non-peptide spacer group, to a non-peptide moiety addressing $\alpha_v\beta_3$ integrin receptors of radicals of the formula (II):

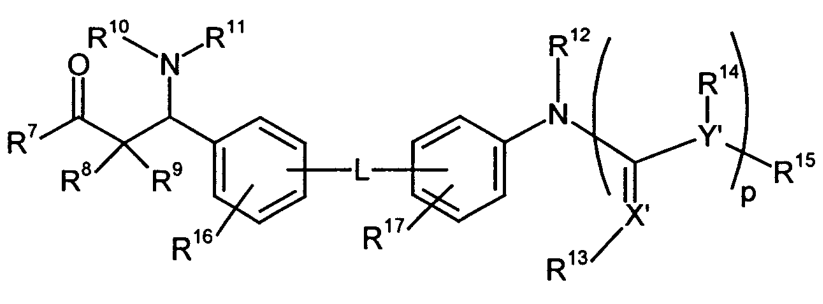

(II)

where the radicals in the formula (II) have the meanings indicated above.

In the conjugates of the formula (I) according to the invention, the toxophore used can be cytostatic radicals or radicals of a cytostatic or of a cytostatic derivative which are conventionally employed in tumour therapy. Camptothecin or derivatives of camptothecin such as 9-aminocamptothecin are preferred here, which can be linked to the rest of the conjugate via the C20-OH group or via a functional group which is optionally present in the molecule, such as the amino group in the case of 9-aminocamptothecin. According to this preferred embodiment, the camptothecin

unit used as a starting compound can be present in the 20(R) or in the 20(S) configuration or as a mixture of these two stereoisomeric forms. The 20(S) configuration is preferred.

In the conjugates of the formula (I), the linking unit preferably consists of a unit of the formula

-LI-Sp-

wherein the unit LI preferably comprises amino acid residues

-AA1-AA2-AA3-

wherein AA1 is bonded to the radical CT, i.e. to the toxophor, and the radical AA3 is bonded to the spacer unit Sp.

The radicals AA1 to AA3, each represent an amino acid in the D or L configuration. In this context, they are particularly preferably one of the naturally occurring amino acids glycine, alanine, valine, leucine, isoleucine, norvaline, serine, threonine, cysteine, methionine, aspartate, glutamate, asparagine, glutamine, arginine, lysine, histidine, tryptophan, phenylalanine, tyrosine or proline. The amino acids used in the process according to the invention can occur in the L or in the D configuration or alternatively as a mixture of D and L form. Preferred is the L-configuration.

The term "amino acids" refers, according to the invention, in particular to the $\alpha$-amino acids occurring in nature, but moreover also includes their homologues, isomers and derivatives. An example of isomers which can be mentioned is enantiomers. Derivatives can be, for example, amino acids provided with protective groups.

According to the present invention, the amino acids can each be linked to one another and to the toxophore or to the moiety addressing $\alpha_v\beta_3$ integrin receptors via their $\alpha$-carboxyl or $\alpha$-amino functions, but also via functional groups optionally present in side chains, such as, for example, amino functions.

In the case of amino acids having functional groups in the side chains, these functional groups can be either deblocked or protected by conventional protective groups used in peptide chemistry. Protective groups employed for these functional groups of the amino acids can be the protective groups known in peptide chemistry, for example of the urethane, alkyl, acyl, ester or amide type.

Amino protective groups in the context of the invention are the customary amino protective groups used in peptide chemistry. These preferably include: benzyloxycarbonyl, 3,4-dimethoxybenzyloxycarbonyl, 3,5-dimethoxybenzyloxycarbonyl, 2,4-dimethoxybenzyloxycarbonyl, 4-methoxybenzyloxycarbonyl, 4-nitrobenzyloxycarbonyl, 2-nitrobenzyloxycarbonyl, 2-nitro-4,5-dimethoxybenzyloxycarbonyl, methoxycarbonyl, ethoxycarbonyl, tert-butoxycarbonyl (Boc), allyloxycarbonyl, vinyloxycarbonyl, 3,4,5-trimethoxybenzyloxycarbonyl, phthaloyl, 2,2,2-trichloroethoxycarbonyl, 2,2,2-trichloro-tert-butoxycarbonyl, menthyloxycarbonyl, 4-nitrophenoxycarbonyl, fluorenyl-9-methoxycarbonyl (Fmoc), formyl, acetyl, propionyl, pivaloyl, 2-chloroacetyl, 2-bromoacetyl, 2,2,2-trifluoroacetyl, 2,2,2-trichloroacetyl, benzoyl, benzyl, 4-chlorobenzoyl, 4-bromobenzoyl, 4-nitrobenzoyl, phthalimido, isovaleroyl or benzyloxymethylene, 4-nitrobenzyl, 2,4-dinitrobenzyl, 4-nitrophenyl or 2-nitrophenylsulphenyl. The Fmoc group and the Boc group are particularly preferred.

The removal of protective groups in appropriate reaction steps can be carried out, for example, by the action of acid or base, hydrogenolytically or reductively in another manner.

According to the present invention, AA1 preferably is a naturally occurring amino acid in the D or L configuration, which is selected from the group consisting of glycine, alanine, valine, leucine, isoleucine, histidine, glutamate, aspartate, serine, lysine, ornithine and phenylalanine. According to the present invention, the L-configuration is preferred, AA1 most preferably is valine, isoleucine or leucine.

According to the present invention, AA2 preferably is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine. According to the present invention, the L-configuration is preferred, AA2 most preferably is proline.

According to the present invention, AA3 preferably is a naturally occurring amino acid in the D or L configuration, which is selected from the group consisting of alanine, valine, phenylalanine, tyrosine, serine, isoleucine, lysine, glutamate, glutamine, histidine, glycine, arginine, asparagine, aspartate, tryptophane, proline, ornithine, methionine, S-methyl-cysteine, norvaline and leucine. According to the present invention, the L-configuration is preferred, AA3 most preferably is alanine, norvaline, histidine, glycine, asparagine or aspartate.

It is preferred according to the invention that the linking unit consists of three amino acids AA1 to AA3 and the spacer unit Sp, it being possible, in particular, for the unit AA2 and AA3 to be modified on the side chain by protective groups. In these cases, the linkage to the toxophore as a rule takes place via the carboxyl function of the amino acid AA1 and the linkage to the moiety addressing $\alpha_v\beta_3$ integrin receptors via the spacer unit Sp takes place using an amino

group or hydroxyl group or a carboxy group of the moiety addressing $\alpha_v\beta_3$ integrin receptors.

In the case in which the linkage is to take place via a carboxyl function of the moiety addressing $\alpha_v\beta_3$ integrin receptors, it is preferred, however, to use linking units without the spacer unit Sp. In this case, the linkage between the linking unit and the moiety addressing $\alpha_v\beta_3$ integrin receptors takes place via an amino function of an amino acid.

The moiety addressing $\alpha_v\beta_3$ integrin receptors can be, for example, a radical of the formula (II):

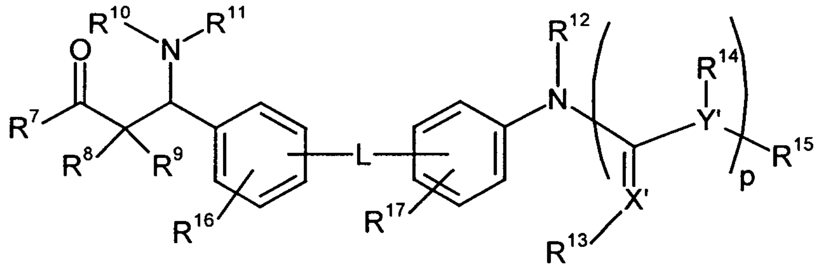

(II)

where the radicals in the formula (II) have the meaning defined above.

In the description below, bivalent substituents are indicated such that their respective left end is connected to the group indicated left of the corresponding substituent in formula (II) and their respective right end is connected to the group indicated right of the corresponding substituent in formula (II). If, for example, the radical L is equal to $-(CH_2)_mNHSO_2(CH_2)_n-$ in formula (II), the nitrogen atom is connected to the phenylene group found left of the radical L in formula (II) via the group $(CH_2)_m$. The following details additionally relate to the radical of the formula (II) in the unlinked state. The linkage of the radical of the formula (II) to the toxophore via the linking unit can take place either via the terminal carboxyl group, the terminal amino group, urea group, thiourea group, guanidine group or the group $NR^{12}CX'R^{13}S$- or via a functional group in the side chain of the radical of the formula (II), i.e. via the amino group or a substituent attached thereon in the β-position relative to the terminal carboxyl group, whereby in the linked state the terminal carboxyl group and the terminal amino group, urea group, thiourea group, guanidine group or the group $NR^{12}CX'R^{13}S$- are converted into corresponding bridging units.

The radicals of the formula (II) according to the invention are characterized in that they have, as a main structural element, two phenyl units connected via a linker group L, one phenylene group of which has a radical derived from a β-amino acid, while the other phenylene group has an amino group, urea group, thiourea group or guanidine group optionally incorporated into a cyclic ring system. The phenylene units connected via a linker group L can moreover carry further substituents in addition to the abovementioned radicals.

The terminal carboxyl units included in the radical derived from a β-amino acid can, if the linkage to the radical of the conjugate does not take place via this, be present as a free carboxylic acid or as an ester. In the case in which the terminal carboxyl unit is esterified, fundamentally all carboxylic acid esters obtainable by conventional processes, such as the corresponding alkyl esters, cycloalkyl esters, aryl esters and heterocyclic analogues thereof can be used according to the invention, where alkyl esters, cycloalkyl esters and aryl esters are preferred and the alcoholic radical can carry further substituents. Particularly preferred $C_{1-6}$-alkyl esters are those such as the methyl ester, ethyl ester, propyl ester, isopropyl ester, butyl ester, isobutyl ester, t-butyl ester, pentyl ester, isopentyl ester, neopentyl ester, hexyl ester, cyclopropyl ester, cyclopropylmethyl ester, cyclobutyl ester, cyclopentyl ester, cyclohexyl ester, or aryl esters such as the phenyl ester, benzyl ester or tolyl ester.

Preferably, the radicals of the formula (II) according to the invention are used in a form in which the terminal carboxyl unit is present as a free carboxylic acid.

The radical bonded to one of the two central phenylene units and derived from a β-amino acid can alternatively carry one or two additional substituents in the α-position relative to the carboxyl group. These substituents can each be selected from the group which consists of hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical. The alkyl radical can preferably be a $C_{1-6}$-alkyl such as, for example, methyl, ethyl, propyl, isopropyl, butyl, isobutyl, t-butyl, pentyl, isopentyl, neopentyl or hexyl. The cycloalkyl radical can preferably be a $C_{3-7}$-cycloalkyl such as, for example, cyclopropyl, cyclopropylmethyl, cyclobutyl, cyclopentyl, cyclohexyl or cycloheptyl. The aryl radical can preferably be phenyl, benzyl or tolyl. The heterocyclic radical can preferably be pyrrole, pyridine, tetrahydrofuran, furan, thiophene, tetrahydrothiophene, oxathiazole, benzofuran, quinoline, isoquinoline or pyrimidine. The alkenyl radical can be a terminal or internal E- or Z-alkene unit. The alkoxy radical can preferably be a $C_{1-6}$-alkoxy radical such as, for example, methoxy, ethoxy, propoxy, butoxy, pentoxy, hexoxy or benzyloxy. The abovementioned radicals can alternatively be substituted by one or more $C_{1-6}$-alkyl radicals such as methyl, ethyl, propyl, isopropyl, butyl, isobutyl, t-butyl, pentyl, isopentyl, neopentyl or hexyl, $C_{3-7}$-cycloalkyl radicals such as cyclopropyl, cyclopropylmethyl, cyclobutyl, cyclopentyl or cyclohexyl, aryl radicals such as phenyl, benzyl, tolyl, naphthyl, heterocyclic radicals such as pyrrole, pyridine, tetrahydrofuran, furan, thiophene, tetrahydrothiophene, oxazole, thiazole, oxathiazole, benzofuran, benzoxazole, benzothiazole, quinoline, isoquinoline, or functional groups such as a double bond to a heteroatom such as oxygen, sulphur or nitrogen, an optionally substituted amino group, a nitro group, a halogen group, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, benzofuranyl, benzoxazolyl, benzothiazolyl, quinolinyl or isoquinolinyl unit or a partially or completely hydrogenated analogue thereof.

Furthermore, the two substituents in the α-position relative to the terminal carboxyl group can, if present, be connected to one another and thus, together with the α-carbon atom of the radical derived from a β-amino acid, form a carbocyclic or heterocyclic ring system. This ring system can optionally carry further substituents and/or contain further heteroatoms. According to the invention, the above ring system, if present, is preferably a 3- to 6-membered carbocyclic or heterocyclic ring system such as, for example, a cyclopropane ring, cyclobutane ring, cyclopentane ring, cyclohexane ring, dihydrofuran ring, tetrahydrofuran ring, dihydropyran ring, tetrahydropyran ring, dioxane ring, dihydrothiophene ring, tetrahydrothiophene ring or a substituted derivative thereof.

In the groups according to the invention, the amino group included in the radical derived from a β-amino acid, if the linkage to the rest of the conjugate does not take place via this, is substituted by one of the radicals $-SO_2R^{10'}$, $-COOR^{10''}$, $-COR^{10'}$, $-CONR^{10'}_2$ or $-CSNR^{10'}_2$, where $R^{10'}$ can be hydrogen, a substituted or unsubstituted alkyl or cycloalkyl radical, a substituted or unsubstituted aryl radical or a saturated or unsaturated, optionally substituted heterocyclic radical and $R^{10''}$ can be a substituted or unsubstituted alkyl or cycloalkyl radical, a substituted or unsubstituted aryl radical or a saturated or unsaturated, optionally substituted heterocyclic radical. Preferably, the alkyl radical in this case is a $C_{1-6}$-alkyl such as, for example, methyl, ethyl, propyl, isopropyl, butyl, isobutyl, t-butyl, pentyl, isopentyl, neopentyl, hexyl, the cycloalkyl radical is a $C_{3-7}$-cycloalkyl such as, for example, cyclopropyl, cyclobutyl, cyclopentyl, cyclohexyl, cycloheptyl, the aryl radical is an aryl such as phenyl, benzyl, tolyl or a substituted derivative thereof such as $-C_6H_2(CH_3)_3$, $-C_6(CH_3)_5$, $-CH_2C_6H_2(CH_3)_3$, 3-aminophenyl, 4-aminophenyl, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonylpiperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl.

According to the invention, the amino group included in the radical derived from a β-amino acid is particularly preferably substituted by $-SO_2R^{10'}$, $-COOR^{10''}$, $-CONR^{10'}_2$ or $-COR^{10'}$, where $R^{10'}$ and $R^{10''}$ are as defined above. In particular, radicals of the formula (II) are preferred here in which the radical derived from a β-amino acid has no substituent in the α-position relative to the carboxyl unit and the amino group included in this radical is substituted by $-SO_2R^{10'}$, $-CONR^{10'}_2$ or $-COR^{10'}$, where $R^{10'}$ is as defined above.

In addition to one of the abovementioned radicals, the nitrogen atom of the amino group found in the β-position can have a substituent which is selected from the group consisting of hydrogen, a substituted or unsubstituted alkyl or cycloalkyl radical, a substituted or unsubstituted aryl radical, a saturated or unsaturated, optionally substituted heterocyclic radical or are bonded to one another and thus, together with the nitrogen atom to which they are bonded, form a heterocyclic ring system. Preferred substituents here are those which can be selected from the group consisting of hydrogen, a $C_{1-6}$-alkyl such as, for example, methyl, ethyl, propyl, isopropyl, butyl, isobutyl, t-butyl, pentyl, isopentyl, neopentyl or hexyl, a $C_{3-7}$-cycloalkyl such as, for example, cyclopropyl, cyclopropylmethyl, cyclobutyl, cyclopentyl, cyclohexyl or cycloheptyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine and can alternatively be substituted by one or more $C_{1-6}$-alkyl radicals such as methyl, ethyl, propyl, isopropyl, butyl, isobutyl, t-butyl, pentyl, isopentyl, neopentyl or

hexyl, $C_{3-7}$-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof. The additional substituent on the nitrogen atom of the β-amino group is particularly preferably hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl,

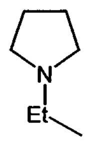 , 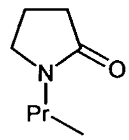 , 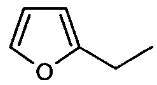 , 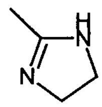 , 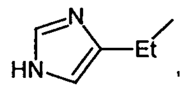 ,

(a1) (a2) (a3) (a4) (a5)

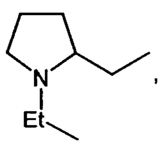 , 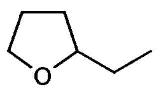 , 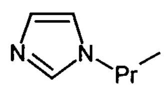 , 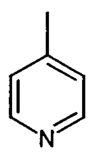 , 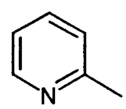 ,

(a6) (a7) (a8) (a9) (a10)

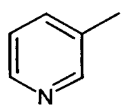 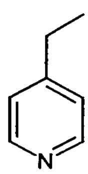 , 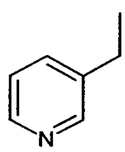 , 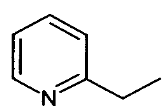 , 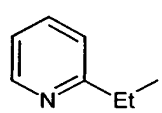 ,

(a11) (a12) (a13) (a14) (a15)

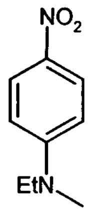 , 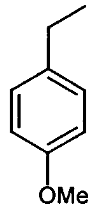 , 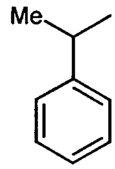 , 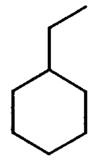 , 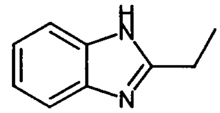

(a16) (a17) (a18) (a19) (a20)

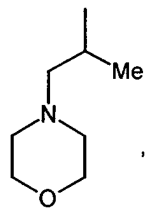 , 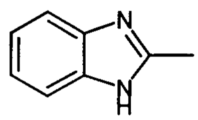 , 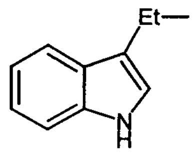 , 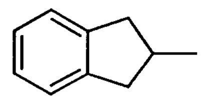 ,

(a21) (a22) (a23) (a24)

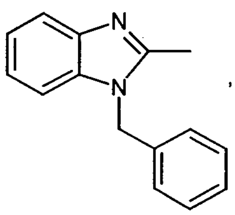 , 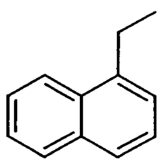 , 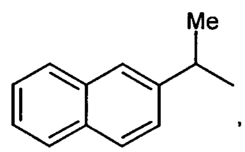 , 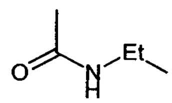 ,

(a25) (a26) (a27) (a28)

The radical derived from a β-amino acid is bonded to one of the two central phenylene units connected via a linker group L, which is to be designated here as phenylene unit A. In addition to the radical derived from a β-amino acid and the linker group L, the phenylene unit A preferably carries no further substituents, but can have one or more radicals which are selected from the group consisting of hydrogen, CN, a substituted or unsubstituted alkyl or cycloalkyl radical, a substituted or unsubstituted alkoxy radical or a halogen atom. The alkyl radical(s) is/are preferably $C_{1-6}$-alkyl radicals such as methyl, ethyl, propyl, isopropyl, butyl, isobutyl, t-butyl, pentyl, isopentyl, neopentyl, hexyl. The cycloalkyl radical(s) is/are preferably $C_{3-7}$-cycloalkyl radicals such as cyclopropyl, cyclobutyl, cyclopentyl, cyclohexyl, cycloheptyl. The alkoxy radical(s) is/are preferably $C_{1-6}$-alkoxy radicals such as methoxy, trifluoromethoxy, ethoxy, propoxy, butoxy, pentoxy or hexoxy, and the halogen atom(s) is/are preferably F, Cl, Br or I.

With respect to the linker group L and the radical derived from a β-amino acid or the amino, guanidine, urea or thiourea unit, the two central phenylene units can be 1,3-or 1,4-linked, i.e. the radical derived from a β-amino acid and the linker group L can be substituted in the meta- or para-position relative to one another in the phenylene unit A, and at the same time the linker group L and the amino, guanidine, urea or thiourea unit in the phenylene unit B can be substituted in the meta- or para-position relative to one another, where each combination of the abovementioned substitution patterns is possible for the'central A-linker L-phenylene B unit of the radicals of the formula (II) according to the invention. Particularly preferred according to the present invention are those radicals of the formula (II1) whose central phenylene A-linker L-phenylene B unit consists according to the above definition of a p-substituted phenylene unit A and a p-substituted phenylene unit B, a p-substituted phenylene unit A and an m-substituted phenylene unit B, an m-substituted phenylene unit A and a p-substituted phenylene unit B or an m-substituted phenylene unit A and an m-substituted phenylene unit B. Particularly preferred according to the present invention are radicals of the formula (II) whose central phenylene A-linker L-phenylene B unit consists according to the present definition of an m-substituted phenylene unit A and an m-substituted phenylene unit B.

According to the present invention, the linker group L is selected from the group which consists of the elements $-(CH_2)_mNHSO_2(CH_2)_n-$, $-(CH_2)_mSO_2NH(CH_2)_n-$, $-(CH_2)_mNHCO(CH_2)_n-$, $-(CH_2)_mCONH(CH_2)_n-$, $-(CH_2)_mOCH_2(CH_2)_n-$, $-(CH_2)_mCH_2O(CH_2)_n-$, $-(CH_2)_mCOO(CH_2)_n-$, $-(CH_2)_mOOC(CH_2)_n-$, $-(CH_2)_mCH_2CO(CH_2)_n-$, $-(CH_2)_mCOCH_2(CH_2)_n-$, -NHCONH-, $-(CH_2)_mSCH_2(CH_2)_n-$, $-(CH_2)_mCH_2S(CH_2)_n-$, $-(CH_2)_mCH_2SO(CH_2)_n-$, $-(CH_2)_mSOCH_2(CH_2)_n$, $-(CH_2)_mCH_2SO_2(CH_2)_n-$ or $-(CH_2)_mSO_2CH_2(CH_2)_n-$,
where m and n each are an integer of 0 or 1 and $m + n \leq 1$.

According to the invention, the linker group L is preferably $-NHSO_2-$, $-CH_2NHSO_2-$, $-NHSO_2CH_2-$, $-SO_2NH-$, $-CH_2SO_2NH-$, $-SO_2NHCH_2-$, -NHCO-, $-CH_2NHCO-$, $-NH-COCH_2-$, -CONH-, $-CH_2CONH-$, $-CONHCH_2-$, $-OCH_2-$, $-CH_2OCH_2$, $-OCH_2CH_2-$, $-CH_2O-$, $-CH_2CH_2O-$, -COO-, $-CH_2COO-$, $-COOCH_2-$, -OOC-, $-OOCCH_2-$, $-CH_2OOC-$, $-CH_2CO-$, $-COCH_2-$, $-CH_2CH_2CO-$, $-COCH_2CH_2-$, $-CH_2COCH_2-$, -NHCONH-, $-SCH_2-$, $-CH_2S-$, $-CH_2SCH_2$, $-SCH_2CH_2-$, $CH_2CH_2S-$, $-SOCH_2-$, $-CH_2SO-$, $-CH_2SOCH_2-$, $-SOCH_2CH_2-$, $-CH_2CH_2SO-$, $-SO_2CH_2-$, $-CH_2SO_2-$, $-CH_2SO_2CH_2-$, $-CH_2CH_2-SO_2-$ or $-SO_2CH_2CH_2-$. Particularly preferred linker groups L here are $-NHSO_2-$, $-CH_2NHSO_2-$, $-NHSO_2CH_2-$, $-SO_2NH-$, $-CH_2SO_2NH-$, $-SO_2NHCH_2-$, -NHCO-, $-CH_2NHCO-$, $-NHCOCH_2-$, -CONH-, $-CH_2CONH-$, $-CONHCH_2-$, $-OCH_2-$, $-CH_2OCH_2$, $-OCH_2CH_2-$, $-CH_2O-$ or $-CH_2CH_2O-$.

The central phenylene unit B carries as a substituent a radical which, if the linkage to the radical of the conjugate does not take place via this, is selected from the group consisting of a group $NR^{12}CX'R^{13}S-$, an amino, guanidine, urea or thiourea unit. This group $NR^{12}CX'R^{13}S-$, amino, guanidine, urea or thiourea unit can be either open-chain or a constituent of a cyclic system. The nitrogen atoms of the respective unit, which are optionally both present and bonded only via single bonds, can carry additional substituents $R^{12}$, $R^{14}$ and $R^{15}$. These substituents can independently of one another or simultaneously be hydrogen, a substituted or unsubstituted alkyl or cycloalkyl radical, a substituted or unsubstituted aryl radical, a saturated or unsaturated, optionally substituted heterocyclic radical or can be bonded to one another and thus, together with the nitrogen atom(s) to which they are bonded, form a heterocyclic ring system. Preferred substituents here are those which are selected from the group consisting of hydrogen, a $C_{1-6}$-alkyl such as, for example, methyl, ethyl, propyl, isopropyl, butyl, isobutyl, t-butyl, pentyl, isopentyl, neopentyl or hexyl, a $C_{3-7}$-cycloalkyl such as, for example, cyclopropyl, cyclopropylmethyl, cyclobutyl, cyclopentyl, cyclohexyl or cycloheptyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine and can alternatively be substituted by one or more $C_{1-6}$-alkyl radicals such as methyl, ethyl, propyl, isopropyl, butyl, isobutyl, t-butyl, pentyl, isopentyl, neopentyl or hexyl, $C_{3-7}$-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof. Particularly preferred substituents are those such as hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl or one of the abovementioned radicals (a1) to (a28). If the linkage of the radical of the formula (II) to the rest of the conjugate takes place via this group, the radical $R^{15}$ represents a direct bond via which the corresponding linkage between the radical of the formula (II) and the rest of the conjugate takes place.

The two radicals $R^{14}$ and $R^{15}$ or the radicals $R^{12}$ and $R^{15}$, if p in the formula (II) represents 0, can be connected to one another and thus with the nitrogen atom form a heterocyclic ring system which can be selected, for example, from the following, non-exclusive list:

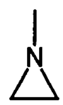 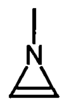 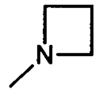 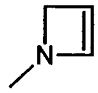 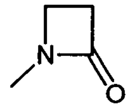 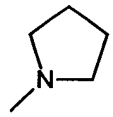

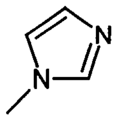 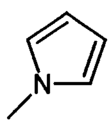 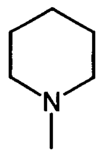 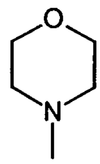 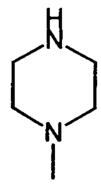

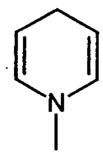 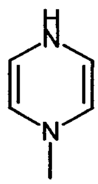 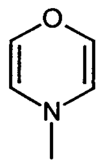 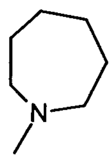

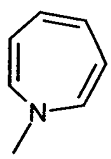 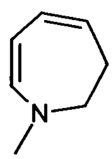

where the ring systems shown can carry one or more radicals which are selected from the group consisting of hydrogen, a $C_{1-6}$-alkyl such as, for example, methyl, ethyl, propyl, isopropyl, butyl, isobutyl, t-butyl, pentyl, isopentyl, neopentyl or hexyl, a $C_{3-7}$-cycloalkyl such as, for example, cyclopropyl, cyclopropylmethyl, cyclobutyl, cyclopentyl or cyclohexyl, an aryl such as, for example, phenyl, benzyl or tolyl, a heterocyclic radical such as, for example, pyrrolidine, piperidine, piperazine, pyrrole, pyridine, tetrahydrofuran, furan, thiophene, tetrahydrothiophene, imidazolidine, imidazole, oxazolidine, oxazole, thiazolidine, thiazole, oxathiazole, benzofuran, benzoxazole, benzothiazole, benzimidazole, quinoline, isoquinoline, tetrahydroquinoline, tetrahydroisoquinoline, triazole, tetrazole, pyrimidine, purine, cytosine, thymine, uracil, adenine, guanine or xanthine, or a terminal or internal E- or Z-alkene unit, and can alternatively be substituted by one or more $C_{1-6}$-alkyl radicals such as methyl, ethyl, propyl, isopropyl, butyl, isobutyl, t-butyl, pentyl, isopentyl, neopentyl or hexyl, $C_{3-7}$-cycloalkyl radicals such as cyclopropyl, cyclopropylmethyl, cyclobutyl, cyclopentyl or cyclohexyl, aryl radicals such as phenyl, benzyl, tolyl, naphthyl, indolyl, heterocyclic radicals such as pyrrolidine, piperidine, piperazine, pyrrole, pyridine, tetrahydrofuran, furan, thiophene, tetrahydrothiophene, imida-zolidine, imidazole, oxazolidine, oxazole, thiazolidine, thiazole, oxathiazole, benz-ofuran, benzoxazole, benzothiazole, benzimidazole, quinoline, isoquinoline, tetra-hydroquinoline, tetrahydroisoquinoline, triazole, tetrazole, pyrimidine, purine, cytosine, thymine, uracil, adenine, guanine or xanthine, or functional groups such as a double bond to a heteroatom such as oxygen, sulphur or nitrogen, an optionally substituted amino group, a nitro group, a halogen, a hydroxyl group, an ether group, a sulphide group, a mercaptan group, a cyano group, an isonitrile group, an alkenyl group, an alkinyl group, an aldehyde group, a keto group, a carboxyl group, an ester group, an amide group, a sulphoxide group or a sulphone group. Furthermore, one or more additionally saturated or unsaturated rings can be fused to the abovementioned cyclic radicals with formation of, for example, a naphthyl, indolyl, benzofuranyl, benzoxazolyl, benzothiazolyl, benzimidazolyl, quinolinyl or isoquinolinyl unit or a partially or completely hydrogenated analogue thereof.

Of the ring systems shown above, the four- to six-membered ring systems are preferred.

As mentioned above, the group $NR^{12}CX'R^{13}S$-, the amino, urea, thiourea or guanidine unit can be open-chain or incorporated into a cyclic system and thus be a constituent of one of the following preferred functional units:

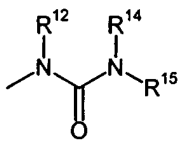 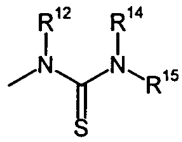 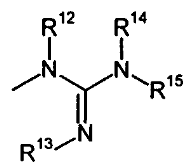 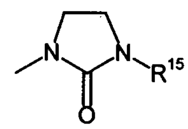

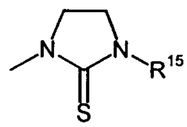 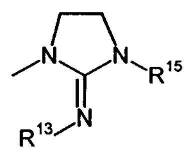 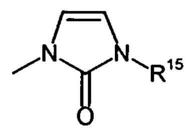 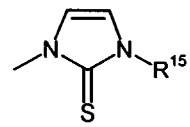

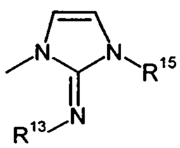 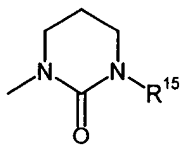 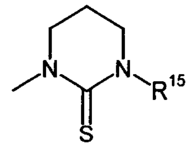 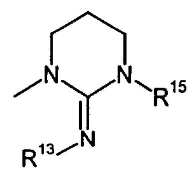

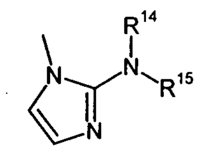 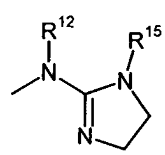 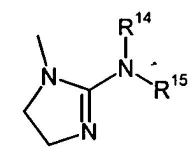

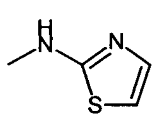 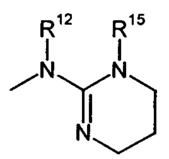 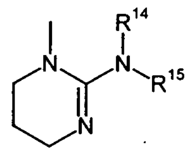

where the above list represents a non-exclusive enumeration of all possible structural units.

According to the invention, in addition to the abovementioned preferred structural units, their analogues are also included in which one or more 4- to 6-membered ring systems are fused to the heterocycle, such as, for example, the corresponding benzo-fused analogues of the above structural units.

In the structural units shown above, $R^{12}$, $R^{14}$ and $R^{15}$ are as defined above.

Furthermore, in the above structural units $R^{13}$ can be absent, hydrogen, a substituted or unsubstituted alkyl or cycloalkyl radical such as, for example, a $C_{1-6}$-alkyl such as methyl, ethyl, propyl, isopropyl, butyl, isobutyl, t-butyl, pentyl, isopentyl, neopentyl, hexyl or a $C_{3-7}$-cycloalkyl such as cyclopropyl, cyclobutyl, cyclopentyl, cyclohexyl, $-NO_2$, -CN, $-COR^{13'}$ or $-COOR^{13'}$, where $R^{13'}$ can be hydrogen, a substituted or unsubstituted alkyl or cycloalkyl radical, a substituted or unsubstituted aryl radical or a saturated or unsaturated, optionally substituted heterocyclic radical, which can be saturated or unsaturated and/or can contain further heteroatoms, and is preferably a $C_{1-6}$-alkyl such as, for example, methyl, ethyl, propyl, isopropyl, butyl, isobutyl, t-butyl, pentyl, isopentyl, neopentyl, hexyl, a $C_{3-7}$-cycloalkyl such as, for example, cyclopropyl, cyclobutyl, cyclopentyl, cyclohexyl, an aryl such as, for example, phenyl, benzyl, tolyl or a substituted derivative.

According to the invention, particularly preferred radicals of the formula (II) are those in which the amino group included in the radical derived from a β-amino acid carries a radical-$SO_2R^{10'}$, where $R^{10'}$ is preferably methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, $-C_6H_2(CH_3)_3$, $-C_6(CH_3)_5$, $-CH_2C_6H_2$

$(CH_3)_3$, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoro-methyl-phenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonylpiperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is $-NHSO_2-$, $-CH_2NHSO_2-$, $-NHSO_2CH_2-$, and the radical found on the phenylene unit is an open-chain or cyclic guanidine unit, a cyclic guanidine unit such as, for example, a 4,5-dihydro-1H-imidazol-2-ylamino unit being particularly preferred.

Furthermore, according to the present invention radicals of the formula (II) are particularly preferred in which the amino group included in the radical derived from a β-amino acid carries a radical-$SO_2R^{10'}$ or a radical $-COOR^{10''}$, where $R^{10'}$ or $R^{10''}$ is preferably methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, $-C_6H_2(CH_3)_3$, $-C_6(CH_3)_5$, $-CH_2C_6H_2(CH_3)_3$, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chloro-phenyl-methyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoro-methyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetra-methylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methyl-benzothiazol-2-yl, N-methoxycarbonyl-piperidin-3-yl, thiophen-2-yl, isoxazol-5-yl, ethoxy, 2-chloro-pyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is $-NHSO_2-$, $-CH_2NH\ SO_2-$, $-NHSO_2CH_2-$ or $-OCH_2-$, $-CH_2O-$, $-CH_2OCH_2-$, $-CH_2CH_2O-$, $-OCH_2CH_2-$, and the radical found on the phenylene unit is an open-chain or cyclic guanidine unit, a cyclic guanidine unit such as, for example, a 4,5-dihydro-1H-imidazol-2-ylamino unit being particularly preferred.

Moreover, according to the present invention radicals of the formula (II) are particularly preferred in which the amino group included in the radical derived from a β-amino acid carries a radical-$COR^{10'}$, where $R^{10'}$ is preferably hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, $-C_6H_2(CH_3)_3$, $-C_6(CH_3)_5$, $-CH_2C_6H_2(CH_3)_3$, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoro-methyl-phenyl, 2-alkylsulphonylphenyl, 2-arylsulphonylphenyl, 3-(N-acetyl-6-methoxy)-aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is $-NHSO_2-$, $-CH_2NHSO_2-$, $-NHSO_2CH_2-$, and the radical found on the phenylene unit is an open-chain or cyclic guanidine unit, a cyclic guanidine unit such as, for example, a 4,5-dihydro-1H-imidazol-2-ylamino unit being particularly preferred.

Moreover, according to the present invention radicals of the formula (II) are particularly preferred in which the amino group included in the radical derived from a β-amino acid carries a radical $-COR^{10'}$, where $R^{10'}$ is preferably hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, $-C_6H_2(CH_3)_3$, $-C_6$

$(CH_3)_5$, $-CH_2C_6H_2(CH_3)_3$, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 3-aminophenyl, 4-aminophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoro-methyl-phenyl, 2-alkylsulphonylphenyl, 2-arylsulphonylphenyl, 3-(N-acetyl-6-methoxy)-aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl or 8-quinolinyl, the linker group L is $-NHSO_2-$, $-CH_2NHSO_2-$, $-NHSO_2CH_2-$, and the radical found on the phenylene unit is an open-chain or cyclic guanidine unit, a cyclic guanidine unit such as, for example, a 4,5-dihydro-1H-imidazol-2-ylamino unit being particularly preferred.

The novel conjugates according to Claim 1 can be prepared by linkage of the toxophore to the linking unit and subsequent linkage to the moiety addressing $\alpha_v\beta_3$ integrin receptors. However, it is also possible to first connect the moiety addressing $\alpha_v\beta_3$ integrin receptors to the linking unit and then to bind the toxophore to the linking unit.

The combination of the individual units of the conjugates according to the invention can preferably be carried out by means of functional groups which can be reacted with one another and, as a result, can be linked by conventional processes known to the person skilled in the art. For example carboxyl functions can be reacted with amino functions with formation of an amide bond. It is also possible to synthesize the linking unit stepwise on one of the two radicals to be connected, i.e. the toxophore or the moiety addressing $\alpha_v\beta_3$ integrin receptors, by conventional processes known to the person skilled in the art and then to link the finished linking unit to the radical which is still to be bound.

The present invention in particular relates to a process for the preparation of conjugates according to formula (I),
comprising

[A] the reaction of a compound from the group of compound of the formula (II) which has a free or optionally activated carboxyl function,
with a compound of the formula (Ia) which has a free primary or secondary amino group

$$CT\text{-}LI \qquad (Ia)$$

in which all radicals have the meaning indicated in Claim 1,
in the presence of a base;

or

[B] the reaction of a compound from the group of compounds of the formula (II) which has a free primary or secondary amino function,
with a carbonic acid derivative such as, for example, phosgene, thiophosgene or a chloroformic acid ester, if appropriate in the presence of a base,
followed by the reaction with a compound of the formula (Ia) which has a free primary or secondary amino group

$$CT\text{-}LI \qquad (Ia)$$

in which all radicals have the meaning indicated in Claim 1,
and
if appropriate the removal of protective groups and/or derivatization of nitrogen atoms present at preferred points of time in the preparation process and/or conversion of the compound obtained into the free acid
and/or conversion of the compound obtained into one of its physiological salts by reaction with an inorganic or organic base or acid;

or

[C] the reaction of a compound from the group of compounds of the formula (II) which contains a free primary or

secondary amino function,

with a compound of the formula (Ia) which contains a free or optionally activated carboxyl function

CT-LI (Ia)

in which all radicals have the meaning indicated in Claim 1,

in the presence of a base;

and

if appropriate the removal of protective groups and/or derivatization of nitrogen atoms present at preferred points in time in the preparation process and/or conversion of the compound obtained into the free acid and/or conversion of the compound obtained into one of its physiological salts by reaction with an inorganic or organic base or acid.

According to a preferred embodiment, several steps of the preparation process are carried out on a solid phase.

In variant [A] of the preparation process according to the invention, a moiety addressing $\alpha_v\beta_3$ integrin receptors from the group of radicals of the formula (II) is linked via its free carboxyl function to the amino function of a toxophore-linking unit conjugate (Ia) with formation of an amide bond. This reaction can be carried out by conventional methods known to the person skilled in the art (cf., for example, J. March, Advanced organic chemistry, 3rd ed., Wiley, p. 370 ff.). It is preferred according to the invention to activate the carboxyl function of the moiety addressing $\alpha_v\beta_3$ integrin receptors and then to react with the compound (Ia) in an organic solvent in the presence of a base.

For the activation of the carboxyl group, the coupling reagents known in peptide chemistry can be used, such as are described, for example, in Jakubke/Jeschkeit: Aminosäuren, Peptide, Proteine [Amino acids, Peptides, Proteins]; Verlag Chemie 1982 or Tetrahedr. Lett. 34, 6705 (1993). Examples mentioned are N-carboxylic acid anhydrides, acid chlorides or mixed anhydrides, adducts with carbodiimides, e.g. N,N'-diethyl-, N,N'-diisopropyl- or N,N'-dicyclohexylcarbodiimide, N-(3-dimethylaminopropyl)N'-ethyl-carbodiimide hydrochloride, N-cyclohexyl-N'-(2-morpholinoethyl)-carbodiimide metho-p-toluenesulphonate, or carbonyl compounds such as carbonyldiimidazole, or 1,2-oxazolium compounds such as 2-ethyl-5-phenyl-1,2-oxazolium-3-sulphate or 2-tert-butyl-5-methyl-isoxazolium perchlorate, or acylamino compounds such as 2-ethoxy-1-ethoxycarbonyl-1,2-dihydroquinoline or propane-phosphonic anhydride or isobutyl chloroformate or benzotriazolyloxy-tris-(dimethylamino)-phosphonium hexafluorophosphate, 1-hydroxybenzotriazole or N-hydroxy-succinimide esters. It is furthermore proposed to employ the acid components in the form of a Leuchs' anhydride.

Variant [A] of the above preparation process according to the invention can be carried out under various pressure and temperature conditions, for example 0.5 to 2 bar and preferably under normal pressure, or -30 to +100°C and preferably -10 to +80°C, in suitable solvents such as dimethylformamide (DMF), tetrahydrofuran (THF), dichloromethane, chloroform, lower alcohols, acetonitrile, dioxane, water or in mixtures of the solvents mentioned. As a rule, reaction in DMF, dichloromethane, THF, dioxane/water or THF/dichloromethane at room temperature or with icecooling and under normal pressure is preferred.

Bases which can be employed in variant [A] of the preparation process according to the invention are, for example, triethylamine, ethyl-diisopropylamine, pyridine, N,N-dimethylaminopyridine or other bases conventionally used in steps of this type.

In variant [B] of the process according to the invention, a moiety addressing $\alpha_v\beta_3$ integrin receptors from the group of radicals of the formula (II) is reacted via its free amino function first with a carbonic acid derivative with formation of a corresponding isocyanate, isothiocyanate or carbamate, which is then linked to the amino function of a toxophore-linking unit conjugate (Ia) with formation of the conjugate (I).

The reaction of the moiety addressing $\alpha_v\beta_3$ integrin receptors from the group of radicals of the formula (II) via its free amino function with a carbonic acid derivative can be carried out by conventional methods known to the person skilled in the art (cf., for example, J. March, Advanced organic chemistry, 3rd ed., Wiley, p. 370 ff.). According to the invention, the reaction is preferably carried out with phosgene or a substitute for phosgene such as, for example, trichloromethyl chloroformate, thiophosgene or a chloroformic acid ester such as chloroformic acid-p-nitrophenylester in a solvent such as dimethylformamide (DMF) or a mixture of dioxane and water (1:1) or of tetrahydrofuran (THF) and dichloromethane (DCM) (1:1) at room - temperature or with cooling, preferably at room temperature, and stirring for approximately 10 minutes up to approximately 3 hours, if appropriate in the presence of a base.

The subsequent reaction of the isocyanate, isothiocyanate or carbamate thus obtained with the amino function of a toxophore-linking unit conjugate (Ia) with formation of a corresponding thiourea or urea bond can be carried out by conventional methods known to the person skilled in the art (cf., for example, J. March, Advanced organic chemistry, 3rd ed., Wiley, p. 802 ff.).

According to the invention, the carbamate or thiocyanate or isothiocyanate is preferably reacted with the amino function of the compound (Ia) at room temperature with stirring for approximately 1 to 5 hours, preferably approximately 2 to 3 hours, in the presence of a base in a solvent such as dimethylformamide (DMF).

Bases which can be employed in variant [B] of the preparation process according to the invention are, for example, triethylamine, ethyldiisopropylamine, pyridine, N,N-dimethylaminopyridine or other bases conventionally used in steps of this type.

In variant [C] of the preparation process according to the invention, a moiety addressing $\alpha_v\beta_3$ integrin receptors from the group of radicals of the formula (II) is linked via its free amino function to the carboxyl function of a toxophore-linking unit conjugate (Ia) with formation of an amide bond. This reaction can be carried out by conventional methods known to the person skilled in the art (cf., for example, J. March, Advanced organic chemistry, 3rd ed., Wiley, p. 370 ff.). It is preferred according to the invention to activate the carboxyl function of the compound (Ia) and then to react it with a moiety addressing $\alpha_v\beta_3$ integrin receptors from the group of radicals of the formula (II) in an organic solvent in the presence of a base.

For activation of the carboxyl group, the coupling reagents known in peptide chemistry can be used, such as are described, for example, in Jakubke/Jeschkeit: Aminosäuren, Peptide, Proteine [Amino acids, Peptides, Proteins]; Verlag Chemie 1982 or Tetrahedr. Lett. 34, 6705 (1993). Examples mentioned are N-carboxylic anhydrides, acid chlorides or mixed anhydrides, adducts with carbodiimides, e.g. N,N'-diethyl-, N,N'-diisopropyl- or N,N'-dicyclohexylcarbodiimide, N-(3-dimethylaminopropyl)-N'-ethyl-carbodiimide hydrochloride, N-cyclohexyl-N'-(2-morpholinoethyl)-carbodiimide metho-p-toluenesulphonate, or carbonyl compounds such as carbonyldiimidazole, or 1,2-oxazolium compounds such as 2-ethyl-5-phenyl-1,2-oxazolium-3-sulphate or 2-tert-butyl-5-methyl-isoxazolium perchlorate, or acylamino compounds such as 2-ethoxy-1-ethoxycarbonyl-1,2-dihydroquinoline, or propane-phosphonic anhydride, or isobutyl chloroformate, or benzotriazolyloxy-tris-(dimethylamino)-phosphonium hexafluorophosphate, 1-hydroxybenzotriazole or N-hydroxysuccinimide esters. It is furthermore proposed to employ the acid components in the form of a Leuchs' anhydride.

Variant [C] of the above preparation process according to the invention can be carried out under various pressure and temperature conditions, for example 0.5 to 2 bar and preferably under normal pressure, or -30 to +100°C and preferably -10 to +80°C, in suitable solvents such as dimethylformamide (DMF), tetrahydrofuran (THF), dichloromethane, chloroform, lower alcohols, acetonitrile, dioxane, water or in mixtures of the solvents mentioned. As a rule, reaction in DMF, dichloromethane, THF, dioxane/water or THF/dichloromethane at room temperature or with icecooling and at normal pressure is preferred.

Bases which can be employed in variant [C] of the preparation process according to the invention are, for example, triethylamine, ethyldiisopropylamine, pyridine, N,N-dimethylaminopyridine or other bases conventionally used in steps of this type such as, for example, Hünig's base.

The compounds obtained according to the process explained above can furthermore be derivatized by removal of protective groups which may be present, further substitution of nitrogen atoms present at preferred positions in the preparation process and/or conversion of the compound obtained into the free acid and/or its physiologically acceptable salts. By way of example, the t-butoxymethoxycarbonyl groups conventionally used as protective groups for nitrogen atoms are removed in acidic medium, for example by addition of trifluoroacetic acid. Suitable alkylating agents for the derivatization of nitrogen atoms in this step are reagents conventionally used for this purpose, using which, for example, a substituted or unsubstituted alkyl or cycloalkyl radical, a substituted or unsubstituted aryl radical or a saturated or unsaturated, optionally substituted heterocyclic radical can be bonded to the appropriate nitrogen atom. With respect to the substituents preferably bonded to the respective nitrogen atoms, reference is made to the above description of the compounds according to the invention. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

The ester derivatives according to the invention can be converted into the corresponding free carboxylic acids in a conventional manner, such as, for example, by basic ester hydrolysis.

If desired, the compounds according to the invention can be converted into their physiologically acceptable salts. This can be carried out either by reaction with an organic or inorganic base such as, for example, an alkali metal hydroxide or alkaline earth metal hydroxide such as KOH, NaOH, LiOH, $Mg(OH)_2$ or $Ca(OH)_2$, as a result of which the terminal carboxyl group is deprotonated and the corresponding carboxylate is formed, or by reaction with an organic or inorganic acid such as, for example, hydrochloric acid, sulphuric acid, phosphoric acid, mandelic acid, oleic acid, linoleic acid or p-toluenesulphonic acid, as a result of which one or more of the nitrogen atoms present are protonated.

The compounds of the formula (Ia) serving as starting substances can be prepared by conventional methods. The linkage of the toxophore to amino acid units can be carried out by conventional methods of peptide chemistry (cf., for example, Jakubke/Jeschkeit: Aminosäuren, Peptide, Proteine [Amino acids, Peptides, Proteins]; Verlag Chemie 1982, Houben-Weyl, Methoden der Organischen Chemie [Methods of Organic Chemistry], Georg Thieme Verlag Stuttgart, Fourth Edition; Volume 15.1 and 15.2, edited by E.Wünsch) and is also described, for example, in WO 96/31532

and WO 98/51703, whose contents are inserted here by means of reference.

The bonding of the appropriate carbonyl or thiocarbonyl radicals can be carried out as described above by reaction of the toxophore or of the toxophore-amino acid conjugate with phosgene or a substitute for phosgene such as, for example, trichloromethyl chloroformate or thiophosgene.

Although according to the invention it is preferred to first synthesize the toxophore-linking unit conjugate (Ia), it is also possible, of course, to build up the linking unit in series first on the moiety addressing $\alpha_v\beta_3$ integrin receptors or to bond it as a whole and then to connect the conjugate thus obtained to the toxophore.

According to a preferred embodiment of the present invention, the synthesis of the compounds according to the invention is partly carried out on a solid phase such as a polystyrene resin, particularly preferably a commercially available Wang polystyrene resin. The resin is in this case first swollen in a solvent such as dimethylformamide (DMF). The moiety of the formula (II) addressing $\alpha_v\beta_3$ integrin receptors is then bonded to the resin via its carboxyl function by standard processes. For example, the bonding of the carboxylic acid to the resin can be carried out in the presence of a base such as pyridine and a reagent activating the carboxyl unit, such as an acid halide, for example dichlorobenzoyl chloride, in a solvent such as dimethylformamide (DMF). However, other reagents conventionally used for this purpose can also be employed. The reaction mixture is stirred at room temperature and normal pressure for at least 2 hours, preferably 12 hours, particularly preferably approximately 24 hours, the carboxylic acid being employed in an excess with respect to the loading of the solid phase, preferably in a two- to three-fold excess. All reactions described herein can then be carried out on the moiety of the formula (II) bound to the resin and addressing $\alpha_v\beta_3$ integrin receptors, as described here.

According to a preferred embodiment of the present invention, the toxophore is camptothecin or a camptothecin derivative. The linkage of these toxophores to the linking unit can be carried out via the C20 OH group or other functional groups in the molecule.

The camptothecin unit used as a starting compound can be present in the 20(R) or in the 20(S) configuration or as a mixture of these two stereoisomeric forms. The 20(S) configuration is preferred.

After linkage of the first amino acid to camptothecin, diastereomer mixtures can be formed. Pure diastereomers of the compounds according to the invention can be prepared by the processes indicated above, for example, by separating the diastereomers in a suitable manner after coupling of the first amino acid unit to the camptothecin and subsequent protective group removal.

The radical of the formula (II) addressing $\alpha_v\beta_3$ integrin receptors can be prepared from commercially obtainable starting compounds by the following steps.

The essential steps of the preparation process according to the invention are the reaction of a β-amino acid of the formula (IIa)

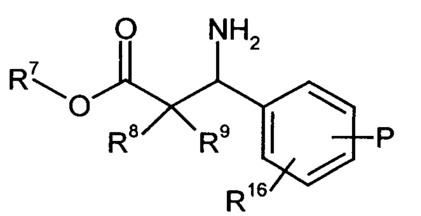

(IIa)

where

P is $-(CH_2)_mNO_2$, $-(CH_2)_mO-C_{1-6}$-alkyl, $-(CH_2)_mSO_2P'$, $-(CH_2)_mCOP'$, $-(CH_2)_mCH_2O-C_{1-6}$-alkyl, where m is in each case an integer of 0 or 1;

P' is -OH, $-O-C_{1-6}$-alkyl,

and the other radicals are as defined above, where $R^7$ can additionally be a solid phase conventionally used for carrying out a solid-phase reaction;
with a compound $R^{10}$-A to give a compound of the formula (IIb)

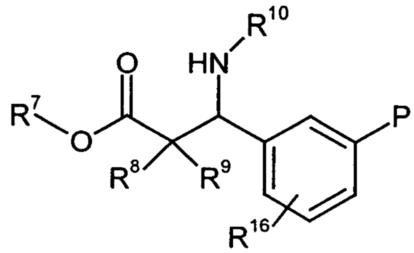

(IIb)

where

| | |
|---|---|
| $R^{10}$ | is $-SO_2R^{10'}$, $-COOR^{10''}$ or $-COR^{10'}$; |
| $R^{10'}$ and $R^{10''}$ | are as defined above; |
| A | is -Cl, -Br, -I, -O-triflyl, -O-tosyl, $-O-C_{1-6}$-alkyl, $-O-CO-C_{1-6}$-alkyl, $-O-CO-O-C_{1-6}$-alkyl, $-OC(CH_3)=CH_2$; |

and the other radicals are as defined above;

the conversion of the radical P into the radical Q,

where

Q is $-(CH_2)_mNH_2$, $-(CH_2)_mOH$, $-(CH_2)_mCH_2OH$, $-(CH_2)_mSO_2A$, $-(CH_2)_mCOA$,

A is as defined above;

m is an integer of 0 or 1;

the reaction of the compound (IIb) obtained above with a compound of the formula (IIc)

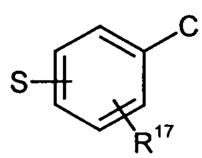

(IIc)

where

S is $ASO_2(CH_2)_n$-, $NH_2(CH_2)_n$-, $ACO(CH_2)_n$-, $HOCH_2(CH_2)_n$-, $M(CH_2)_n$-, $MCH_2(CH_2)_n$-, $HSCH_2(CH_2)_n$- or $HS(CH_2)_n$-,

where

| | |
|---|---|
| n | is an integer of 0 or 1; |
| M | is a radical including Mg, Li, Cd or Sn; |
| A | is as defined above; and |
| C | is $-NO_2$ or |

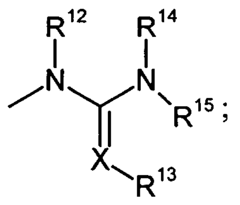

and

X, $R^{12}$, $R^{13}$, $R^{14}$ and $R^{15}$ are as defined above;

to give a compound of the formula (IId)

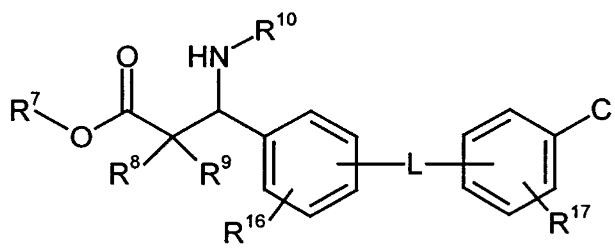

(IId)

where the radicals are as defined above;

if appropriate the conversion of C, if C is a nitro group, into an optionally cyclic urea, thiourea or guanidine unit with retention of the radical (II); and

if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid.

The β-amino acid derivatives of the formula (IIa) are either commercially obtainable or are accessible in a simple manner by standard chemical processes, such as are known to any person skilled in the art and are described in standard works such as Houben-Weyl, Methoden der organischen Chemie [Methods of Organic Chemistry], Georg Thieme-Verlag, Stuttgart. In particular, reference is made to the preparation processes for β-amino acid derivatives described by Rodionow et al., J. Am. Chem. Soc. 51, 1929, 844-846, Kunz et al., Angew. Chem. 101, 1989, 1042-1043 and Ishihara et al., Bull. Chem. Soc. Jpn., 68, 6, 1995, 1721-1730.

According to a preferred embodiment of the present invention, the β-amino acid derivatives of the formula (IIa) are obtained by reaction of malonic acid with a benzaldehyde derivative of the formula (IIa')

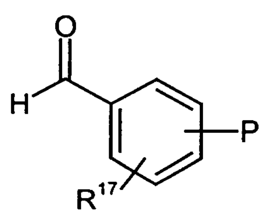

(IIa')

where $R^{17}$ and P are as defined above, in the presence of ammonia, ammonium compounds or amines. Instead of malonic acid, an ester, if appropriate with addition of a base conventionally employed for these purposes, such as NaH or a sodium alkoxide, preferably sodium methoxide or sodium ethoxide, can also be used. Preferably, an ammonium compound such as, for example, ammonium acetate is employed as the nitrogen compound.

The benzaldehyde derivatives (IIa') are either commercially obtainable or are accessible in a simple manner by standard chemical processes, such as are known to any person skilled in the art and are described in standard works such as Houben-Weyl, Methoden der organischen Chemie [Methods of Organic Chemistry], Georg Thieme-Verlag, Stuttgart.

According to a preferred embodiment of the present invention, a nitrobenzaldehyde derivative such as 3- or 4-nitrobenzaldehyde or an alkoxybenzaldehyde derivative such as 3- or 4-methoxybenzaldehyde is employed as the compound of the formula (IIa').

According to a preferred embodiment of the present invention, the β-amino acid of the formula (IIa) is obtained by reaction of approximately equimolar amounts of malonic acid, ammonium acetate and 3-nitrobenzaldehyde or 3-methoxybenzaldehyde in a solvent such as isopropanol with heating for a number of hours, preferably 2 to 6 hours, at 50 to 110°C, preferably with reflux of the solvent, in the surrounding atmosphere (i.e. in the air and under normal pressure).

For the following reaction steps, the carboxyl group is blocked by a conventional protective group P. Protective groups of this type are known to the person skilled in the art and do not have to be expressly mentioned here. The carboxyl group is particularly preferably esterified, where P is a $C_{1-6}$-alkyl such as, for example, methyl, ethyl, propyl, isopropyl, butyl, isobutyl, t-butyl, pentyl, isopentyl, neopentyl, hexyl, a $C_{3-7}$-cycloalkyl such as, for example, cyclopropyl, cyclopropylmethyl, cyclobutyl, cyclopentyl, cyclohexyl, an aryl such as, for example, phenyl, benzyl, tolyl or a substituted derivative thereof.

According to a preferred embodiment of the present invention, the synthesis of the radicals of the formula (II) according to the invention is carried out on a solid phase such as a polystyrene resin, particularly preferably a commercially obtainable Wang polystyrene resin. In this connection, the resin is first swollen in a solvent such as dimethylformamide (DMF). The appropriate carboxylic acid serving as a starting compound is then bonded to the resin by standard processes. For example, the bonding of the carboxylic acid to the resin can be carried out in the presence of a base such as pyridine and a reagent activating the carboxyl unit, such as an acid halide, for example dichlorobenzoyl chloride, in a solvent such as dimethylformamide (DMF). However, other reagents conventionally used for this purpose can also be employed. The reaction mixture is stirred at room temperature and normal pressure for at least 2 hours, preferably 12 hours, particularly preferably approximately 24 hours, the carboxylic acid being employed in an excess, preferably in a two- to three-fold excess, with respect to the loading of the solid phase.

After removal of reagents which may be unreacted, if desired a derivatization of the carboxylic acid bonded to the resin can be carried out without this previously needing to be removed from the resin. Removal from the resin is carried out in a conventional manner in an acidic medium. The product removed from the resin can be purified by known purification processes such as, for example, chromatographic processes after removal of solvents which may be present.

According to a preferred embodiment according to the invention, the carboxyl group of the above β-amino acid is esterified by reaction with an alcohol such as ethanol or a polymer conventionally used for carrying out a solid-phase reaction. This can be carried out under conditions known to the person skilled in the art, such as acid catalysis and, if appropriate, addition of a dehydrating agent such as dicyclohexylcarbodiimide. Preferably, however, the β-amino acid is suspended in the appropriate alcohol present in an excess, such as ethanol, HCl is passed through for a period of approximately 30 minutes to approximately 2 hours and the mixture is then heated in a surrounding atmosphere for a number of hours, preferably approximately 1 to 6 hours and particularly preferably approximately 3 to 5 hours, at approximately 50 to approximately 100°C, preferably under reflux of the alcohol.

The carboxyl-protected β-amino acids accessible in this way are reacted with a suitable sulphonating, carbamoylating or acylating reagent in order to obtain the corresponding sulphonamide, carbamate or amide derivatives. The sulphonating reagent used is preferably a sulphonyl chloride of the formula $R^{10''}$-$SO_2Cl$ or a carbamoyl chloride of the formula $R^{10''}$-OCOCl, where $R^{10''}$ is a $C_{1-10}$-alkyl such as methyl, ethyl, propyl, isopropyl, butyl, isobutyl, t-butyl, pentyl, isopentyl, neopentyl, hexyl, heptyl, octyl, nonyl, decyl, cyclopropyl, cyclobutyl, cyclopentyl, cyclohexyl, or camphor-10-yl, an aryl such as phenyl, benzyl, tolyl, mesityl or substituted derivatives of these such as $-C_6H_2(CH_3)_3$, $-C_6(CH_3)_5$, $-CH_2C_6H_2(CH_3)_3$, 2-chlorophenyl, 3-chlorophenyl, 4-chlorophenyl, 3-aminophenyl, 4-aminophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichlorophenylmethyl, 2,6-dichlorophenylmethyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methyl-thiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methyl-benzothiazol-2-yl, N-methoxycarbonyl-piperidin-3-yl, thiophen-2-yl, isoxazol-5-yl, 2-chloropyridin-3-yl, pyridin-3-yl, 5-methylisoxazol-3-yl, 1-adamantyl, 4-chlorophenoxymethyl, 2,2-dimethylethenyl, 2-chloropyridin-5-methyl, 5,7-dimethyl-1,3,4-triazaindolizin-2-yl, (S)-camphan-1-yl, (R)-camphan-1-yl, 8-quinolinyl, or a heterocyclic analogue of the abovementioned cyclic radicals. Instead of the abovementioned sulphonyl or carbamoyl chlorides, it is also possible to employ the corresponding fluorides, bromides or iodides. As acylating reagent, the appropriate carboxylic acid halides or carboxylic acid

anhydrides are reacted with the amino group, the appropriate $C_{1-6}$-alkyl carboxylic acid chlorides such as methyl-, ethyl-, propyl-, isopropyl-, butyl-, isobutyl-, t-butyl-, pentyl-, isopentyl-, neopentyl-, hexyl-, $C_{3-7}$-cycloalkyl such as cyclopropyl-, cyclobutyl-, cyclopentyl-, cyclohexyl-, aryl such as phenyl-, benzyl-, tolylcarboxylic acid chlorides or substituted derivatives thereof being preferred according to the invention. For the preparation of the urea or thiourea radicals, the amino group is preferably first reacted with a carbonic acid or thiocarbonic acid derivative such as a chloroformic acid ester or thiophosgene and then with a desired amine. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

According to a preferred embodiment of the invention, the carboxyl-protected β-amino acid of the formula (IIa) is treated with an equimolar amount or a slight excess of the appropriate sulphonylating agent, for example phenylsulphonyl chloride, or acylating agent, for example mesitylacetyl chloride, with cooling, preferably at 0°C, in a solvent such as pyridine or dioxane in a surrounding atmosphere in the presence of a base such as an amine, preferably triethylamine or diisopropylethylamine, and the mixture is stirred at this temperature for a period of approximately 10 minutes to approximately 2 hours. In the case of sulphonylation, this is followed by stirring at room temperature for a number of hours, preferably approximately 2 to 6 hours.

Before the synthesis of the linker group L, the radical P of the compound of the formula (IIb) must be converted into a group Q which can participate in a nucleophilic substitution either as a nucleophilic reagent or as a substrate. If P includes a nitro group, this will be reduced to the corresponding amino group, which according to the present invention can preferably be carried out by addition of tin(II) chloride to a solution of the compound of the formula (IIb) in a solvent such as ethanol and subsequent heating to approximately 50 to 110°C, preferably under reflux of the solvent, for a number of hours, preferably approximately 1 to 4 hours, in a surrounding atmosphere. If P includes an ether group, the liberation of the corresponding hydroxyl group is preferably carried out by addition of a Lewis acid such as boron tribromide in a solvent such as dichloromethane with cooling, preferably at -78°C, and subsequent stirring for a number of hours, preferably 6 to 24 hours, at room temperature. If P includes a sulphonic acid or carboxylic acid group, a conversion into the corresponding sulphonyl or carboxylic acid halide is preferably carried out. This can be carried out in a manner known to the person skilled in the art, for example by reaction of the corresponding sulphonic or carboxylic acid with thionyl chloride.

The compound prepared in this way is then reacted with a compound of the formula (IIc)

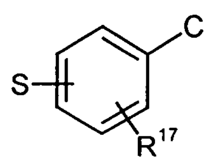

(IIc)

where

S is $ASO_2(CH_2)_n$-, $NH_2(CH_2)_n$-, $ACO(CH_2)_n$-, $HOCH_2(CH_2)_n$-, $M(CH_2)_n$-, $MCH_2(CH_2)_n$-, $HSCH_2(CH_2)_n$- or $HS(CH_2)_n$-,

where

n is an integer of 0 or 1;

M is a radical including Mg, Li, Cd or Sn;

A is as defined above; and

C is $-NO_2$ or

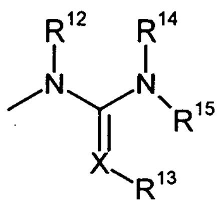

;

and

X, $R^{12}$, $R^{13}$, $R^{14}$ and $R^{15}$ are as defined above;

to give a compound of the formula (IId)

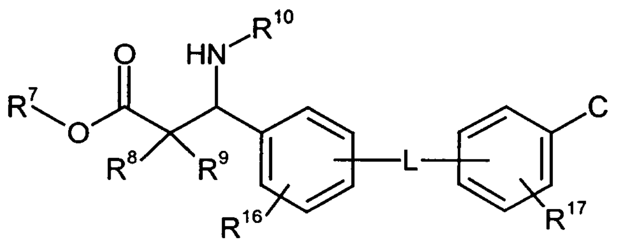

(IId)

where the radicals are as defined above. This reaction formally represents the substitution of a leaving group in one of the starting compounds by a nucleophilic unit in the other starting compound in each case.

According to a preferred embodiment of the present invention, the reactants are mixed together in approximately equimolar amounts in the presence of a base such as pyridine or sodium hydride and, if appropriate, in a solvent such as, for example, tetrahydrofuran (THF) or dimethylformamide (DMF) in a surrounding atmosphere at room temperature or with cooling, preferably at approximately 0°C, and stirred for a number of hours, preferably approximately 1 h to approximately 24 hours, at room temperature or with cooling, for example at 0°C.

The compounds of the formula (IId) thus obtained are converted into the radicals of the formula (II) according to the invention by conversion of the terminal nitro group into an open-chain or cyclic guanidine, urea or thiourea unit.

For this, the nitro group is first converted according to the invention into an amino group, preferably by addition of a customary reducing agent such as tin-(II) chloride, if appropriate in the presence of solvents such as ethanol, by stirring the reaction mixture with heating at approximately 50 to 110°C, preferably under reflux of the solvent, in a surrounding atmosphere for approximately 2 hours.

The amino group thus obtained is then converted into a guanidine, urea or thiourea unit. For this, the above amino group is first preferably reacted with a carbonic acid ester or thiocarbonic acid ester derivative in a solvent such as dimethylformamide (DMF) in the presence of mercury-(II) chloride with cooling, preferably at approximately 0°C, and stirring for approximately 10 minutes to approximately 3 hours with cooling, preferably at approximately 0°C, and if appropriate subsequently at room temperature. The carbonic acid ester or thiocarbonic acid ester derivative employed can preferably be phosgene, triphosgene, thiophosgene, chloroformic acid esters or thiopseudourea derivatives, commercially obtainable chloroformic acid esters being preferred for the preparation of the urea derivatives, thiophosgene being preferred for the preparation of the thiourea derivatives and thiopseudourea derivatives being preferred for the preparation of guanidine derivatives.

The carbamates or isothiocyanates formed in this way can be converted into the corresponding urea, thiourea and guanidine derivatives by reaction with appropriate amines. The amines used can be substances of the formula HNRR', where R and R' independently of one another or simultaneously can be hydrogen, a substituted or unsubstituted alkyl or cycloalkyl radical, a substituted or unsubstituted aryl radical, a saturated or unsaturated, optionally substituted heterocyclic radical, an alkylamine radical, an alkylamide radical or can be connected to one another and together with the nitrogen atom can form an optionally substituted heterocyclic ring system which can be saturated or unsaturated and/or can contain further heteroatoms. With respect to the preferred radicals on the amine, reference is made to the above description of the compounds according to the invention. According to the invention, the carbamate or isothiocyanate is preferably reacted with an amine at room temperature with stirring for approximately 1 to 5 hours, preferably approximately 2 to 3 hours, in the presence of an auxiliary base such as diisopropylethylamine in a solvent such as dimethylformamide (DMF). In the case of the preparation of cyclic guanidine derivatives, the corresponding isothiocyanate is preferably first heated in ethanol for a number of hours, preferably approximately 12 to 24 hours, and then heated with a diamine such as diaminoethane in a solvent such as toluene, dimethylformamide (DMF) or a mixture of both.

According to a further preferred embodiment of the present invention, it is also possible to generate the above guanidine, urea or thiourea group on the compound of the formula (IIc) in the above manner and then to react the compound of the formula (IIc) thus obtained with the compound of the formula (IIb) in the manner described above.

The compounds obtained according to the process explained above can furthermore be derivatized by removal of protective groups which may be present, further substitution of nitrogen atoms present at preferred positions

in the preparation process and/or conversion of the compound obtained into the free acid and/or its physiologically acceptable salts. For example, the t-butoxymethoxycarbonyl groups conventionally used as protective groups for nitrogen atoms are removed in an acidic medium, for example by addition of trifluoroacetic acid. Suitable alkylating agents for derivatization of nitrogen atoms are reagents conventionally used for this purpose in this step, to which, for example, a substituted or unsubstituted alkyl or cycloalkyl radical, a substituted or unsubstituted aryl radical or a saturated or unsaturated, optionally substituted heterocyclic radical can be bonded to the corresponding nitrogen atom. With respect to the substituents preferably bonded to the respective nitrogen atoms, reference is made to the above description of the compounds according to the invention. The above reactions and their implementation are well known to the person skilled in the art and are described in detail in standard works such as, for example, Houben-Weyl, Methoden der organischen Chemie [Methods of Organic Chemistry], Georg Thieme Verlag, Stuttgart.

The ester derivatives according to the invention can be converted into the corresponding free carboxylic acids in a conventional manner, such as, for example, by basic ester hydrolysis.

If desired, the compounds according to the invention can be converted into their physiologically acceptable salts. This can be carried out either by reaction with an organic or inorganic base such as, for example, an alkali metal hydroxide or alkaline earth metal hydroxide such as KOH, NaOH, LiOH, $Mg(OH)_2$ or $Ca(OH)_2$, whereby the terminal carboxyl group is deprotonated and the corresponding carboxylate is formed, or by reaction with an organic or inorganic acid such as, for example, hydrochloric acid, sulphuric acid, phosphoric acid, mandelic acid, oleic acid, linoleic acid or p-toluenesulphonic acid, whereby one or more of the above nitrogen atoms are protonated.

The conjugates according to the invention can be used as active compound components for the production of medicaments against carcinomatous disorders. For this, they can be converted into the customary formulations such as tablets, coated tablets, aerosols, pills, granules, syrups, emulsions, suspensions and solutions in a known manner using inert, non-toxic, pharmaceutically suitable excipients or solvents. Preferably, the compounds according to the invention are used here in an amount such that their concentration in the total mixture is approximately 0.5 to approximately 90% by weight, the concentration, inter alia, being dependent on the corresponding indication of the medicament.

The abovementioned formulations are produced, for example, by extending the active compounds with solvents and/or excipients having the above properties,
where, if appropriate, additionally emulsifiers or dispersants and, in the case of water as the solvent, alternatively an organic solvent, have to be added.

The medicaments according to the invention can be administered in a customary manner.

The present invention is illustrated below with the aid of non-restricting examples and comparison examples.

## Examples

In the examples below, all quantitative data, if not stated otherwise, relate to percentages by weight. The mass determinations were carried out by high-performance liquid chromatography-mass spectrometry (HPLC-MS) using the electron spray ionization (ESI) method or by FAB or MALDI mass spectroscopy.

List of the abbreviations used

HPLC - high-performance liquid chromatography
RP - reverse phase
ACN - acetonitrile
DMF - dimethylformamide
DCM - dichloromethane
THF - tetrahydrofuran
DIEA - diisopropylethylamine (Hünig's base)
NMP - N-methylpyrrolidone
TFA - trifluoroacetic acid
Fmoc - fluorenyl-9-methoxycarbonyl
RT - room temperature
MTBE - methyl tert-butyl ether
Boc - tert-butyloxycarbonyl
TLC - thin-layer chromatography
DMAP - dimethylaminopyridine
DMSO - dimethyl sulphoxide

Abu - γ-amino butyric acid

**I. Synthesis of starting materials:**

*I.1 20-O-L-Valyl-camptothecin trifluoroacetate*

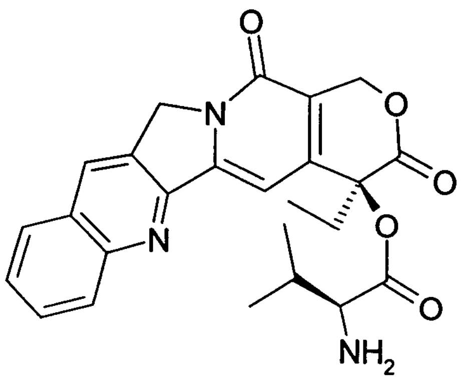

x 2 $CF_3COOH$

A suspension of 10 g (28.7 mmol) of 20(S)-camptothecin in 500 ml of absolute dichloromethane is treated with stirring with 14 g (2 eq.) of N-(tert-butoxycarbonyl)-valine-N-carboxyanhydride and 1 g of 4-(N,N-dimethylamino)-pyridine. After heating under reflux for 4 days, the mixture is concentrated in vacuo. The residue is stirred with 100 ml of MTBE for 20 min. 200 ml of petroleum ether are then added and the mixture is filtered. 14.9 g of the Boc-protected intermediate compound are obtained, which can contain small amounts of D-valine epimer which, however, can be removed without problems after removal of the protective group.

11.65 g of this Boc-protected intermediate compound are then stirred at 5°C for 1 h in a mixture of 300 ml of dichloromethane and 70 ml of anhydrous trifluoroacetic acid. After concentrating in vacuo to a small volume, the product is precipitated with diethyl ether and thoroughly washed with diethyl ether. The product is again precipitated from dichloromethane/methanol using diethyl ether. If appropriate, the crude product is again taken up in 40 ml of methanol, and the solution is treated with 120 ml of MTBE and cooled to 0°C. The precipitate is filtered off and 9.4 g (80%) of 20-O-(valyl)-camptothecin trifluoroacetate are obtained after drying.
[TLC: acetonitrile/water (20:1); $R_f$ = 0.39].

*I.2 20-O-[L-Prolyl-L-valyl]-camptothecin trifluoroacetate*

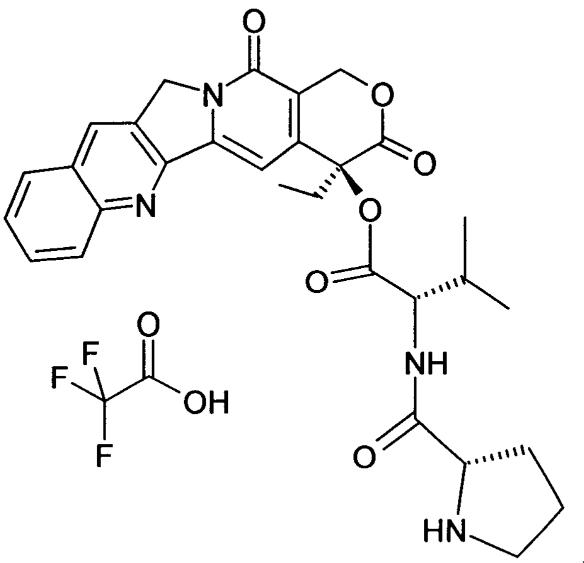

1 g (3.2 mmol) of N-tert-butoxycarbonyl-prolin-N-hydroxy succinimide ester (Boc-Pro-OSu) are dissolved in 75 ml of DMF. 1.5 g (2.67 mmol) of the compound from Example I.I and 1.4 ml of Hünig's base are added and the mixture is stirred for 6 h. Another amount of 1g of Boc-Pro-OSu are added and the mixture is stirred overnight. The solvent is evaporated and the residue is stirred with water and filtrated. The filter residue is dissolved in dichloromethane and precipitated with diethyl ether and petrol ether 1:1. The recrystallization is repeated and the product is isolated by filtration and dried in vacuo. Yield: 1.715g = 75%; TLC: acetonitrile/water (20:1) $R_f$ = 0.73.

1.715 g (2.66 mmol) of this Boc-protected intermediate compound are then stirred at 5°C for 1 h in a mixture of 100 ml of dichloromethane and 20 ml of anhydrous trifluoroacetic acid. After concentrating in vacuo, the product is taken up in dichloromethane/methanol, precipitated with diethyl ether and thoroughly washed with diethyl ether. The product is again precipitated from dichloromethane/methanol using diethyl ether. The precipitate is filtered off and 1.46 g (83%) of the target compound are obtained after drying.
[TLC: acetonitrile/water/glacial acetic acid 5/1/0.2 $R_f$ = 0.43].

*I.3 20-O-[L-Prolyl-L-leucyl]-camptothecin trifluoroacetate*

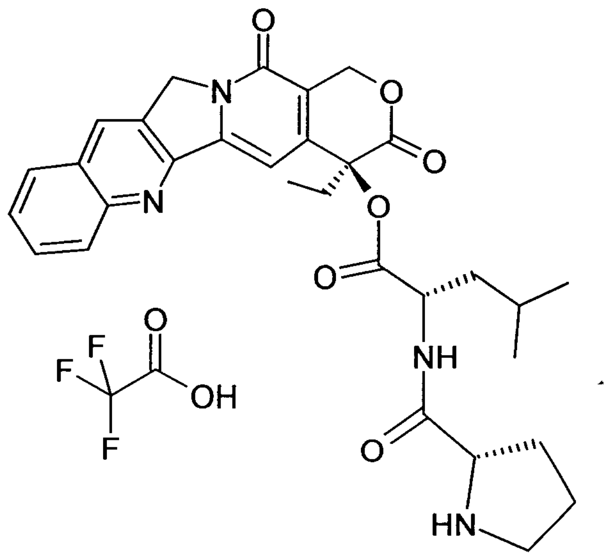

This compound is prepared in analogy to example I.2 and I.1.

*I.4 20-O-[Glycyl-L-prolyl-L-valyl]-camptothecin trifluoroacetate*

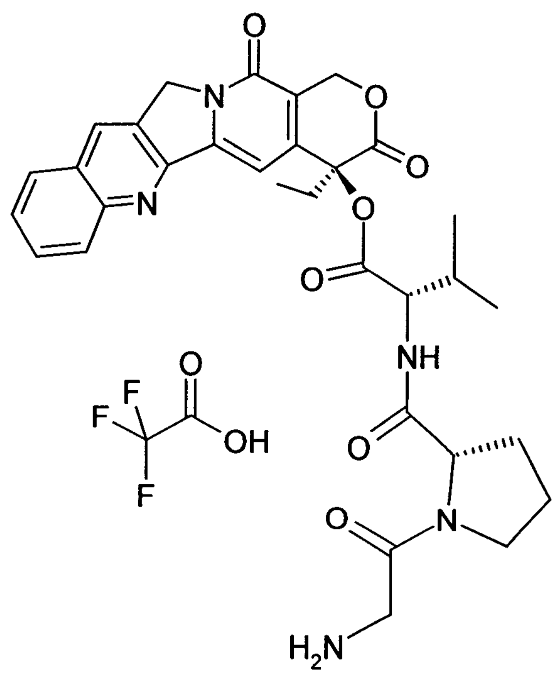

1 g (1.52 mmol) of compound 1.2 are suspended in 50 ml dichloromethane and 653 mg (3 mmol) N-(t-butoxycarbonyl)-glycin-N-carboxy-anhydride (Boc-Gly-NCA) and 371 mg (3 mmol) 4-dimethylamino pyridine are added. After stirring for 1 h the solvent is removed , the residue is stirred with water and filtrated. The crude product is purified by flash chromatography using acetonitrile as eluent. Isolation of relevant fractions and evaporation of solvent gives

748 mg (70 %) of the protected intermediate product.
The t-butoxycarbonyl protecting group is removed as described in example I.2. 709 mg (93 %) of the target compound are obtained.
[TLC: acetonitrile/water/glacial acetic acid 5/1/0.2 $R_f$ = 0.34].

*I.5 20-O-[L-Alanyl-L-prolyl-L-valyl]-camptothecin trifluoroacetate*

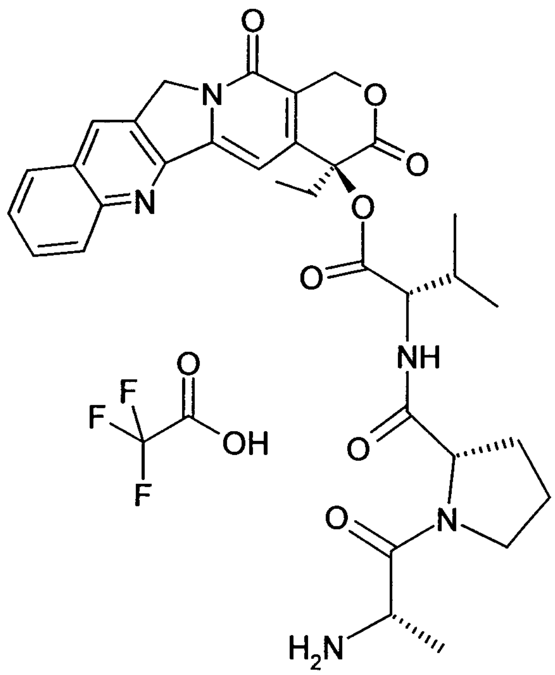

135 mg (0.2 mmol) of compound 1.2 are suspended in 25 ml DMF and 65 mg (0.23 mmol) N-(t-butoxycarbonyl)-alanine-N-hydroxysuccinimide ester (Boc-Ala-OSu) and 80 mg Ethyl-diisopropylamine are added. After stirring for 16 h another amount of 32 mg Boc-Ala-OSu are added and stirring is continued for 6 h. The solvent is removed, the residue is stirred with water and filtrated. The crude product is purified by flash chromatography using acetonitrile as eluent. Isolation of relevant fractions and evaporation of solvent gives 107 mg (73 %) of the protected intermediate product.
The t-butoxycarbonyl protecting group is removed as described in example I.2. 92 mg (87 %) of the target compound are obtained.
[TLC: acetonitrile/water/glacial acetic acid 5/1/0.2 $R_f$ = 0.32].

I.6 20-O-[L-Aspartyl-L-prolyl-L-valyl]-camptothecin trifluoroacetate

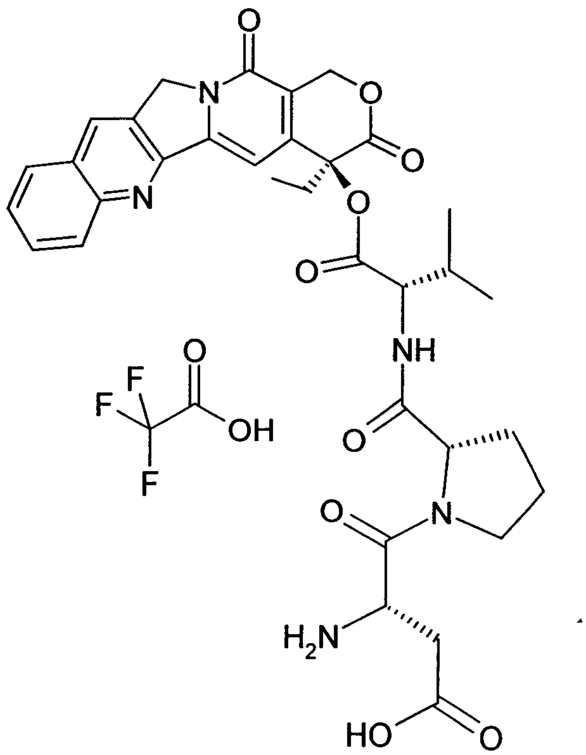

21 mg (0.073 mmol) N-(t-butoxycarbonyl)-aspartatic acid- γ-t-butylester (Boc-Asp(OtBu)-OH) are dissolved in 10 ml DMF. 12 mg 1-hydroxy-1H-benzotriazole (HOBT), 14 mg (0.073 mmol) of N-(3-dimethylaminopropyl)-N'-ethyl-carbodiimide hydrochloride and 24 mg Ethyl-diisopropylamine are added. The mixture is stirred for 30 min. Subsequently, 40 mg (0.061 mmol) of compound I.2 are added. After sonification for 2 the reaction is complete and the solvent is removed. The residue is distributed between dichloromethane and water. The organic phase is dried over sodium sulfate and concentrated. The residue is precipitated from dichloromethane with a mixture of petrol ether and diethyl ether. 47 mg (95 %) of the protected intermediate compound are obtained.
Both, the t-butylester and the t-butoxycarbonyl protecting group are removed as described in example I.2.
33 mg (74 %) of the target compound are obtained.
[TLC: acetonitrile/water/glacial acetic acid 5/1/0.2 $R_f$ = 0.28].

Further camptothecin peptide conjugates shown in table 1 are synthesized following standard procedures as described in *Houben Weyl; Methoden der Organischen Chemie; Vierte Auflage; Band XV Teil 1 und 2; Georg Thieme Verlag Stuttgart 1974,* or in *Hans-Dieter Jakubke and Hans Jeschkeit: Aminosäuren, Peptide, Proteine; Verlag Chemie, Weinheim 1982* or as exemplified in the previous examples.

Table 1:

| Ex. | Compound | $R_f$ |
|---|---|---|
| I.7 | 20-O-[L-Asn-L-Pro-L-Val]-camptothecin trifluoroacetate | 0.25 1) |
| I.8 | 20-O-[L-His-L-Pro-L-Val]-camptothecinbis-trifluoroacetate | 0.17 1) |
| I.9 | 20-O-[L-Nva-L-Pro-L-Val]-camptothecin trifluoroacetate | 0.38 1) |
| I.10 | 20-O-[Gly-L-Pro-L-Leu]-camptothecin trifluoroacetate | 0.27 1) |

1) Acetonitrile/water/glacial acetic acid 5/1/0.2
2) Acetonitrile/water/glacial acetic acid 10/1/0.1

*I.11 N-Leucyl-quinolone-a trifluoroacetate*

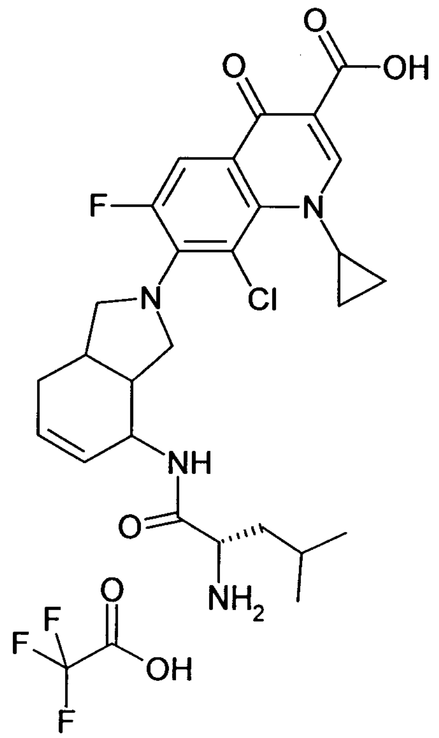

122 mg (0.53 mmol) N-(t-butoxycarbonyl)-leucine (Boc-Leu-OH) are dissolved in 10 ml DMF. 108 mg 1-hydroxy-1H-benzotriazole (HOBT), 122.3 mg (0.64 mmol) of N-(3-dimethylaminopropyl)-N'-ethylcarbodiimide hydrochloride and 206 mg Ethyl-diisopropylamine are added. The mixture is stirred for 30 min. Subsequently, 200 mg (0.48 mmol) of quinolone-a are added. After stirring for 4h the reaction is complete and the solvent is removed. The residue is stirred with water and filtrated. The residue is precipitated from dichloromethane with diethyl ether. 247 mg (74 %) of the protected intermediate compound are obtained.
The t-butoxycarbonyl protecting group is removed as described in example I.1. 245 mg (98 %) of the target compound are obtained.
[TLC: acetonitrile/water/glacial acetic acid 5/1/0.2 $R_f$ = 0.46].

*I.12 N-(Alanyl-prolyl-leucyl)-quinolone-a trifluoroacetate*

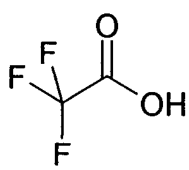

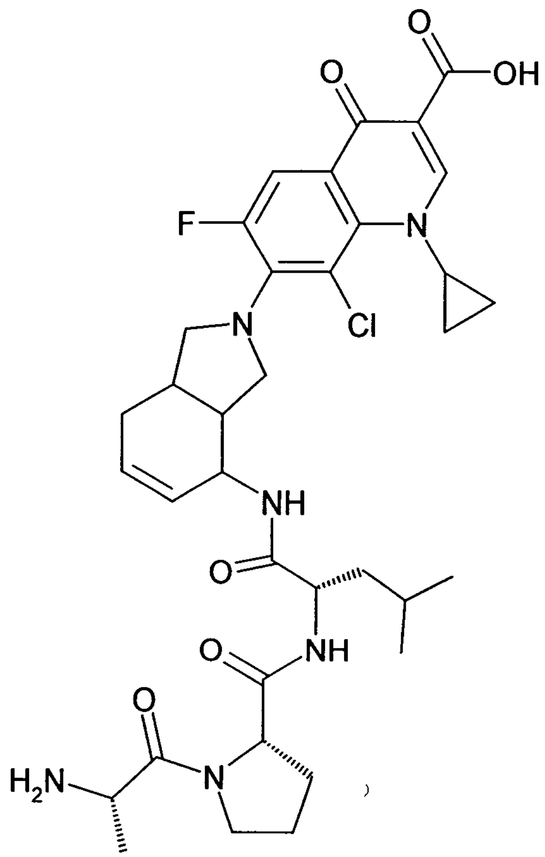

Starting from compound I.11 this conjugate is synthesized in analogy to example I.5. [TLC: acetonitrile/water/glacial acetic acid 5/1/0.2 $R_f$ = 0.35].

*I.13 N-(Glycyl-prolyl-leucyl)-quinolone-a trifluoroacetate*

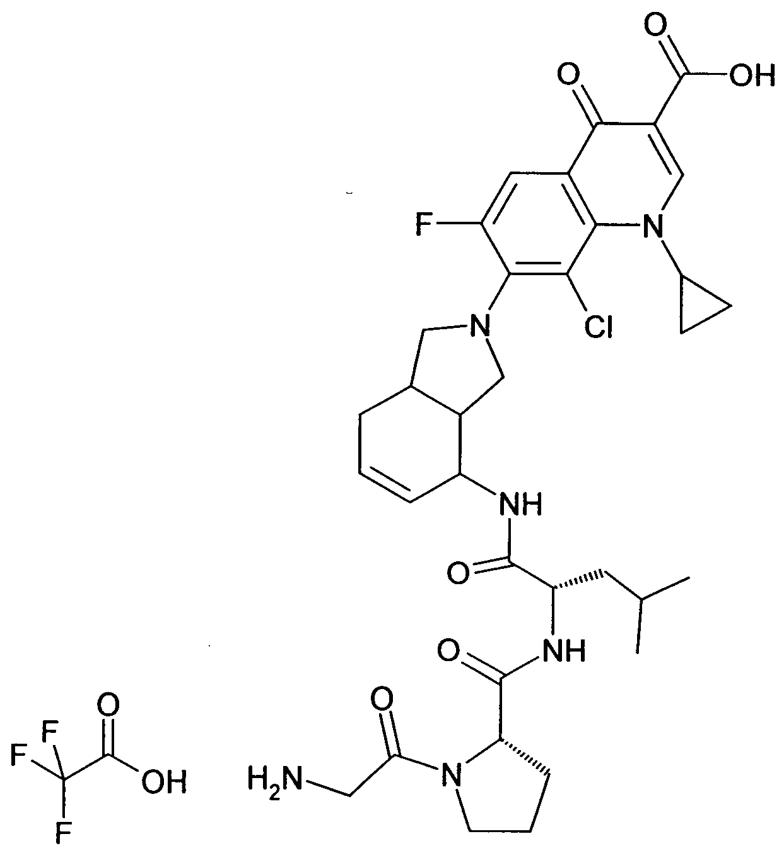

Starting from compound I.11 this conjugate is synthesized in analogy to example I.5. [TLC: acetonitrile/water/glacial acetic acid 5/1/0.2 $R_f$ = 0.4].

*I.14 N-(Glycyl-prolyl-valyl)-quinolone-a trifluoroacetate*

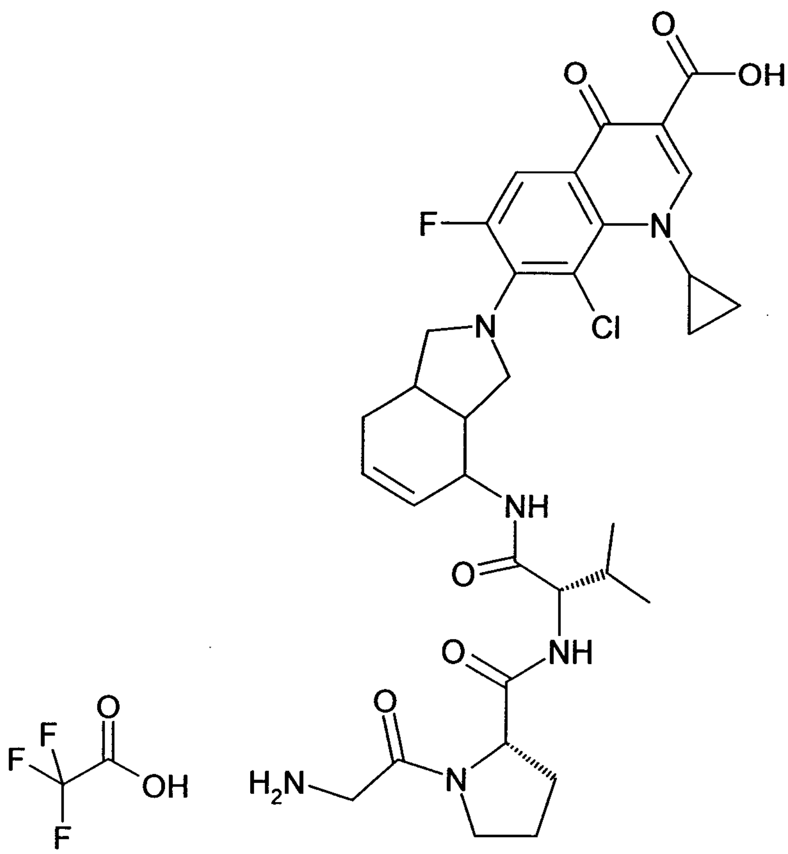

This conjugate is synthesized in analogy to example I.12.
[TLC: acetonitrile/water/glacial acetic acid 5/1/0.2 $R_f$ = 0.5].

## II. Preparation of integrin ligands

Example II.1

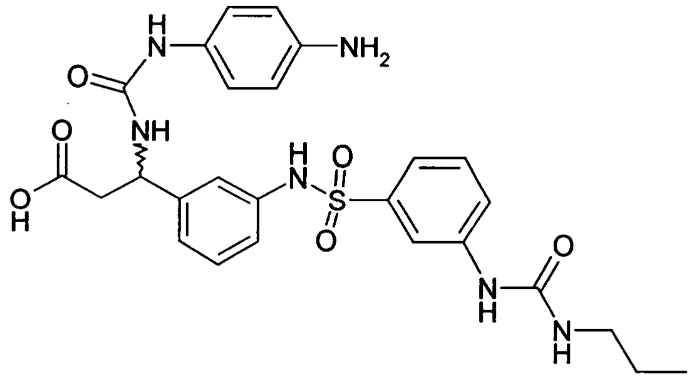

*II.1-a 3-Amino-3-[3-(propylaminocarbonylamino-phenyl-sulphonylamino)-phenyl]-propionic acid*

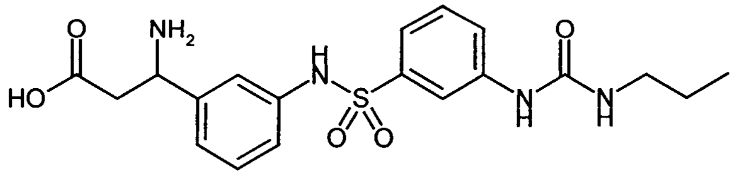

1.2 g of polystyrene Wang resin (loading 1.08 mmol/g) are swollen in DMF. The solvent is filtered off with suction and a solution of 841 mg of (3R,S)-3-(9-fluorenyl-methoxycarbonylamino)-3-(3-nitrophenyl)-propionic acid (amino acid reagent) in 15 ml of DMF are added. After shaking at room temperature for 15 min, the suspension is treated with 350 µl of pyridine and 540 mg of 2,6-dichlorobenzoyl chloride. It is shaken overnight at room temperature. The resin is then washed with DMF, MeOH and DCM. The resin is treated with a solution of 5400 mg of tin(II) chloride dihydrate in 12 ml of NMP and shaken overnight at room temperature. The resin is then washed with NMP, MeOH, THF and DCM. The resin is treated with a solution of 450 µl of DIEA in 500 µl of THF and a solution of 430 mg of 3-nitrobenzenesulphonyl chloride in 500 µl of THF. It is shaken overnight at room temperature. The resin is then washed with DMF, MeOH and THF.

The resin is treated with a solution of 5400 mg of tin(II) chloride dihydrate in 12 ml of NMP and shaken overnight at room temperature. The resin is then treated with NMP, MeOH, THF and DCM. The resin is treated with a solution of 500 µl of DIEA in 12 ml of THF/DCM 1:1 and a solution of 2757 mg of 4-nitrophenylchloroformic acid ester in 12 ml of THF/DCM 1:1. After shaking at room temperature for 45 min, it is washed with THF and DMF and a solution of 943 mg of propylamine and 2780 µl of DIEA in 20 ml of NMP is added. After shaking for 10 h, the resin is washed with DMF, MeOH, THF and DCM.

For the removal of the product, the resin is shaken for 1 h with 12 ml of TFA/DCM and filtered off, and the filtrate is concentrated in vacuo.

*II.1 3-(4-Aminophenylaminocarbonylamino)-3-[3-(3-propylaminocarbonylaminophenyl-sulphonylamino)-phenyl]-propionic acid*

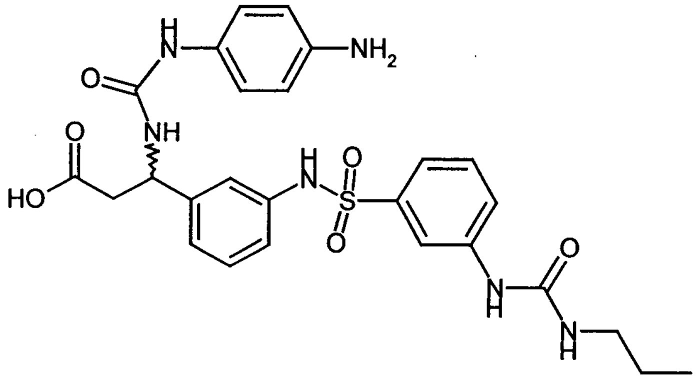

70 mg (0.166 mmol) of the compound II.1-a are stirred with 54 mg (2 eq) of 4-nitrophenyl isocyanate for 1 h in 10 ml of DMF. The mixture is concentrated and the residue is purified by flash chromatography on silica gel using dichloromethane/methanol/ammonia 17% strength (15:2:0.2). After precipitation from dichloromethane/methanol using ether, the intermediate a (29 mg; 30%) is obtained.

This is dissolved in methanol and hydrogenated over palladium/carbon. The catalyst is separated off, the solution is concentrated and the residue is lyophilized from dioxane/water. 18 mg (74%) of the target compound are obtained.

<u>II.2: Enantiomer A of II.1 (3-(4-Aminophenylaminocarbonylamino)-3-[3-(3-propylaminocarbonylamino-phenyl-sulphonylamino)-phenyl]-propionic acid)</u>

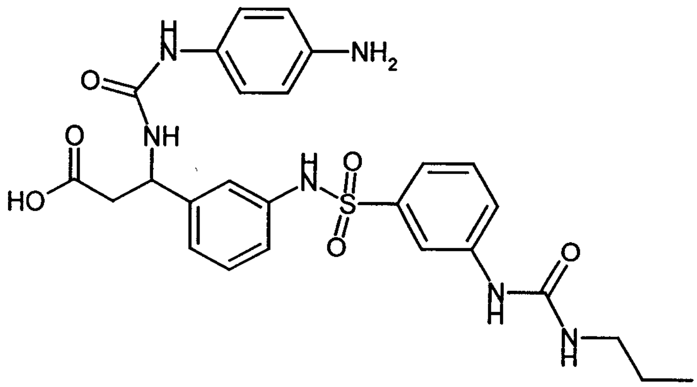

*II.2.a 3-(Amino)-3-(3-nitrophenyl)propionic acid hydrochloride*

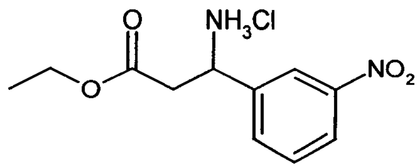

151 g of 3-nitrobenzaldehyde, 94 g of ammonium acetate and 127 g of malonic acid were heated under reflux for 5 h in 1 l 2-propanol. The solution was filtered and the precipitate was washed with 0.7 1 of hot 2-propanol. The crude product was dried in vacuo, suspended in 1.5 l of water, treated with 1 N hydrochloric acid and filtered. The filtrate was concentrated to yield 146 g.
$^1$H-NMR (400 MHz, $D_4$-MeOH): 3.09 (m, 2 H), 4.88 (m, 1 H), 7.74 (t, 1 H), 7.90 (d, 1 H), 8.33 (d, 1 H), 8.43 (s, 1 H).

*II.2.b Ethyl 3-(amino)-3-(3-nitrophenyl)propanoate hydrochloride*

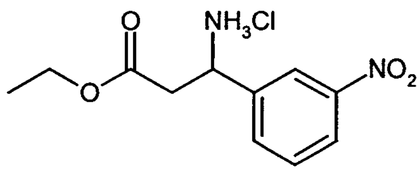

60 g 3-amino-3-(3-nitrophenyl)-propionic acid hydrochloride from example II.2.a were suspended in 660 ml of ethanol and gaseous HCl was passed in the mixture for 1 h. The reaction mixture was then heated under reflux for 4 h and then cooled and concentrated. 62 g of a white solid were obtained.
$^1$H-NMR (400 MHz, $D_4$-MeOH): 1.22 (t, 3 H), 3.12 (dd, 1 H), 3.20 (dd, 1 H), 4.18 (q, 2 H), 4.95 (t, 1 H), 7.77 (t, 1 H), 7.94 (d, 1 H), 8.35 (d, 1 H), 8.43 (s, 1 H).

*II.2.c Ethyl 3-{[(allyloxy)carbonyl]amino}-3-(3-nitrophenyl)propanoate*

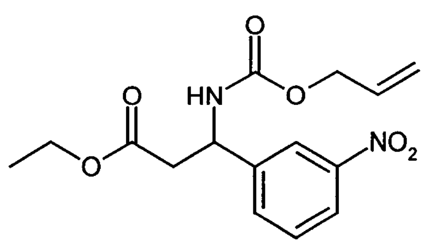

To 41.2 g ethyl 3-amino-3-(3-nitrophenyl)-propioniate hydrochloride from example II.2.b in 350 ml dichloromethane 48.8 g diisopropylethylamine and 24.8 g allyloxycarbonyl chloride in 150 ml dichloromethane were added at 0°C. After stirring for 30 min the mixture was washed with water, dried over $MgSO_4$ and concentrated to give a white solid (yield: 56.4 g).
$^1$H-NMR (400 MHz, $CDCl_3$): 1.18 (t, 3 H), 2.90 (d, 2 H), 4.11 (q, 2 H), 4.58 (m, 2 H), 5.15-5.40 (m, 3 H), 5.90 (m, 1 H),

6.05 (m, 1 H), 7.55 (t, 1 H), 7.70 (d, 1 H), 8.12 (d, 1 H), 8.19 (s, 1 H).

*II.2.d Ethyl 3-{[(allyloxy)carbonyl]amino}-3-(3-aminophenyl)propanoate*

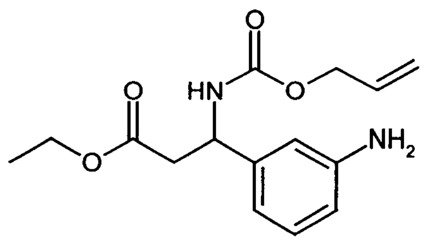

64.6 g tin-(II) chloride were added to a solution of 18.8 g ethyl 3-{[(allyloxy)carbonyl]amino}-3-(3-nitrophenyl) propanoate from example II.2.c in 245 ml ethanol, and the mixture was heated to reflux for 2 h. The reaction mixture was hydrolized with 300 ml of 2N NaOH solution, then filtered through silica gel and washed with dichloromethane. The organic phase was dried over $MgSO_4$ and concentrated to yield 13.1 g of a white solid.
$^1$H-NMR (400 MHz, $CDCl_3$): 1.19 (t, 3 H), 2.80 (m, 2 H), 4.06 (q, 2 H), 4.57 (m, 2 H), 5.07 (m, 1 H), 5.20 (d, 1 H), 5.29 (d, 1 H), 5.70 (m, 1 H), 5.89 (m, 1 H), 6.57 (d, 1 H), 6.62 (s, 1 H), 6.68 (d, 1 H), 7.11 (t, 1 H).

*II.2.e Ethyl-3-{[(allyloxy)carbonyl]amino}-3-(3-{[(3-nitrophenyl)sulfonyl]amino}-phenyl)propanoate*

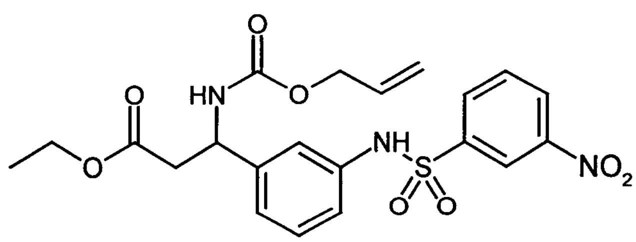

3Nitrophenylsulphonyl chloride was added at 0°C to a solution of 11.6 g ethyl 3-{[(allyloxy)carbonyl]amino}-3-(3-aminophenyl)propanoate from example II.2.d in 110 ml pyridine. After a reaction time of 2 h, the mixture was concentrated, treated with 1 N HCl and extracted with dichloromethane. After drying over $MgSO_4$, the solvent was removed and the crude product was purified by chromatography on silica gel (dichloromethane/methanol = 40:1) to give 17.8 g of a solid.

*II.2.f Ethyl-3-{[(allyloxy)carbonyl]amino}-3-(3-{[(3-aminophenyl)sulfonyl]amino}-phenyl)propanoate*

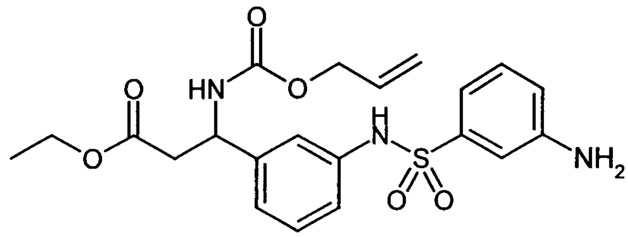

43.5 g tin-(II) chloride were added to a solution of 17.8 g ethyl 3-{[(allyloxy)carbonyl]amino}-3-(3-{[(3-nitrophenyl)sulfonyl]amino}phenyl)propanoate from example II.2.e in 165 ml ethanol, and the mixture was heated to reflux for 2 h. The reaction mixture was hydrolized with 200 ml of 2N NaOH solution, then filtered through silica gel and washed with dichloromethane. The organic phase was dried over $MgSO_4$ and concentrated to yield 9.2 g of a solid.
The material was separated into ist enantiomers by chiral chromatography with the selector Bayer-CSP (N-methacryloyl-L-valin-3-pentylamide) using THF as solvent. Similar selectors have been described in the literature (Angew. Chem. Int. Ed. Engl. 30 (1991), 1662-1664.). This separation yielded in the two products fraction 1 and fraction 2.
$^1$H-NMR (400 MHz, $CDCl_3$): 1.18 (t, 3 H), 2.80 (m, 2 H), 3.95 (br.s, 2 H), 4.08 (q, 2 H), 4.54 (m, 2 H), 5.08 (m, 1 H), 5.22 (d, 1 H), 5.30 (d, 1 H), 5.78 (m, 1 H), 5.90 (m, 1 H), 6.58 (s, 1 H), 6.75 (d, 1 H), 6.88 (d, 1 H), 6.95 (s, 1 H), 7.05 (d, 1 H), 7.11 (d, 1 H), 7.15-7.22 (m, 3 H).

*II.2.g Ethyl-3-{[(allyloxy)carbonyl]amino}-3-(3-{[(3-{[(propylamino)carbonyl]-amino}phenyl)sulfonyl]amino}phenyl) propanoate*

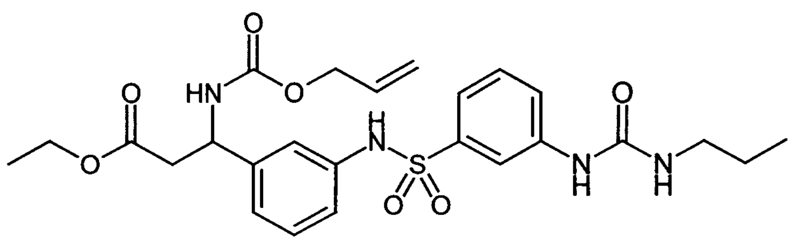

9.6 g propylisocyanate was added to a solution of 50 g of enantiopure ethyl 3-{[(allyloxy)carbonyl]amino}-3-(3-{[(3-aminophenyl)sulfonyl]amino}phenyl)propanoate from fraction 1 from example II.2.f in 500 ml dioxane and the mixture was stirred for 18 h at 50°C. After evaporation of the solvent 1.5 l hydrochloric acid (1 M) was added and the solution was extracted with dichloromethane. The organic phase was dried over $MgSO_4$ and concentrated. Chromatographie on silica gel (dichloromethane/methanol = 30:1) yielded 19.1 g of the compound.
$^1$H-NMR (400 MHz, $CDCl_3$): 0.90 (t, 3 H), 1.19 (t, 3 H), 1,52 (q, 2 H), 2.83 (m, 2 H), 3.18 (m, 2 H), 4.10 (q, 2 H), 4.56 (m, 2 H), 5.05-5.35 (m, 3 H), 5.90 (m, 1 H), 6.18 (m, 1 H), 6.77 (m, 1 H), 6.88 (s, 1 H), 7.00-7-45 (m, 8 H), 7.98 (m, 1 H).

*II.2.h Ethyl 3-amino-3-(3-{[(3-{[(propylamino)carbonyl]amino}phenyl)sulfonyl]-amino}phenyl)propanoate*

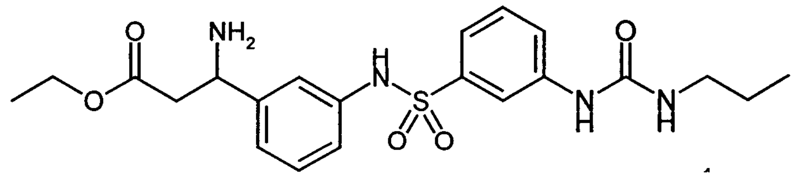

2.57 ml acetic acid, 5.32 ml tributyltinhydride and 177 mg bistriphenylphosphine palladium(II) chloride were added to a solution of 9.58 g ethyl 3-{[(allyloxy)carbonyl]amino}-3-(3-{[(3-{[(propylamino)carbonyl]amino}phenyl)sulfonyl]amino}phenyl)propanoate from example II.2.g in 245 ml dichloromethane and stirred at room temperature for 22 h. A solution of $NaHCO_3$ was added and the mixture was extracted with dichloromethane. After drying over $MgSO_4$, the solvent was removed and the crude product was purified by chromatographie on silica gel (dichloromethane/methanol = 10:1) to give 2.30 g of the desired compound.
$^1$H-NMR (400 MHz, $CDCl_3$): 0.88 (t, 3 H), 1.20 (t, 3 H), 1,50 (q, 2 H), 2.60 (dd, 1 H), 2.67 (dd, 1 H), 3.17 (m, 2 H), 4.11 (q, 2 H), 4.33 (m, 1 H), 5.21 (m, 1 H), 6.89 (d, 1 H), 7.07 (d, 1 H), 7.16 (t, 1 H), 7.22-7.34 (m, 5 H), 7.38 (s, 1 H), 7.49 (s, 1 H).

*II.2 3-(4-Aminophenylaminocarbonylamino)-3-[3-(propylaminocarbonylaminophenyl-sulphonylamino)-phenyl]-propionic acid, enantiomer A*

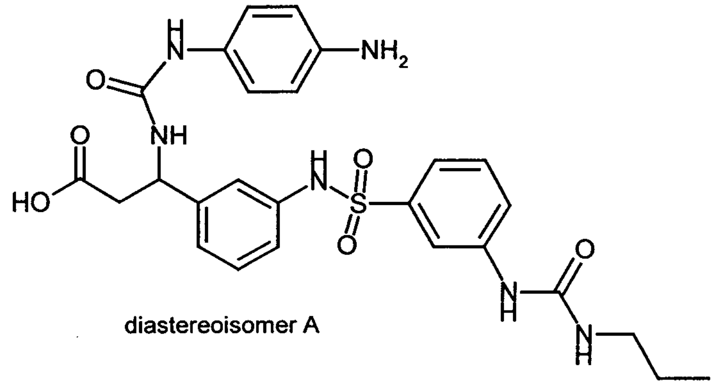

diastereoisomer A

700 mg (1.56 mmol) of the compound II.2.h are stirred with 510 mg (2 eq) of 4-nitrophenyl isocyanate for 1 h in 100 ml of DMF. The mixture is concentrated and the residue is purified by flash chromatography on silica gel using dichloromethane/methanol/ammonia 17% strength (15:2:0.2). After precipitation from dichloromethane/methanol using ether, the intermediate a (290 mg; 30%) is obtained. This is dissolved in methanol and hydrogenated upon palladium/carbon. The catalyst is separated off, the solution is concentrated and the residue is lyophilized from dioxane/water. 204 mg (74%) of intermediate b are obtained. 200 mg (0.34 mmol) of intermediate b are dissolved in methanol and treated with 1 ml of a 2M lithium hydroxide solution. After 6h additional 300 μl of lithium hydroxide are added and the mixture is stirred until the de-esterification is complete. The solution is concentrated and precipitated from dichloromethane using ether. 141 mg (75%) of compound II.2 are obtained [TLC: (acetonitrile/water/glacial acetiic acid 10/1/0.1 $R_f$ = 0.6].
ESI-MS: m/e = 555 $(M+H)^+$].

*II.3: Enantiomer B of 3-(4-Aminophenylaminocarbonylamino)-3-[3-(propyaminocarbonylamino-phenyl-sulphonylamino)-phenyl]-propionic acid*

The same protocol as for the synthesis of enantiomer A was used except that fraction 2 of ethyl 3-{[(allyloxy) carbonyl]amino}-3-(3-{[(3-aminophenyl)sulfonyl]amino}-phenyl)propanoate was was used after the seperation of the enantiomers.

**III Preparation of conjugates of integrin ligands with cytotoxic agents**

**General procedure A (thiourea linkage):**

0.09 mmol of an integrin ligand from series II are dissolved in 10 ml of dioxane/water (1:1) and treated with 9.6 µl (0.13 mmol) of thiophosgene. After stirring at room temperature for 15 min 94 µl (0.54 mmol) of Hünig's base are added, the mixture is stirred for a further 10 min and then concentrated. The residue is taken up in dichloromethane and precipitated using ether. The obtained isothiocyanates are reacted in the next step without further purification.

0.09 mmol of the isothiocyanate is dissolved in 15 ml of DMF and then treated with 0.08 mmol of one of the peptide conjugates in series I in the presence of 43 µl of Hünig's base. After stirring at room temperature for 30 min, the mixture is concentrated and the residue is stirred with water. The residue is separated and dissolved in methanol/ dichloromethane. The mixture is precipitated using ether.

If neccessary, further purification is done by flash chromatography at silica gel. Appropriate eluent systems are:

Dichloromethane/methanol/ammonia 17% 15/3/0.3
Dichloromethane/methanol/ammonia 17% 16/2/0.2

The relevant fractions are collected, concentrated and the target products are isolated by precipitation from methanol/dichloromethane using ether. In many cases conjugates are transformed into respective sodium salts to improve water solubility. Therefore, the conjugates are suspended in water and 1-2 equivalents of a 0.01 N aqueous solution of sodium hydroxide is added until solution occurs and a pH of about 8 is reached. Subsequent lyophilisation yields the sodium salts of conjugates of integrin ligands with cytotoxic agents.

**General procedure B (urea linkage)**

0.07 mmol 4-Nitrophenyl chloroformic acid ester are dissolved in 10 ml THF and 16 µl Hünig's base are added. Subsequently, 0.05 mmol of one of the integrin ligands from series II dissolved in a mixture of 5 ml THF and 0.5 ml DMF are added in small portions and the mixture is stirred at room temperature for 10 min. 0.04 mmol of a peptide conjugate from series I dissolved in 2 ml of DMF and 24 µl Hünig's base are added and the mixture is stirred for an additional hour at room temperature. The solvent is removed and the residue is purified by flash chromatography at silica gel. Appropriate eluent mixtures are:

Dichloromethane/methanol/ammonia 17% 15/3/0.3
Dichloromethane/methanol/ammonia 17% 16/2/0.2

The relevant fractions are collected, concentrated and the target products are isolated by precipitation from methanol/dichloromethane using ether. In many cases conjugates are transformed into respective sodium salts to improve water solubility. Therefore, the conjugates are suspended in water and 1-2 equivalents of a 0.01 N aqueous solution of sodium hydroxide is added until solution occurs and a pH of about 8 is reached. Subsequent lyophilisation yields the sodium salts of conjugates of integrin ligands with cytotoxic agents.

For TLC the following eluent systems have been used:

1) Dichloromethane/methanol/ammonia 17% 15/4/0.5
2) Acetonitrile/water/glacial acetic acid 10/1/0.1

**Example 1:**

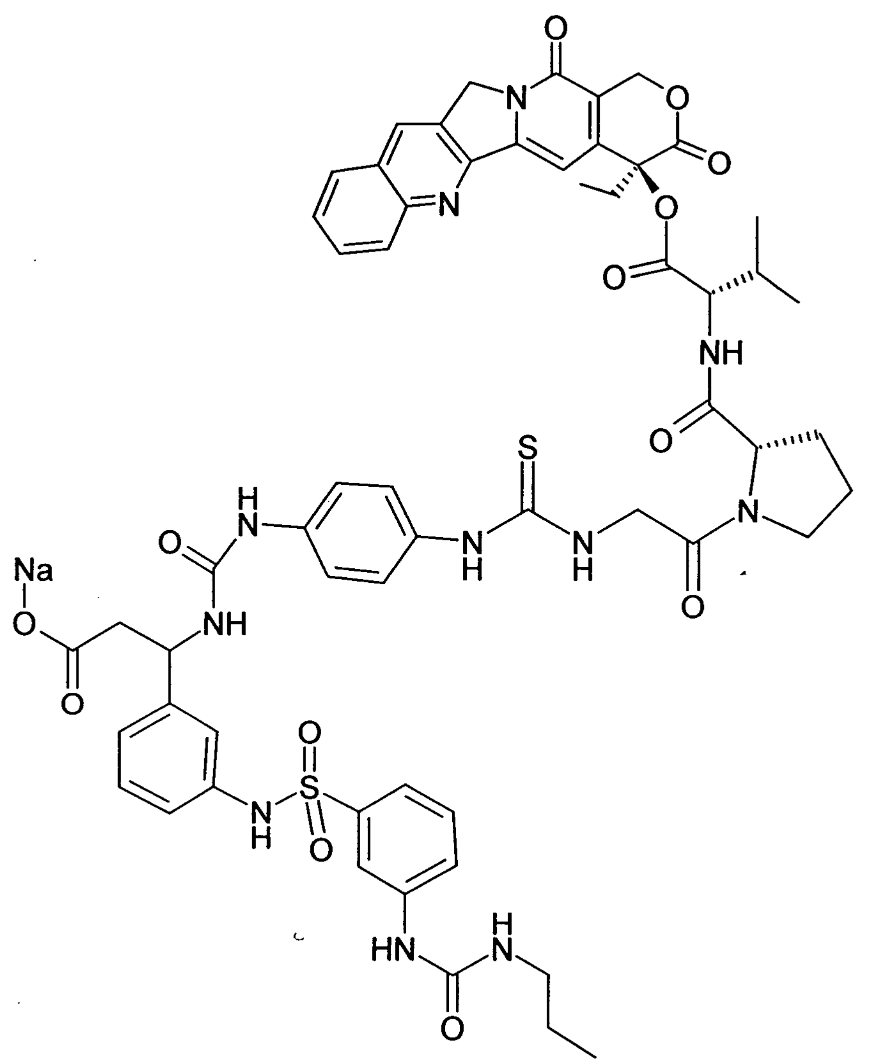

Diastereoisomer A:
Educts: I.4, II.2
Procedure: A
Yield: 51% (2 steps)
$R_f = 0.37$ 1)
[ESI-MS: m/e = 1198 = (M (acid) + H)$^+$]

**Example 2:**

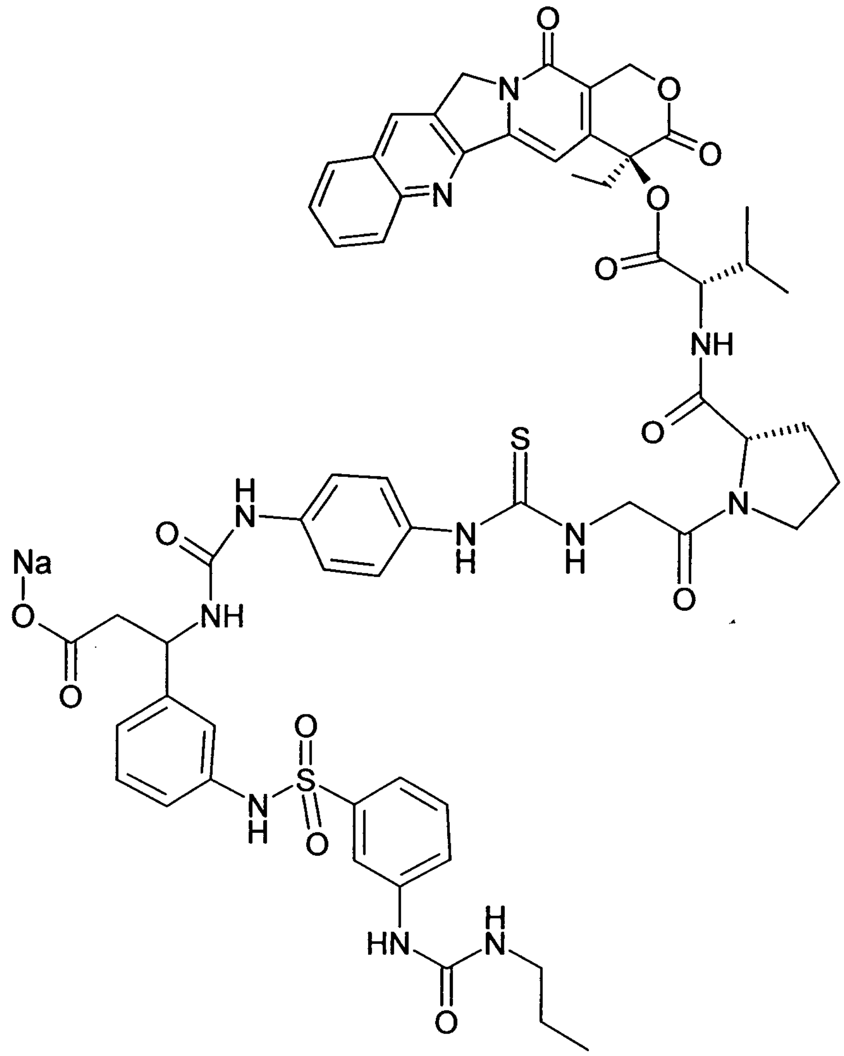

Diastereoisomer B:
Educts: I.4, II.3
Procedure: A
Yield: 51% (2 steps)
$R_f$ = 0.37 [1)]
[ESI-MS: m/e = 1198 = (M (acid) + H)$^+$]

**Example 3:**

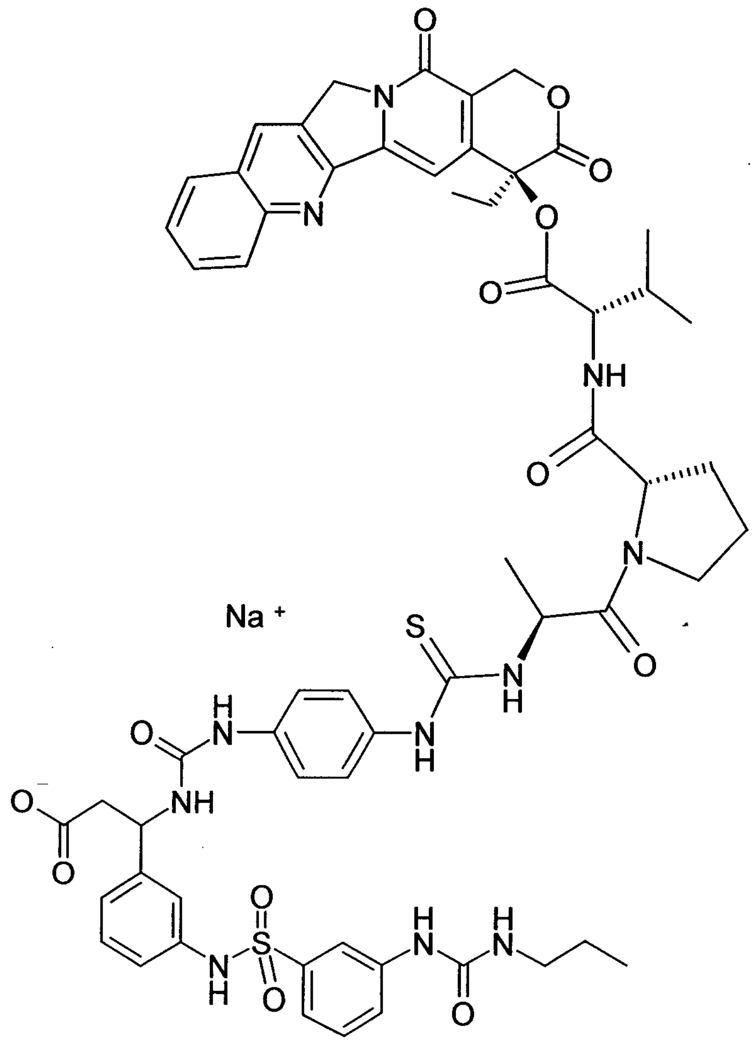

Diastereoisomer A:
Educts: I.5, II.2
Procedure: A
Yield: 69% (2 steps)
$R_f$ = 0.39 [1)]
[ESI-MS: m/e = 1212 = (M (acid) + H)$^+$]

**Example 4:**

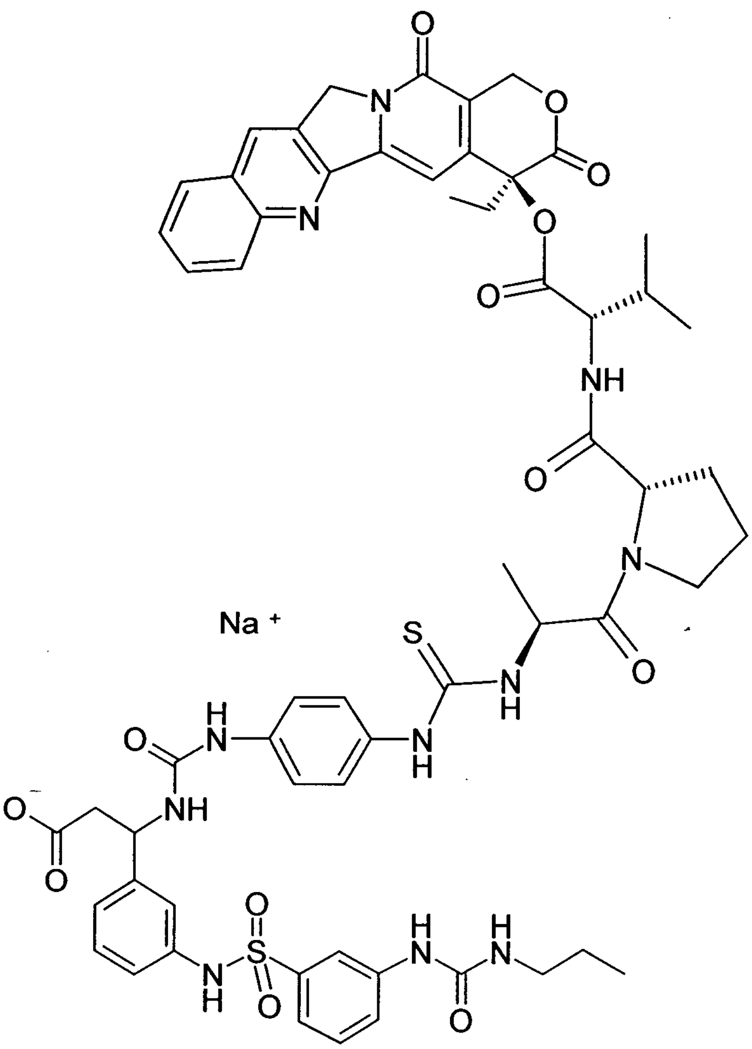

Diastereoisomer B:
Educts: I.5, II.3
Procedure: A
Yield: 66% (2 steps)
$R_f$ = 0.39 [1)]
[ESI-MS: m/e = 1212 = (M (acid) + H)$^+$]

**Example 5:**

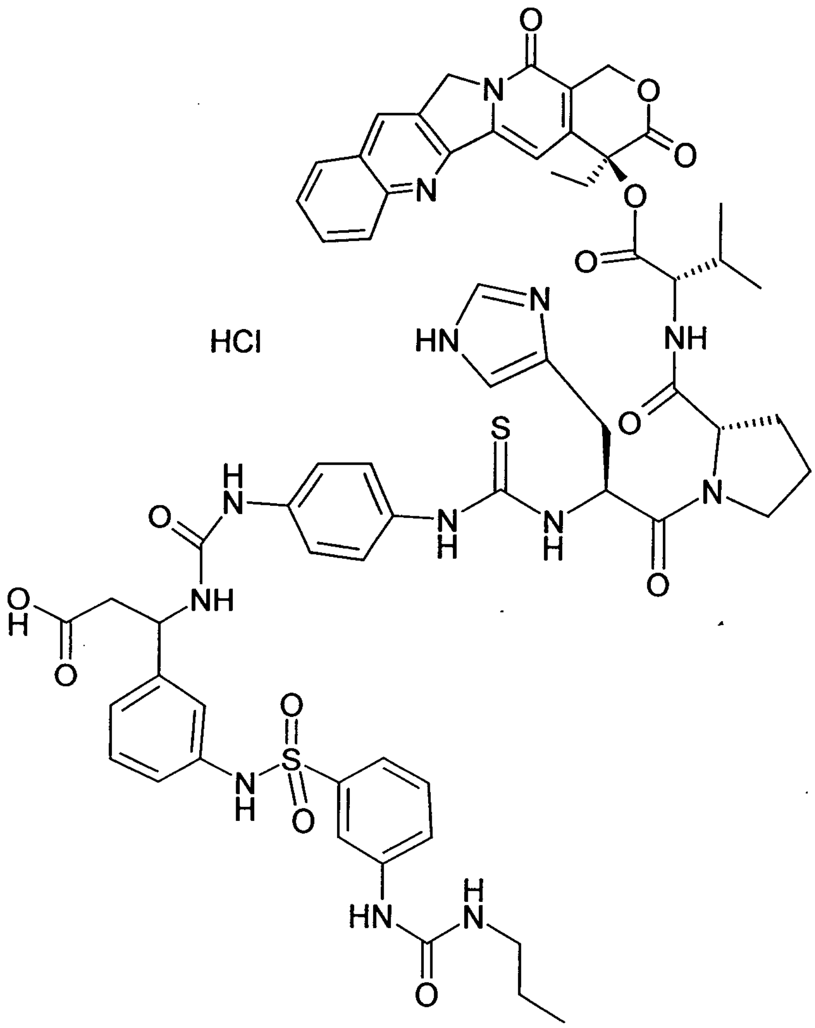

Diastereoisomer A:
Educts: I.8, II.2
Procedure: A
This compound is transformed into the hydrochloride, not into the sodium salt.
The betain is suspended in water and treated with 1 equivalent of 0.1 N aqueous HCl. The same volume of dioxane is added and the mixture is lyophilized.
Yield: 5% (2 steps)
$R_f = 0.45$ 1)
[ESI-MS: m/e = 1278 = (M (acid) + H)$^+$]

**Example 6:**

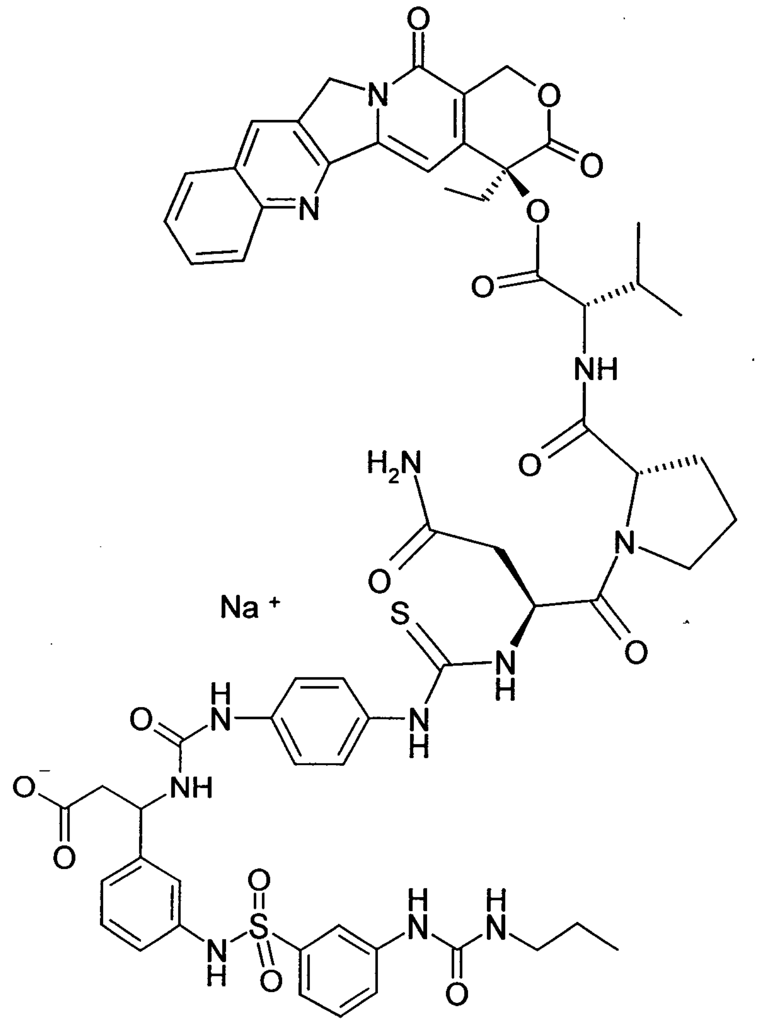

Diastereoisomer A:
Educts: I.7, II.2
Procedure: A
Yield: 44% (2 steps)
$R_f$ = 0.3 [1)]
[ESI-MS: m/e = 1255 = (M (acid) + H)$^+$]

**Example 7:**

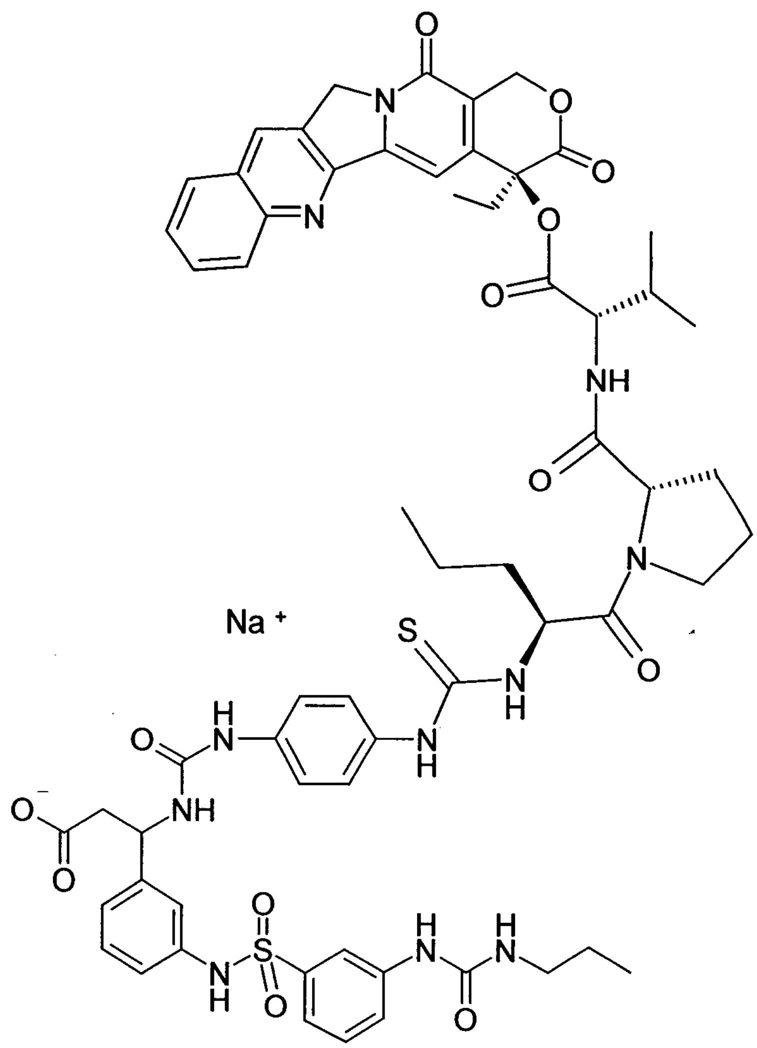

Diastereoisomer A:
Educts: I.9, II.2
Procedure: A
Yield: 57 % (2 steps)
$R_f$ = 0.44 [1)]
[ESI-MS: m/e = 1240 = (M (acid)+ H)$^+$]

**Example 8:**

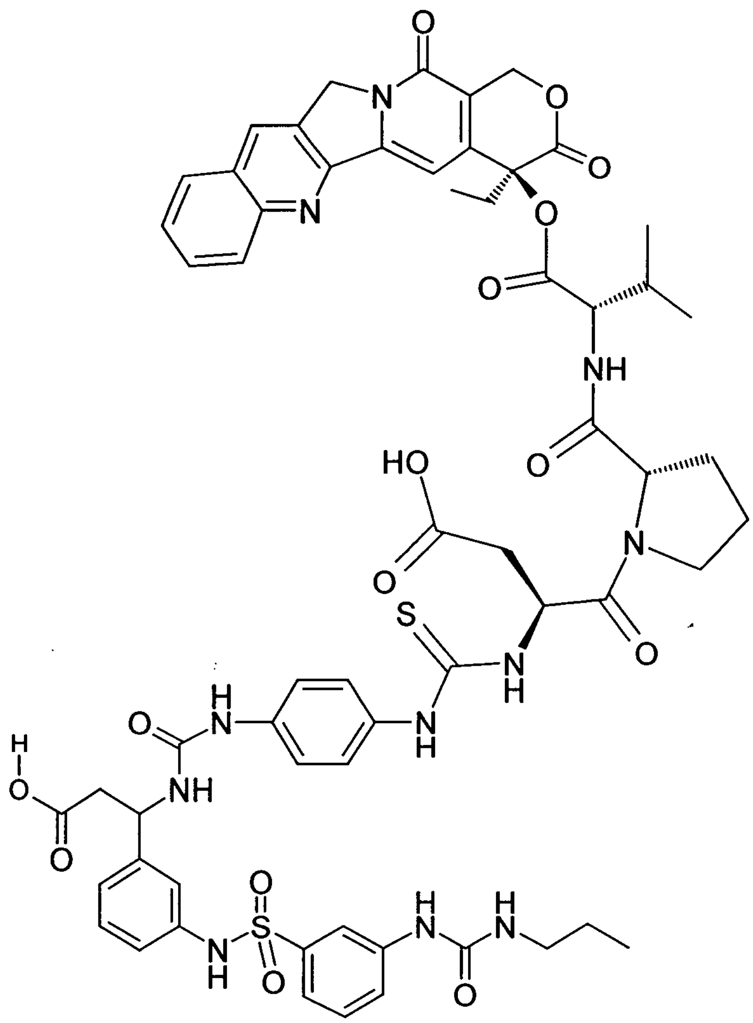

Diastereoisomer A:
Educts: I.6, II.2
Procedure: A, no transformation into sodium salt.
Yield: 12%
$R_f$ = 0.13 [1)]
[ESI-MS: m/e = 1256 = $(M + H)^+$]

**Example 9:**

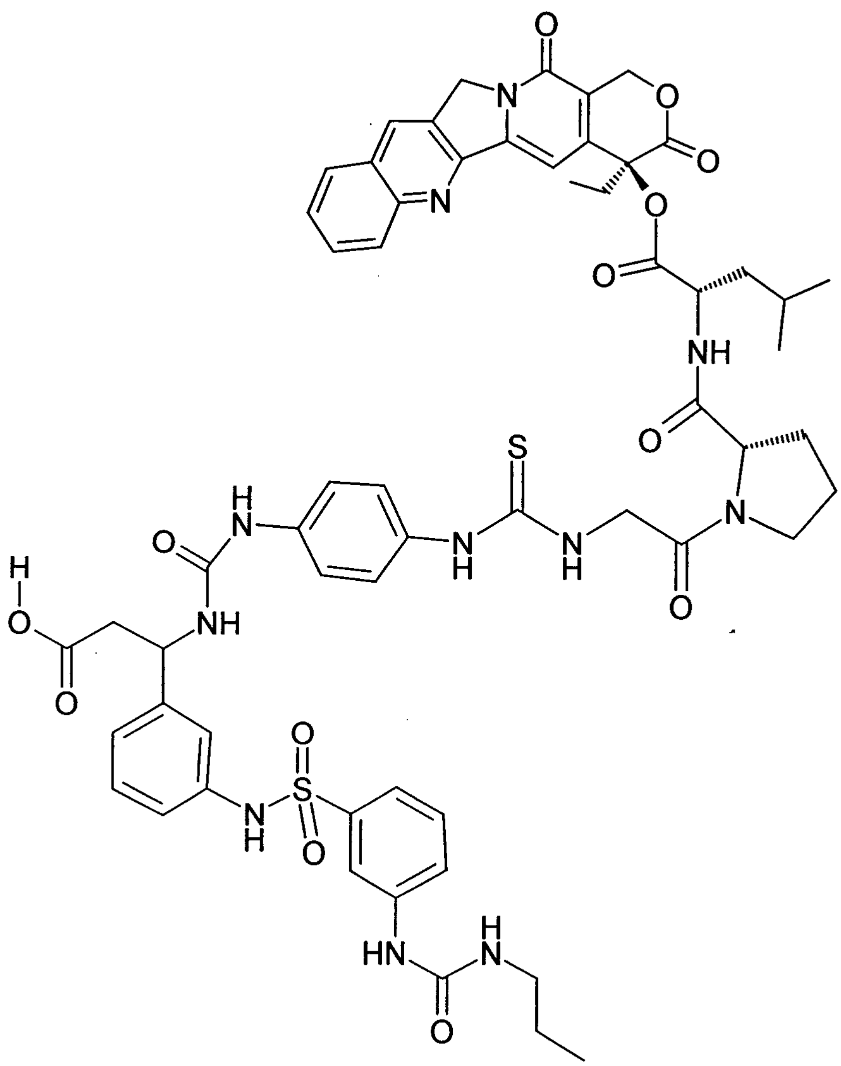

Diastereoisomer B:
Educts: I.10, II.3
Procedure: A, no transformation into sodium salt.
Yield: 40% (2 steps)
$R_f$ = 0.41 [1)]
[ESI-MS: m/e = 1212 = $(M + H)^+$]

**Example 10:**

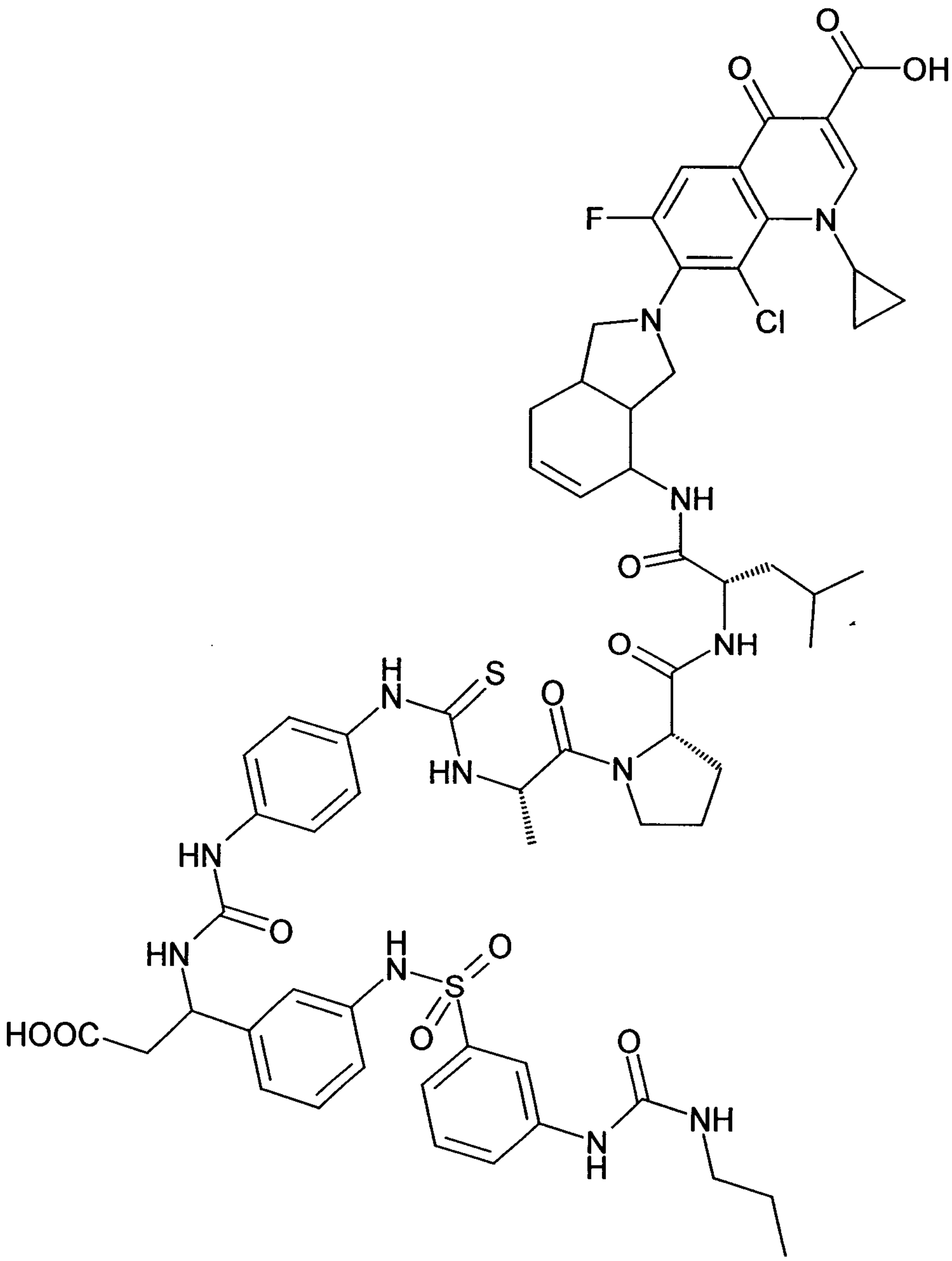

Diastereoisomer A:
Educts: I.12, II.2
Procedure: A, no transformation into sodium salt.
Purification by flash chromatography using acetonitrile/water 10/1 as eluent. Yield: 36%
$R_f = 0.5$ [2)]
[ESI-LC-neg: m/e = 1293 = $(M - H)^-$]

**Biological Tests**

**A: $\alpha_v\beta_3$ Binding test**

$\alpha_v\beta_3$ from human A375 cells was purified analogously to a procedure which was described by Wong et al. (Molecular Pharmacology, 50, 529-537 (1996)). In each case, 10 µl of $\alpha_v\beta_3$ (5 ng) in TBS pH 7.6, 2 mM $CaCl_2$, 1 mM $MgCl_2$, 1% n-octylglucopyranoside (Sigma); 10 µl of test substance in TBS pH 7.6, 0.1% DMSO and 45 µl of TBS pH 7.6, 2 mM $CaCl_2$, 1 mM $MgCl_2$, 1mM $MnCl_2$ were incubated at room temperature for 1 h. In each case, 25 µl of WGA SPA beads (Amersham, 4 mg/ml) and 10 µl of echistatin (0.1 µCi, Amersham, chloramine-T labelled) were then added. After 16 hours at room temperature, the samples were measured in a scintillation measuring apparatus (Wallac 1450). The test results are shown in Table 2 below.

Table 2:

| $IC_{50}$ values of the binding to the $\alpha_v\beta_3$ receptor [nM] | |
|---|---|
| Example | IC50 [nM] |
| 1 | 8 |
| 2 | 800 |
| 3 | 15 |
| 4 | 500 |
| 5 | 30 |
| 6 | 100 |
| 7 | 100 |
| 10 | 30 |

**B: Growth inhibition test for the determination of the cytotoxic properties on various tumour cell lines:**

The human large intestine cell lines SW 480 and HT29 (ATCC No. CCL 228 and HTB38 and the mouse melanoma cell line B16F10 (CRL 6475) were grown to confluence in Roux dishes in RPMI 1640 medium with addition of 10% FCS. They were then trypsinized and taken up in RPMI plus 10% FCS to a cell count of 50,000 cells or, for B16F10, 20,000 cells per ml. 100 µl of cell suspension/well were added to a 96 microwell plate and incubated at 37°C for 1 day in a $CO_2$ incubator. A further 100 µl of RPMI medium and 1 µl of DMSO were then added with the test substances. The growth was checked after day 6. For this, 25 µl of MTT solution (3-(4,5-dimethylthiazol-2-yl)-2,5-diphenyltetrazolium bromide) was added to each well at a starting concentration of 5 mg/ml of $H_20$. The plate was incubated at 37°C for 5 hours in a $CO_2$ incubator. The medium was then aspirated and 100 µl of i-propanol/well were added. After shaking with 100 µl of $H_2O$ for 30 min, the extinction was measured at 595 nm using a Multiplate Reader (BIO-RAD 3550-UV).

The cytostatic action is indicated in Table 3 as an $IC_{50}$ value, in each case for the individual cell lines.

Table 3:

| $IC_{50}$ values of the cytotoxic action on tumour cell lines [nM] | | | |
|---|---|---|---|
| | IC50 [nM] | | |
| Example | SW480 | HT29 | B16F10 |
| 1 | 150 | 60 | 160 |
| 2 | 400 | 150 | 400 |
| 3 | 500 | 150 | 700 |
| 4 | 600 | 150 | 700 |
| 5 | 700 | 300 | 1000 |
| 6 | 400 | 200 | 600 |
| 7 | 600 | 350 | 1000 |

Table 3: (continued)

| IC$_{50}$ values of the cytotoxic action on tumour cell lines [nM] | | | |
|---|---|---|---|
| | IC50 [nM] | | |
| Example | SW480 | HT29 | B16F10 |
| 8 | 50 | 50 | 300 |
| 9 | 70 | 30 | 80 |
| 10 | 1000 | 600 | 2000 |

**C. In-vivo inhibition or tumour growth using a nude mouse model**

*Material:*

In all in-vivo experiments for investigating the inhibition of tumour growth, athymic nude mice (NMRI nu/nu strain) were used. The tumour was developed by serial passage in nude mice. The human origin of the tumour was confirmed by isoenzymatic and immunohistochemical methods.

*Experimental set-up:*

The tumour was implanted subcutaneously in both flanks of nu/nu nude mice 6 to 8 weeks old. The treatment was started, depending on the doubling time, as soon as the tumours had reached a diameter of 5 - 7 mm. The mice were assigned to the treatment group or the control group (5 mice per group having 8 - 10 assessable tumours) by randomization. The individual tumours of the control group all grew progressively.

The size of the tumours was measured in two dimensions by means of a slide gauge. The tumour volume, which correlated well with the cell count, was then used for all assessments. The volume was calculated according to the formula "length x breadth x breadth / 2" ([a x $b^2$] / 2, a and b represent two diameters arranged at right angles).

The values of the relative tumour volume (RTV) were calculated for each individual tumour by dividing the tumour size on day X with the tumour size on day 0 (at the time of randomization). The average values of the RTV were then used for the further assessment.

The inhibition of the increase of the tumour volume (tumour volume of the test group/control group, T/C, in per cent) was the final measured value.

*Treatment:*

The compounds can be administered with a daily or an intermittent therapy schedule through a couple of days either by intraperitoneal, intravenious, oral or subcutaneous route.

In a subcutaneously growing melanoma xenograft model (MEXF 989) several compounds effected inhibitions of the tumor growth (eg. compound of example 1 and 3). The compounds are dissolved in 1% aqueous dextrose solution and administered intravenously from day 1-3 and day 15-17. The optimal calculated T/C values are given in table 4.

Table 4:

| Example | dose | lethality | optimal T/C in % |
|---|---|---|---|
| 1 | 18 mg/kg/day | 1/5 | 7.2 |
| 1 | 24 mg/kg/day | 1/5 | 3.2 |
| 3 | 6mg/kg/day | 0/5 | 10.3 |

Furthermore, in a subcutaneously growing renal xenograft model (RXF 944) several compounds also effected inhibitions of the tumor growth (eg. compound of example 1 and 3). The compounds are dissolved in 1% aqueous dextrose solution and administered intravenously from day 1-3 and day 15-17. The optimal calculated T/C values are given in table 53.

Table 5:

| Example | dose | lethality | optimal T/C in % |
|---|---|---|---|
| 1 | 18 mg/kg/day | 1/7 | 12.4 |
| 1 | 24 mg/kg/day | 0/7 | 5.1 |
| 3 | 6mg/kg/day | 1/7 | 24.0 |
| camptothecin | 2.5 mg/kg/day | 0/6 | 41.1 |
| topotecan | 1.8 mg/kg/day) | 0/7 | 47.8 |

Furthermore, in a subcutaneously growing breast cancer model model (MX-1) several compounds also effected inhibitions of the tumor growth (eg. compound of example 1 and 3). The compounds are dissolved in 1% aqueous dextrose solution and administered intravenously from day 1-3 and day 15-17. The optimal calculated T/C values are given in table 6.

Table 6:

| Example | dose | lethality | optimal T/C in % |
|---|---|---|---|
| 1 | 18 mg/kg/day | 0/5 | 1.7 |
| 3 | 6mg/kg/day | 0/5 | 12.4 |

**D. CSF-induced proliferation of hemopoietic stem cells**

Bone marrow cells are flushed out of the femur of mice. $10^5$ cells are incubated in McCoy 5A medium (0.3% agar) together with recombinant murine GM-CSF (Genzyme; parent cell colony formation) and the substances ($10^{-4}$ to 100 µg/ml) at 37°C and 7% $CO_2$. 7 days later, the colonies (<50 cells) and clusters (17-50 cells) are counted.
A series of compounds exhibits a drastically reduced toxicity against stem cells in vitro compared to camptothecin.

Table 7:

| $IC_{50}$ values of toxicity against hemopoietic stem cells [ng / ml] | |
|---|---|
| Example | $IC_{50}$[ng/ml] |
| 1 | 300 |
| 2 | 300 |
| 3 | 40 |
| 4 | 50 |
| Camptothecin | 3 |

**E. Cleavage of conjugates by elastase in culture medium of HT29**

Table 8:

| Ratio of peak areas of released camptothecin vs. conjugate after 24 incubation in the supernatant of HT29 supplemented with elastase | |
|---|---|
| Example | elastase mediated cleavage Peak area product / educt |
| 1 | 43/53 |
| 2 | 33/63 |
| 3 | 96/1 |
| 4 | 93/4 |

Table 8: (continued)

| Ratio of peak areas of released camptothecin vs. conjugate after 24 incubation in the supernatant of HT29 supplemented with elastase | |
|---|---|
| Example | elastase mediated cleavage Peak area product / educt |
| 6 | 91/0 |
| 7 | 95/0 |

## Claims

**1.** Conjugate, **characterized by** the formula (I)

$$CT - LI - Sp - IA \quad (I)$$

in which

CT denotes a cytotoxic radical or a radical of a cytostatic or of a cytostatic derivative, which can additionally carry a hydroxyl, carboxyl or amino group,

LI is a linker group comprising 3 amino acid residues in the D or L configuration, which can each optionally carry protective groups ,

Sp is absent or a carbonyl or a thiocarbonyl radical,

IA is a non-peptide radical addressing an $\alpha_v\beta_3$ integrin receptor, which is selected from the group consisting of a radical of the formula (II)

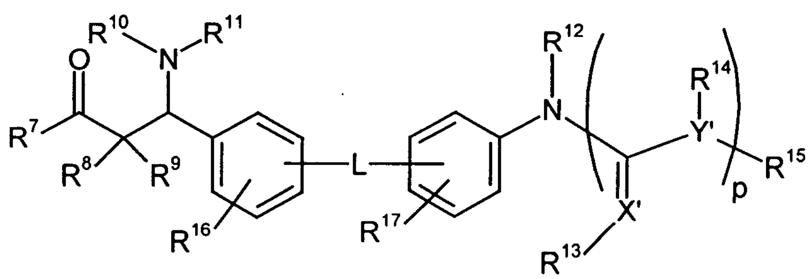

(II)

in which

$R^7$ is OH, a substituted or unsubstituted alkoxy or cycloalkoxy radical, a substituted or unsubstituted aryloxy radical or a saturated or unsaturated, optionally substituted heterocyclyloxy radical, or optionally represents a direct bond or an atom from the group consisting of N, O and S, via which the radical of the formula (II) is bonded to the rest of the conjugate;

$R^8$ is hydrogen, a substituted or unsubstituted alkyl or cycloalkyl radical, a substituted or unsubstituted aryl radical, a saturated or unsaturated, optionally substituted heterocyclic radical, an optionally substituted alkenyl radical, an optionally substituted alkinyl radical, a hydroxyl radical or an alkoxy radical or is bonded to $R^9$ with formation of an optionally substituted carbocyclic or heterocyclic ring system which includes the carbon atom to which $R^8$ is bonded and can optionally contain heteroatoms;

$R^9$ is hydrogen, a substituted or unsubstituted alkyl or cycloalkyl radical, a substituted or unsubstituted aryl radical, a saturated or unsaturated, optionally substituted heterocyclic radical, an optionally

substituted alkenyl radical, an optionally substituted alkinyl radical, a hydroxyl radical or an alkoxy radical or is bonded to $R^8$ with formation of an optionally substituted carbocyclic or heterocyclic ring system which includes the carbon atom to which $R^9$ is bonded and can optionally contain heteroatoms;

$R^{10}$ is $-SO_2R^{10'}$, $-COOR^{10''}$, $-COR^{10'}$, $-CONR^{10'}_2$ or $-CS-NR^{10'}_2$, or represents a direct bond via which the radical of the formula (II) is optionally bonded to the rest of the conjugate;

$R^{10'}$ independently of one another is hydrogen, a substituted or unsubstituted alkyl or cycloalkyl radical, a substituted or unsubstituted aryl radical or a saturated or unsaturated, optionally substituted heterocyclic radical, via which the radical of the formula (II) is optionally bonded to the rest of the conjugate;

$R^{10''}$ is a substituted or unsubstituted alkyl or cycloalkyl radical, a substituted or unsubstituted aryl radical or a saturated or unsaturated, optionally substituted heterocyclic radical, via which the radical of the formula (II) is optionally bonded to the rest of the conjugate;

R" is hydrogen, a substituted or unsubstituted alkyl or cycloalkyl radical or a substituted or unsubstituted aryl radical,

$R^{16}$ is hydrogen, CN, a substituted or unsubstituted alkyl or cycloalkyl radical, a substituted or unsubstituted alkoxy radical or a halogen atom;

$R^{17}$ is hydrogen, CN, a substituted or unsubstituted alkyl or cycloalkyl radical, a substituted or unsubstituted alkoxy radical or a halogen atom;

L is $-(CH_2)_nNHSO_2(CH_2)_o-$, $-(CH_2)_nSO_2NH(CH_2)_o-$, $-(CH_2)_nNH-CO(CH_2)_o-$, $-(CH_2)_nCONH(CH_2)_o-$, $-(CH_2)_nOCH_2(CH_2)_o-$, $-(CH_2)_nCH_2O(CH_2)_o-$, $-(CH_2)_nCOO(CH_2)_o-$, $-(CH_2)_nOOC-(CH_2)_o-$, $-(CH_2)_nCH_2CO(CH_2)_o-$, $-CH_2)_nCOCH_2(CH_2)_o-$, -NHCONH-, $-(CH_2)_nSCH_2(CH_2)_o-$, $-(CH_2)_nCH_2S(CH_2)_o-$, $-(CH_2)_nCH_2SO(CH_2)_o-$, $-(CH_2)_nSOCH_2(CH_2)_o-$, $-(CH_2)_nCH_2SO_2(CH_2)_o-$ or $-(CH_2)_nSO_2CH_2(CH_2)_o-$,
where n and o each is an integer of 0 or 1 and $n + o \leq 1$;

$R^{12}$ is hydrogen, a substituted or unsubstituted alkyl or cycloalkyl radical, a substituted or unsubstituted aryl radical, a saturated. or unsaturated, optionally substituted heterocyclic radical or is bonded to one of $R^{13}$, $R^{14}$ or $R^{15}$, if present, with formation of an optionally substituted heterocyclic ring system which includes the nitrogen atom, to which $R^{12}$ is bonded and can be saturated or unsaturated and/or can contain further heteroatoms;

X' is N, O or S;

p is 0 or 1;

$R^{13}$ is absent, is -H, a substituted or unsubstituted alkyl or cycloalkyl radical, $-NO_2$, -CN, $-COR^{13'}$, $-COOR^{13'}$, or is bonded to one of $R^{12}$, $R^{14}$ or $R^{15}$ with formation of an optionally substituted heterocyclic ring system which includes X' and can be saturated or unsaturated and/or can contain further heteroatoms;

$R^{13'}$ is hydrogen, a substituted or unsubstituted alkyl or cycloalkyl radical, a substituted or unsubstituted aryl radical or a saturated or unsaturated, optionally substituted heterocyclic radical which can be saturated or unsaturated and/or can contain further heteroatoms;

Y' is N or S;

$R^{14}$ is absent, hydrogen, a substituted or unsubstituted alkyl or cycloalkyl radical, a substituted or unsubstituted aryl radical, a saturated or unsaturated, optionally substituted heterocyclic radical or is bonded to one of $R^{12}$, $R^{13}$ or $R^{15}$, if present, with formation of an optionally substituted heterocyclic ring system which includes the nitrogen atom to which $R^{14}$ is bonded and can be saturated or unsaturated and/or can contain further heteroatoms;

$R^{15}$ is hydrogen, a substituted or unsubstituted alkyl or cycloalkyl radical, a substituted or unsubstituted aryl radical, a saturated or unsaturated, optionally substituted heterocyclic radical or is bonded to one of $R^{12}$, $R^{13}$ or $R^{14}$, if present, with formation of an optionally substituted heterocyclic ring system which includes the nitrogen atom to which $R^{15}$ is bonded and can be saturated or unsaturated and/or can contain further heteroatoms, or optionally represents a direct bond via which the radical of the formula (II) is bonded to the rest of the conjugate;

and their physiologically acceptable salts and stereoisomers.

**2.** Conjugate according to Claim 1, **characterized in that**

LI is a linker group having the formula

-AA1-AA2-AA3-

wherein AA1 is bonded to the radical CT and

AA1 is a naturally occurring amino acid in the D or L configuration, which is selected from the group consisting of glycine, alanine, valine, leucine, isoleucine, histidine, glutamate, aspartate, serine, lysine, ornithine and phenylalanine;

AA2 is a naturally occurring amino acid in the D or L configuration, which is selected from the group consisting of alanine, valine, phenylalanine, tyrosine, threonine, serine, isoleucine, lysine, glutamate, histidine, glycine, arginine, asparagine, glutamine, S-methyl-cysteine, methionine, arginine, aspartate, tryptophane, proline, ornithine and leucine, and can optionally carry protective groups,

AA3 is a naturally occurring amino acid in the D or L configuration, which is selected from the group consisting of alanine, valine, phenylalanine, tyrosine, serine, isoleucine, lysine, glutamine, glutamate, histidine, glycine, arginine, asparagine, aspartate, tryptophane, proline, ornithine, methionine, S-methyl-cysteine, norvaline and leucine, and can optionally carry protective groups,

and the other radicals CT, Sp and IA are as defined in claim 1.

**3.** Conjugate according to claim 2, **characterized in that**

LI is a linker group having the formula

-AA1-AA2-AA3

wherein AA1 is bonded to the radical CT and

AA1 is valine, leucine, isoleucine;

AA2 is proline,

AA3 is alanine, norvaline, histidine, glycine, asparagine, aspartate,

and the other radicals CT, Sp and IA are as defined in claim 1.

**4.** Conjugate according to one of the preceeding claims, **characterized in that**

CT is camptothecin, which can be linked to the rest of the conjugate via the C20-OH group;

LI is as defined in one of claim 1 to 3;

Sp is absent, or a carbonyl or a thiocarbonyl radical,

IA is a non-peptide radical of the formula (II) addressing an $\alpha_v\beta_3$ integrin receptor, in which

$R^7$ is OH, methoxy, ethoxy, propoxy, isopropoxy, butoxy, isobutoxy, t-butoxy, pentoxy, isopentoxy, neopentoxy, hexoxy, cyclopropoxy, cyclopropylmethoxy, cyclobutoxy, cyclopentoxy, cyclohexoxy, phenoxy, benzyloxy, tolyloxy or a substituted derivative thereof, or optionally represents a direct bond or an atom from the group consisting of N, O and S, via which the radical of the formula (II) is bonded to the rest of the conjugate;

$R^8$ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -OH, methoxy, ethoxy, propoxy, butoxy, pentoxy, hexoxy, benzyloxy or is bonded to $R^9$ with formation of an optionally substituted 3- to 6-membered carbocyclic or heterocyclic ring system, which includes the carbon atom to which $R^8$ is bonded and can optionally contain heteroatoms;

$R^9$ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, -OH, methoxy, ethoxy, propoxy, butoxy, pentoxy, hexoxy or is bonded to $R^8$ with formation of an optionally substituted 3- to 6-membered carbocyclic or heterocyclic ring system which includes the carbon atom to which $R^9$ is bonded and can optionally contain heteroatoms;

$R^{10}$ is $SO_2R^{10'}$, $-COOR^{10''}$, $-COR^{10'}$, $-CONR^{10'}_2$ or $-CSNR^{10'}_2$ or represents a direct bond, via which the radical of the formula (II) is optionally bonded to the rest of the conjugate;

$R^{10'}$ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof, $-C_6H_2(CH_3)_3$, $-C_6(CH_3)_5$, $-CH_2C_6H_2(CH_3)_3$, 2-chlorophenyl, 3-chlorophenyl, 4-chlorophenyl, 2,3-dichlorophenyl, 2,4-dichlorophenyl, 3,4-dichlorophenyl, 2,5-dichlorophenyl, 3,5-dichlorophenyl, 2,6-dichlorophenyl, 4-chlorophenylmethyl, 2,4-dichloro-phenylmethyl, 2,6-dichlorophenylmethyl, 3-aminophenyl, 4-aminophenyl, 3-carboxyphenyl, 2-methoxycarbonylphenylmethyl, 3-trifluoromethylphenyl, 4-trifluoromethylphenyl, 3,5-bis(trifluoromethyl)phenyl, 4-trifluoromethoxyphenyl, phenylmethyl, 2-acetamido-4-methylthiazol-5-yl, phenylethyl, 1-phenylpropyl, (S)-(+)-camphor-10-yl, (R)-(-)-camphor-10-yl, 2-phenylethenyl, 2-thiophenyl, 4-methoxyphenyl, 3,5-dimethoxyphenyl, 3-methylphenyl, 4-methylphenyl, 4-t-butylphenyl, 4-propylphenyl, 2,5-dimethylphenyl, 2-methoxy-5-methylphenyl, 2,3,5,6-tetramethylphenyl, 1-naphthyl, 2-naphthyl, 4-fluorophenyl, 2,4-difluorophenyl, 2-chloro-6-methylphenyl, 2-chloro-4-fluorophenyl, 2,5-dimethoxyphenyl, 3,4-dimethoxyphenyl, 3-chloro-6-methoxyphenyl, 2-trifluoromethylphenyl, 2-alkylsulphonylphenyl, 2-arylsulphonylphenyl, 3-(N-acetyl-6-methoxy)aniline, 4-acetamidophenyl, 2,2,2-trifluoroethyl, 5-chloro-3-methylbenzothiazol-2-yl, N-methoxycarbonyl-piperidin-3-yl, thiophen-2-yl, isoxazol-5-yl, ethoxy, 2-chloropyridin-3-yl, pyridin-3-yl, benzyloxy, 5-methylisoxazol-3-yl, 1-adamantyl, 4-chlorophenoxymethyl, 2,2-dimethylethenyl, 2-chloropyridine-5-methyl, 5,7-dimethyl-1,3,4-triazaindolizin-2-yl, (S)-camphan-1-yl, (R)-camphan-1-yl or 8-quinolinyl;

$R^{10''}$ is a $C_{1-6}$-alkyl radical, a $C_{3-7}$-cycloalkyl radical, a substituted or unsubstituted aryl radical or a saturated or unsaturated, optionally substituted heterocyclic radical, via which the radical of the formula (II) is optionally bonded to the rest of the conjugate;

$R^{11}$ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl, dialkylamino-$C_{1-4}$-alkyl, amino-$C_{1-4}$-alkyl, $C_{1-4}$-alkyloxy-$C_{1-4}$-alkyl or

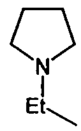 , 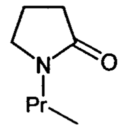 , 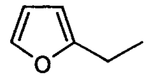 , 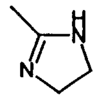 , 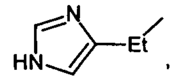 ,

(a1) (a2) (a3) (a4) (a5)

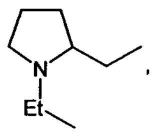 , 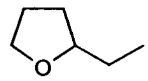 , 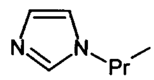 , 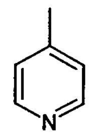 , 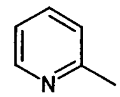 ,

(a6) (a7) (a8) (a9) (a10)

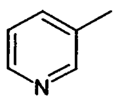 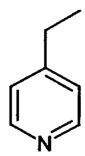 , 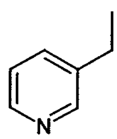 ; 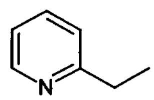 , 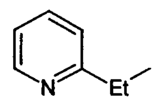 ,

(a11) (a12) (a13) (a14) (a15)

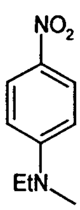 , 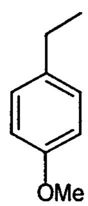 , 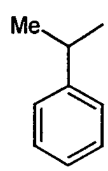 , 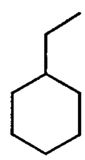 , 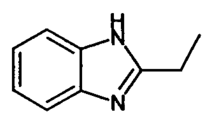

(a16) (a17) (a18) (a19) (a20)

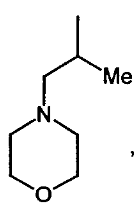 , 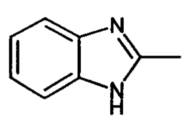 , 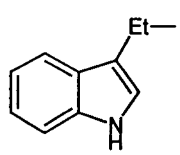 , 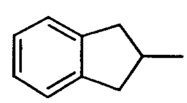 ,

(a21) (a22) (a23) (a24)

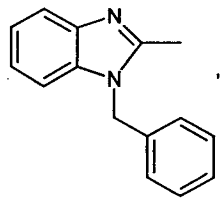 , 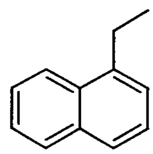 , 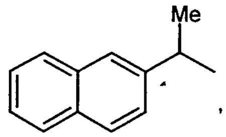 , 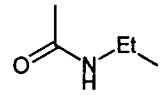 ,

(a25) (a26) (a27) (a28)

$R^{16}$ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;

$R^{17}$ is hydrogen, CN, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, methoxy, trifluoromethoxy, ethoxy, propoxy, butoxy, pentoxy or hexoxy, fluorine, chlorine, bromine or iodine;

L is $-NHSO_2-$, $-CH_2NHSO_2-$, $-NHSO_2CH_2-$, $-SO_2NH-$, $-CH_2SO_2NH-$, $-SO_2NHCH_2-$, -NHCO-, $-CH_2NHCO-$, $-NHCOCH_2-$, -CONH-, $-CH_2CONH-$, $-CONHCH_2-$, $-OCH_2-$, $-CH_2OCH_2$, $-OCH_2CH_2-$, $-CH_2O-$ or $-CH_2CH_2O-$;

$R^{12}$ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl, one of the radicals (a1) to (a28) or is bonded to one of $R^{13}$, $R^{14}$ or $R^{15}$, if present, with formation of an optionally substituted heterocyclic 4- to 6-membered ring system which includes the nitrogen atom to which $R^{12}$ is bonded and can be saturated or unsaturated and/or can contain further heteroatoms;

X' is N, O or S;

p is 0 or 1;

$R^{13}$ is absent, is -H, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, $-NO_2$, -CN, $-COR^{7'}$, $-COOR^{7'}$, or is connected to one of $R^{12}$, $R^{14}$ or $R^{15}$ with formation of an optionally substituted carbocyclic or heterocyclic 4- to 6-membered ring system which includes X' and can be saturated or unsaturated and/or can contain further heteroatoms;

$R^{13'}$ is hydrogen, methyl, ethyl, propyl, isopropyl, butyl, isobutyl, t-butyl, pentyl, isopentyl, neopentyl, hexyl, cyclopropyl, cyclobutyl, cyclopentyl, cyclohexyl, cycloheptyl, phenyl, benzyl, tolyl or a substituted derivative thereof;

Y' is N or S;

$R^{14}$ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl, one of the radicals (a1) to (a28), or is bonded to one of $R^{12}$, $R^{13}$ or $R^{15}$, if present, with formation of an optionally substituted heterocyclic 4- to 6-membered ring system which includes the nitrogen atom to which $R^{14}$ is bonded and can be saturated or unsaturated and/or can contain further heteroatoms; and

$R^{15}$ is hydrogen, methyl, ethyl, propyl, isopropyl, butyl, isobutyl, t-butyl, pentyl, isopentyl, neopentyl, hexyl, cyclopropyl, cyclopropylmethyl, cyclobutyl, cyclopentyl, cyclohexyl, cycloheptyl, 4-methylcyclohexyl, 3,3,5-trimethylcyclohexyl, 5-methyl-2-hexyl, phenyl, benzyl, tolyl or a substituted derivative thereof, $C_{1-4}$-alkylamino-$C_{1-4}$-alkyl, $C_{1-4}$-dialkylamino-$C_{1-4}$-alkyl, amino-$C_{1-4}$-alkyl, $C_{1-4}$-alkyloxy-$C_{1-4}$-alkyl, one of the radicals (a1) to (a28) or is bonded to one of $R^{12}$, $R^{13}$ or $R^{14}$, if present, with formation of an optionally substituted heterocyclic 4- to 6-membered ring system which includes the nitrogen atom to which $R^{15}$ is bonded and can be saturated or unsaturated and/or can contain further heteroatoms, and or optionally represents a direct bond via which the radical of the formula (II) is bonded to the rest of the conjugate.

**5.** Conjugate according to Claim 4, **characterized in that**

$R^7$ represents a direct bond or an atom from the group consisting of N, O and S, via which the radical of the formula (II) is bonded to the rest of the conjugate;

and the other radicals of the formula (II) are as defined in Claim 4.

**6.** Conjugate according to Claim 4, **characterized in that**

$R^{15}$ represents a direct bond, via which the radical of the formula (II) is bonded to the rest of the conjugate;

and the other radicals of the formula (II) are as defined in Claim 4.

**7.** Conjugate according to Claim 4, **characterized in that**
the radical of the formula (II) is linked to the rest of the conjugate via a radical in the α- or β-position relative to the carboxyl group,
and the other radicals of the formula (II) are as defined in Claim 4.

**8.** Conjugate according to claim 7, **characterized in that**

CT is camptothecin which is linked to the rest of the conjugate via the C20-OH group;

LI is as defined in claim 3;

Sp is a thiocarbonyl radical,

IA is a non-peptide radical of the formula (II) addressing an $\alpha_v\beta_3$ integrin receptor, in which

| | |
|---|---|
| $R^7$ | is OH; |
| $R^8$, $R^9$, $R^{12}$, $R^{14}$, $R^{16}$ and $R^{17}$ | are H; |
| $R^{10}$ | is $CONHR^{10'}$; |
| $R^{10'}$ | is -Ph-NH-, wherein the radical of the formula (II) is linked to the rest of the conjugate via the nitrogen atom in $R^{10'}$; |
| L | is $-NH-SO_2-$ being linked to the adjacent phenylene units such that each of these phenylene units is 1,3-substituted; |
| X' | is O; |
| $R^{13}$ | is absent; |
| P | is 1; |
| R15 | is propyl. |

**9.** Process for the preparation of conjugates according to Claim 1, comprising

[A] the reaction of a compound from the group of compounds of the formula (II), which has a free or optionally activated carboxyl function,

with a compound of the formula (Ia) which has a free primary or secondary amino group

CT-LI (Ia)

in which all radicals have the meaning indicated in Claim 1,

in the presence of a base;

or

[B] the reaction of a compound from the group of compounds of the formula (II) which has a free primary or secondary amino function,

with a carbonic acid derivative such as, for example, phosgene, thiophosgene or a chloroformic acid ester, if appropriate in the presence of a base,

followed by the reaction with a compound of the formula (Ia) which has a free primary or secondary amino group

CT-LI (ia)

in which all radicals have the meaning indicated in Claim 1,

and

if appropriate the removal of protective groups and/or derivatization of nitrogen atoms present at preferred points of time in the preparation process and/or conversion of the compound obtained into the free acid and/or conversion of the compound obtained into one of its physiological salts by reaction with an inorganic or organic base or acid;

or

[C] the reaction of a compound from the group of compounds of the formula (II) which contains a free primary or secondary amino function,

with a compound of the formula (Ia) which contains a free or optionally activated carboxyl function

CT-LI (Ia)

in which all radicals have the meaning indicated in Claim 1,

in the presence of a base;

and

if appropriate the removal of protective groups and/or derivatization of nitrogen atoms present at preferred points in time in the preparation process and/or conversion of the compound obtained into the free acid and/or conversion of the compound obtained into one of its physiological salts by reaction with an inorganic or organic base or acid.

**10.** Process according to Claim 9, **characterized in that** several steps of the process are carried out on a solid phase.

**11.** Compounds according to one of claims 1 to 8 for treating diseases.

**12.** Medicament, comprising at least one of the conjugates according to one of Claims 1 to 8.

**13.** Use of compounds according to one of Claims 1 to 8 for the production of medicaments for the treatment of carcinomatous disorders.

European Patent Office

# EUROPEAN SEARCH REPORT

Application Number
EP 01 10 5350

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y,D | WO 00 41469 A (BAYER AKTEINGESELLSCHAFT) 20 July 2000 (2000-07-20)<br>* the whole document *<br>--- | 1-13 | A61K47/48 |
| Y | WO 99 51638 A (G.D. SEARLE & CO.) 14 October 1999 (1999-10-14)<br>* page 9, line 24 - page 11, line 20 *<br>* page 11, line 17 - page 11, line 19 *<br>--- | 1-13 | |
| Y,D | WO 00 69472 A (BOEHRINGER INGELHEIM PHARMACEUTICALS, INC.) 23 November 2000 (2000-11-23)<br>* claims *<br>--- | 1-13 | |
| Y | DE MARRE A. ET AL: "Synthesis and evaluation of macromolecular prodrugs of mitomycin C"<br>J. OF CONTROLLED RELEASE,<br>vol. 36, 1995, page 87-97 XP004037471<br>* abstract *<br>* page 91, right-hand column, line 6 - page 92, left-hand column, line 5 *<br>--- | 1-13 | TECHNICAL FIELDS SEARCHED (Int.Cl.7) |
| Y | SOYEZ ET AL: "Macromolecular derivatives of N,N-di-(2-chloroethyl)-4-phenylene diamine mustard. "<br>J. OF CONTROLLED RELEASE,<br>vol. 57, 1999, page 187-196 XP004157775<br>* abstract *<br>* page 192, left-hand column, line 5 - page 192, left-hand column, line 15 *<br>--- | 1-13 | A61K |
| A,D | WO 98 10795 A (THE BURNHAM INSTITUTE) 19 March 1998 (1998-03-19)<br>* page 46, paragraph 2 *<br>* page 56 - page 58 *<br>---<br>-/-- | 1-13 | |

The present search report has been drawn up for all claims

| Place of search | Date of completion of the search | Examiner |
|---|---|---|
| MUNICH | 28 June 2001 | Gohlke, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 01 10 5350

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | KASAFTREK E. ET AL: "Role of amino acid residues in chromogenic substrates cleaved by pancreatic elastase" COLLECTION CZECHOSLOVAK CHEM. COMMUN., vol. 52, 1987, page 1625-1633 XP002147482 * abstract * | 1-13 | |
| E | WO 01 17563 A (BAYER AKTIENGESELLSCHAFT) 15 March 2001 (2001-03-15) * the whole document * | 1-13 | |
| | | | TECHNICAL FIELDS SEARCHED (Int.Cl.7) |

The present search report has been drawn up for all claims

| Place of search | Date of completion of the search | Examiner |
|---|---|---|
| MUNICH | 28 June 2001 | Gohlke, P |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 10 5350

This annex lists the patent family members relating to the patent documents cited in the above-mentioned European search report.
The members are as contained in the European Patent Office EDP file on
The European Patent Office is in no way liable for these particulars which are merely given for the purpose of information.

28-06-2001

| Patent document cited in search report | Publication date | Patent family member(s) | Publication date |
|---|---|---|---|
| WO 0041469 A | 20-07-2000 | AU 2541000 A | 01-08-2000 |
| WO 9951638 A | 14-10-1999 | AU 3545399 A | 25-10-1999 |
| | | EP 1070085 A | 24-01-2001 |
| WO 0069472 A | 23-11-2000 | NONE | |
| WO 9810795 A | 19-03-1998 | AU 4412297 A | 02-04-1998 |
| | | EP 0927045 A | 07-07-1999 |
| | | JP 2001501600 T | 06-02-2001 |
| WO 0117563 A | 15-03-2001 | NONE | |

EPO FORM P0459

For more details about this annex : see Official Journal of the European Patent Office, No. 12/82